(19) Europäisches Patentamt
European Patent Office
Office européen des brevets

(11) Publication number: 0 513 760 A2

# (12) EUROPEAN PATENT APPLICATION

(21) Application number: 92108073.5

(22) Date of filing: 13.05.92

(51) Int. Cl.5: A61K 31/66, A61K 31/665, A61K 31/67, A61K 31/675

(30) Priority: 13.05.91 US 699049

(43) Date of publication of application: 19.11.92 Bulletin 92/47

(84) Designated Contracting States: AT BE CH DE DK ES FR GB GR IT LI LU MC NL PT SE

(71) Applicant: E.R. SOUIBB & SONS, INC.
P.O.Box 4000
Princeton New Jersey 08543-4000(US)

(72) Inventor: Biller, Scott A.
136 Nancy Lane
Ewing, NJ(US)
Inventor: Magnin, David R.
40 Cottage Court
Hamilton, NJ(US)

(74) Representative: Vossius & Partner
Siebertstrasse 4 P.O. Box 86 07 67
W-8000 München 86(DE)

## (54) Use of biphosphonate squalene synthetase inhibitors in pharmaceutical compositions useful in lowering cholesterol.

(57) The present invention relates to the use of a bisphosphonate squalene synthetase inhibitor for the preparation of a pharmaceutical composition useful in inhibiting cholesterol biosynthesis by inhibiting de novo squalene production.

EP 0 513 760 A2

The present invention relates to the use of a bisphosphonate squalene synthetase inhibitor for the preparation of a pharmaceutical composition useful in inhibiting cholesterol biosynthesis by inhibiting de novo squalene production.

Squalene synthetase is a microsomal enzyme which catalyzes the reductive dimerization of two molecules of farnesyl pyrophosphate (FPP) in the presence of nicotinamide adenine dinucleotide phosphate (reduced form) (NADPH) to form squalene (Poulter, C. D.; Rilling, H. C., "Biosynthesis of Isoprenoid Compounds", Vol. I, Chapter 8, pp. 413-441, J. Wiley and Sons, 1981 and references therein). This enzyme is the first committed step of the de novo cholesterol biosynthetic pathway. The selective inhibition of this step should allow the essential pathways to isopentenyl tRNA, ubiquinone, and dolichol to proceed unimpeded. Squalene synthetase, along with HMG-CoA reductase has been shown to be down-regulated by receptor mediated LDL uptake (Faust, J. R.; Goldstein, J. L.; Brown, M. S. Proc. Nat. Acad. Sci. USA, 1979, 76, 5018-5022), lending credence to the proposal that inhibiting squalene synthetase will lead to an up-regulation of LDL receptor levels, as has been demonstrated for HMG-CoA reductase, and thus ultimately should be useful for the treatment and prevention of hypercholesterolemia and atherosclerosis.

In accordance with the present invention, pharmaceutical compositions are provided for inhibiting cholesterol biosynthesis employing a bisphosphonate squalene synthetase inhibitor which includes a methylene bridge between the phosphonate moities and includes at least one lipophilic group attached to the methylene group which bridges the phosphonate moieties.

As will be seen hereinafter, the terms "bisphosphonic," "diphosphonic," "bisphosphonates" and "diphosphonates" are used interchangeably.

The term "lipophilic group" refers to a group which preferably contains at least six carbons (more preferably greater than 10) and preferably less than 2 polar substituents bearing OH, NH or C = O functions. The lipophilic substituent is required for strong enzyme inhibitor binding and inhibition of the enzyme squalene synthetase or other enzymes in the cholesterol biosynthetic pathway such as in the pathway from isopentenyl diphosphate to squalene, that is, farnesyl diphosphate synthetase and isopentenyl diphosphate - dimethylallyl diphosphate isomerase.

According to one aspect of the present invention, pharmaceutical compositions are provided for inhibiting or treating hypercholesterolemia, and thereby inhibiting or treating atherosclerosis, by administering to a patient in need of such treatment a squalene synthetase inhibiting amount of a bisphosphonate compound having the structure

I

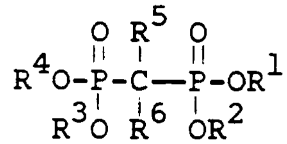

wherein $R^1$, $R^2$, $R^3$ and $R^4$ are the same or different and are H, alkyl, aryl, alkylaryl, arylalkyl, ammonium, alkali metal or a prodrug ester, preferably no more than one of $R^1$, $R^2$, $R^3$ and $R^4$ is alkyl, wherein at least one of $R^5$ and $R^6$ is a hydrocarbyl group having at least 6 carbons (such as alkyl, alkenyl, alkynyl, cycloalkyl, aryl, alkylaryl, arylalkyl, arylalkenyl); heterocyclic (such as succinimdyl, pyridyl, quinalyl, morpholino, furanyl, indolyl, picolinyl, thiophene, imidazole, oxazole, isoxazole, thiazole, pyridine, 1,2,3-triazole, 1,2,4-triazole, benzimidazole, tetrahydrofuranyl, pyrrolidino, piperidino, 5-membered heteroarylmethyl containing 2 to 4 N atoms or 1-2 N atoms plus an O or S atom); heterocyclicalkyl (wherein heterocyclic is as defined above such as 1-(decahydroquinolin-3-yl)methane); amino; alkylamino; dialkylamino; arylalkylaminoalkyl; ethylcarbonyloxymethylamino; cycloalkyl(alkyl)amino; alkenylamino, cycloalkylamino, aminocycloalkyl; aminocycloalkylalkyl; N-hydroxy-N-ethylamino; acetylamino; aminoalkyloxyalkyl; (benzo-or cyclohexeno-fused) 5 membered heteroaryl containing 2-4 N atoms or 1-2 Natoms plus an O or S atom; $R^8$-X-$(CH_2)_a$- (wherein $R^8$ is H, alkyl, or a nitrogen containing 6-membered aromatic ring such as pyridyl, indanyl, hexahydroindanyl or picolyl; X is O, NH or a single bond and a is 0 to 7);

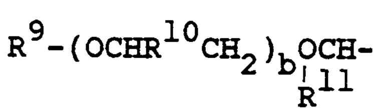

(wherein $R^9$ is $C_1$-$C_{10}$ alkyl, optionally substituted aryl, phenylalkyl or naphthylalkyl),

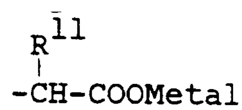

or

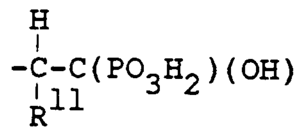

(wherein $R^{10}$ and and $R^{11}$ are the same or different and are H or methyl, b is 1 to 20));

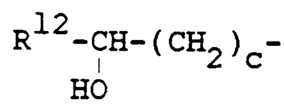

(wherein $R^{12}$ is H, phenyl or phenyl substituted with halogen, alkyl or hydroxy and c is 0 to 9);

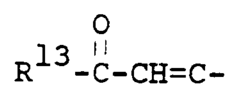

(wherein $R^{13}$ is tert-alkyl ($CR^{14}R^{15}R^{16}$ wherein $R^{14}$ and $R^{15}$ are independently $C_1$-$C_3$ alkyl and $R^{16}$ is $C_1$-$C_{10}$ alkyl), cycloalkyl, aryl or heteroaryl, or substituted cycloalkyl, substituted aryl or substituted heteroaryl wherein the substituent is halogen, $C_1$-$C_4$alkyl, alkoxy or dialkylamino);

4-Cl-$C_6H_5$-S-$CH_2$; aryloxy;

$R^{17}$-$(QCH_2CH_2)_dO$- (wherein $R^{17}$ is $C_1$-$C_{10}$ alkyl, $C_2$-$C_{10}$ alkenyl, aryl or arylalkyl, or each of the above $R^{17}$ groups optionally substituted with $C_1$-$C_4$ alkyl, amino, alkylamino, carboxyl, alkoxycarbonyl, hydroxy, alkoxy, phenoxy, mercapto, alkylthio, phenylthio, halogen or trifluoromethyl, Q is O or S and d is 0, 1 or 2);

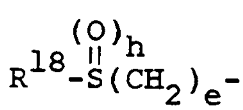

(wherein e is 0 to 10, h is 0, 1 or 2, $R^{18}$ is H, cycloalkyl, aryl, alkyl, each optionally substituted with OH, SH, halogen, alkoxycarbonyl or $NZ_1Z_2$, phenyl optionally substituted with halogen, nitro, lower alkyl, alkoxy, trifluoromethyl, amino, carboxyl, $CO_2$alkyl, -$CONZ_1Z_2$, -$CSNZ_1Z_2$, a 5- or 6-membered heterocyclic radical containing 1 or 2 heteroatoms, which are N or S, which may or may not be fused to a benzene ring, $Z_1$ and $Z_2$ are independently H or lower alkyl);

thiol; phenylthio; chlorophenylthio; 4-thiomorpholinyl;

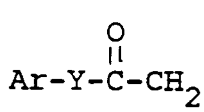

(wherein Ar is aryl, pyrrolyl or aryl optionally substituted with $C_1$-$C_4$alkyl, alkoxy, halo (F, Cl), naphthyl, biphenyl or thienyl and Y is NH or a single bond);

$R^{19}SCH_2$- (wherein $R^{19}$ is alkyl, aryl or arylalkyl);

A-$(CH_2)_f$-NH- (wherein A is $C_5$-$C_8$ cycloalkenyl, bicycloheptyl, bicycloheptenyl, saturated $C_4$-$C_7$

heterocycle containing O,S,SO or $SO_2$);

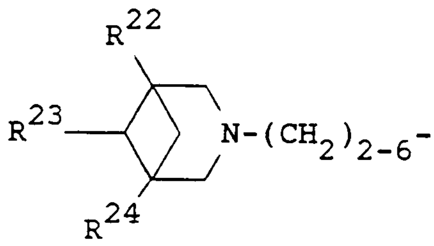

(wherein $R^{22}$ is H, $C_1$-$C_{20}$alkyl, alkoxy, aryl, $R^{23}$ is H, $C_1$-$C_{20}$alkyl, alkoxy, aryl, halo, carboxyl, $R^{24}$ is H, $C_1$-$C_{20}$ alkyl, alkoxy);

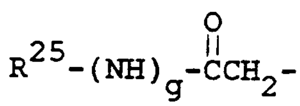

(wherein $R^{25}$ is (alkyl-substituted)pyrrolyl or phenyl and g is 0 or 1);

aromatic-substituted mono- or biazacyclylalkyl (alkyl group bonds with the N in the heterocycle) (such as 3-(4-phenylpiperidino)propyl);

$R^{31}$-Ax-CO-$CH_2$-

(wherein Ax is phenyl, naphthyl, mono- or bicyclic-N-containing heterocycle and $R^{31}$ is H, halo, lower alkyl or lower alkoxy);

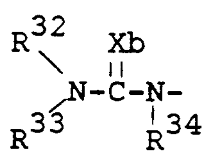

(wherein $R^{32}$ is aryl, aralkyl, alkyl, $R^{33}$ is H or aryl, Xb is O or S, and $R^{34}$ is H or alkyl);

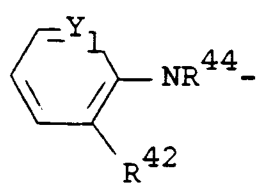

(wherein $R^{42}$ is H, alkyl or halo, $Y_1$ is N, NO, or $NR^{43}Y_2$ wherein $R^{43}$ is alkyl and $Y_2$ is halo; and $R^{44}$ is H or aliphatic acyl);

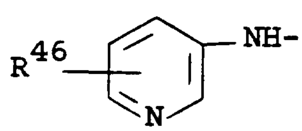

(wherein $R^{46}$ is H, halo or alkyl);

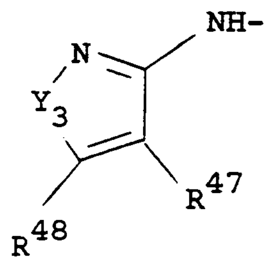

(wherein $Y_3$ is O or NH, $R^{47}$ is H, alkyl or halo, and $R^{48}$ is H or alkyl);

$R^{50}$-NH-

(wherein $R^{50}$ is

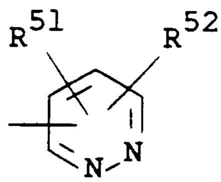

,

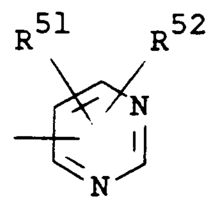

or

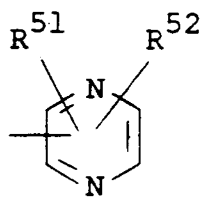

wherein $R^{51}$ and $R^{52}$ are H, halo, alkyl or hydroxy);

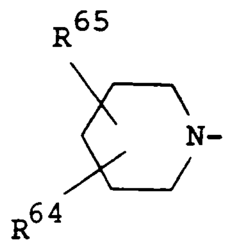

(wherein $R^{64}$ is alkyl and $R^{65}$ is H or alkyl;

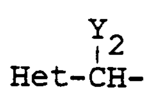

wherein Het is a heteroaromatic 5-membered ring with 2 or 3 heteroatoms, optionally partially hydrogenated and optionally substituted by one or more alkyl, alkoxy, phenyl, cyclohexyl, cyclohexylmethyl, halo or amino, with 2 adjacent alkyl optionally together forming a ring (Het cannot be pyrazole), and $Y_2$ is H or lower alkyl);

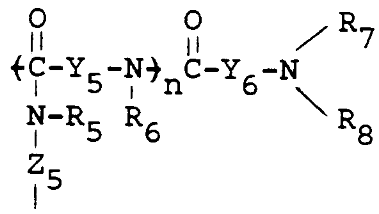

(wherein $Y_4$ is H or OH, $R_5$-$R_8$ are independently H or lower alkyl, whereby $R_7$ and $Y_6$ or $R_6$ and $Y_5$ or $R_5$ and $Z_5$, together with the nitrogen atom to which they are attached can form a 5- or 6-membered ring, $Y_6$ and $Y_5$ which can be the same or different are $C_1$-$C_6$ alkylene chains optionally substituted by aromatic or heteroaromatic radicals, $Z_5$ is $C_1$ to $C_6$ alkylene which can include heteroatoms and optionally substituted by aromatic or heteroaromatic, n is 0, 1 or 2;

$R_{27}$-$Z_9$-

(wherein $R_{27}$ is aryl or heterocyclyl both optionally substituted by one or more of lower alkyl, lower alkoxy, lower alkylthio, halo(lower)alkyl, acyl, acylamino or halo, or $R_{27}$ is lower alkyl substituted by heterocyclyl which is optionally substituted by acyl); $R_{27}$-$Z_9$ is $R_{27}$-NHC(=$X_9$), $R_{27}$-C(=O)NH-, $R_{27}$-$SO_2$-NH- (wherein $X_9$ is O or S);

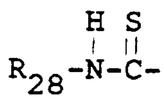

(wherein $R_{28}$ is phenyl, pyridyl or quinolyl substituted by lower alkylsulphonylamino, halo-lower alkylsulphonylamino, arylsulphonylamino and mono- or di-lower alkylamino);

$R_{29}$-CO-[-$R_{30}$($CH_2$)$_o$CO-]$_p$-NH-

(wherein $R_{29}$-CO- is a residue of a pharmaceutically active compound $R_{29}$-COOH, wherein $R_{29}$ is an anti-inflamatory agent, or antioncotic agent or hormone ,

$R_{30}$ is -NH- or -O-
p is 0 or 1;
o is 1-10);
$R_{33}$-($CH_2$)$_q$-

(wherein $R_{33}$ is an N-bonded azabicycloalkyl group with 3 to 8-membered rings and q is 2 to 4);

$R_{34}$-($CH_2$)$_r$-

(wherein $R_{34}$ is an N-bonded, aryl-substituted mono-or diazacycloaliphatic group);

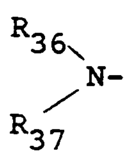

(wherein $R_{36}$ is 5 membered heteroaryl with 2-4 N or with 1-2 N plus an O or S atom, optionally fused to a benzo or cyclohexeno ring;

$R_{36}$ can be C substituted by lower alkyl, phenyl (optionally substituted by lower alkyl, alkoxy and/or halo), lower alkoxy, OH, di(lower alkyl)amino, lower alkylthio and/or halo, and/or N substituted by lower alkyl or phenyl (lower) alkyl (optionally substituted by lower alkyl, lower alkoxy and/or halo);

$R_{37}$ is H or lower alkyl; provided $R_{37}$ is not H if $R_{36}$ is optionally substituted alkyl and/or halo substituted 3-pyrazolyl or 3-isoxazolyl);

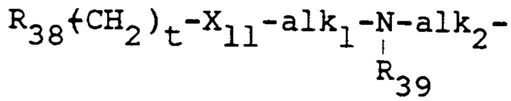

(wherein $R_{38}$ is aromatic residue;

t is 0-3;

$X_{11}$ is 0 S (optionally oxidized) or imino (optionally substituted by aliphatic group);

$alk_1$ and $alk_2$ are divalent aliphatic groups;

$R_{39}$ is H or monovalent aliphatic group);

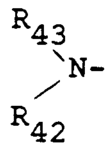

(wherein $R_{42}$ and $R_{43}$ are hydrogen, alkyl having one to 22 carbon atoms, cycloalkyl having five to six carbon atoms, phenyl alkylphenyl having seven to 18 carbon atoms, phenylalkyl having seven to 18 carbon atoms and together with the nitrogen atom, piperidino, pyrrolidino and morpholino);

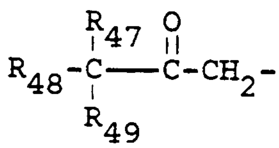

(wherein $R_{47}$ is optionally branched $C_1$-$C_8$alkyl,

$R_{48}$ and $R_{49}$ are each methyl or ethyl, and

M is H or a cation of a water-soluble base);

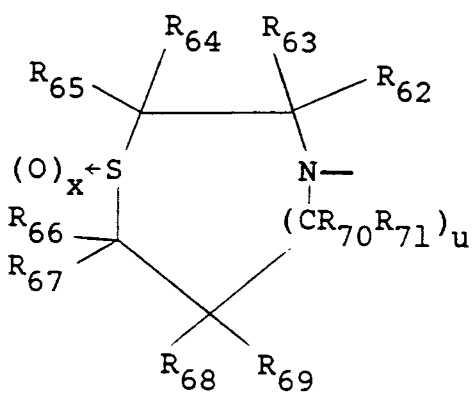

(wherein $R_{62}$-$R_{71}$ is H, straight, branched or alicyclic 1-10C hydrocarbyl, aryl or aryl-(1-4C)-alkyl;

x is 0 or 1;

u is 0, 1 or 2;

or $R_{62}$ and $R_{64}$ may complete a 5- to 7-membered saturated aliphatic ring optionally substituted by 1 or more alkyl groups);

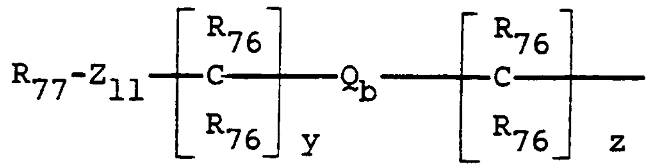

(wherein $Z_{11}$ is an N-containing 6-membered ring heterocycle moiety selected from piperidinyl, diazinyl or triazinyl;

$Q_b$ is a covalent bond, O, S or $NR_{76}$;

y, z, and y + z are integers of 0-10;

$R_{76}$ is H, or $C_1$-$C_3$alkyl;

$R_{77}$ is one or more substituted selected from H, halogen, 1-3C alkyl, unsubstituted amino and its amide derived from a 1-3C carboxylic acid, mono(1-3C alkyl) amino and its amide derived from a 1-3C carboxylic acid, di(1-3C alkyl)amino, tri(1-3C alkyl) ammonium, hydroxy or its ester derived from a 1-3C carboxylic acid, ether having 1-3C, $CO_2H$ and its salts and esters derived from 1-3C alcohols, its amide optionally substituted with one or two 1-3C alkyl groups, and $NO_2$);

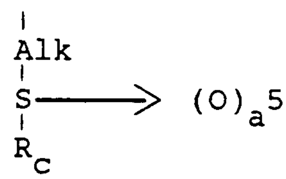

(wherein $R_c$ represents:

$C_1$-$C_6$ alkyl group,

$C_5$-$C_7$ cycloalkyl group,

phenyl group optionally monosubstituted or polysubstituted by a halogen, a $C_1$-$C_6$ alkyl group or a trifluoromethyl group, or

5-membered or 6-membered heterocycle containing 1 or 2 heteroatoms chosen from nitrogen and sulfur,

Alk denoted a linear or branched $C_1$-$C_6$ alkylene group,

$a^5$ represents 0 or the integer 1 or 2);

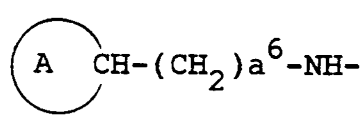

(wherein $a^6$ is 0 to 4 and

Ring A is 5-8C cycloalkenyl, bicycloheptyl, bicycloheptenyl or 4-7C saturated heterocyclyl containing 0, S, SO or $SO_2$);

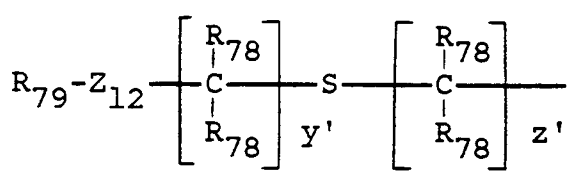

(wherein $Z_{12}$ is a 6-membered aromatic ring containing ≧ 1 N atom(s); where:

the ring is optionally substituted by (optionally substituted, optionally unsaturated) 1-6C alkyl, (optionally substituted) aryl, (optionally substituted) benzyl, OH, halogen, carbonyl, alkoxy, $NO_2$, $CONH_2$, (optionally substituted) $NH_2$ and/or carboxylate, such as pyridine, pyridazine, pyrimidine or pyrazine ring;

$R_{78}$ is H or (optionally substituted, optionally unsubstituted) 1-4C alkyl;

$R_{79}$ is H, (optionally substituted, optionally unsubstituted) 1-6C alkyl, (optionally substituted) aryl,

(optionally substituted) benzyl, OH, halogen, carbonyl, alkoxy, $NO_2$, $CONH_2$, (optionally substituted) amino or carboxylate,

y' + z' is 0 to 5);

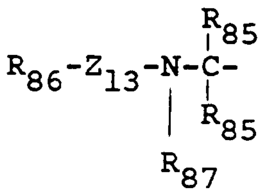

or

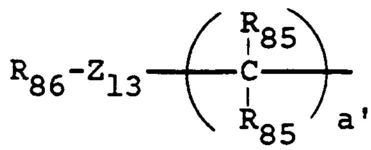

(wherein $Z_{13}$ is a pyridine, pyridazine, pyrimidine or pyrazine ring, optionally substituted by optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{86}$ is H or optionally substituted, optionally unsaturated 1-4C alkyl;

$R_{86}$ is one or more of H, optionally substituted, optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{87}$ is H, optionally substituted, optionally unsaturated 1-4C alkyl or acyl;

a' is 1-5);

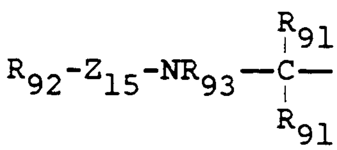

or

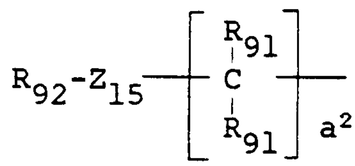

(wherein $Z_{15}$ is a 6 membered aromatic ring containing one or more N atoms such as pyridine, pyridazine, pyrimidine or pyrazine, which ring may be substituted with one or more optionally substituted, optionally unsaturated 1-6C alkyl, optionally substituted aryl, optionally substituted benzyl, OH, halogen, carbonyl, alkoxy, $NO_2$, amido, optionally substituted amino or carboxylate);

$R_{91}$ is H or optionally substituted, optionally unsaturated 1-4C alkyl;

$R_{92}$ is H or one or more substituents selected from optionally substituted, optionally unsaturated 1-6C alkyl, optionally substituted aryl, optionally substituted benzyl, OH, halogen, carbonyl, alkoxy, $NO_2$, amido, optionally substituted amino or carboxylate;

$R_{93}$ is H, optionally substituted, optionally unsaturated 1-4C alkyl or acyl;

$a^2$ is 1 to 5);

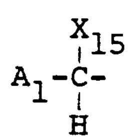

(wherein $X_{15}$ is hydrogen, methyl, or ethyl, and $A_1$ is phenyl substituted in the para-position by isobutyl, cyclohexyl, alkoxy, or 1-pyrrolinyl and, optionally substituted additionally in the meta-position by fluorine or chlorine, or phenyl substituted in the meta-position by benzoyl or phenoxy, or phenyl substituted in the ortho-position by 2,4-dichlorophenoxy or 2,6-dichlorophenylamino);

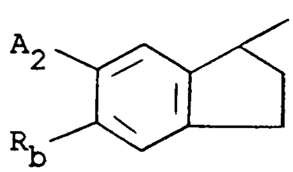

(wherein

$R_b$ is cyclohexyl or cyclophenylmethyl; and

$A_2$ is hydrogen or chlorine);

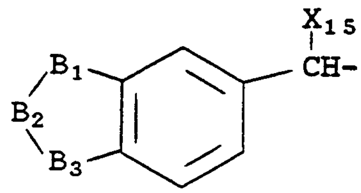

(wherein $X_{15}$ is as defined above, and

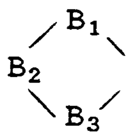 is

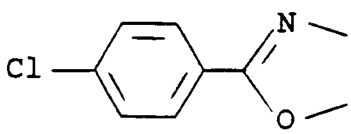

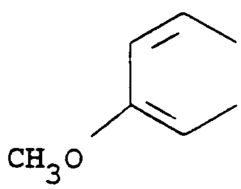 or 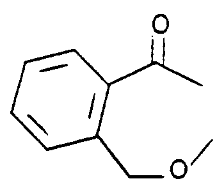 ;

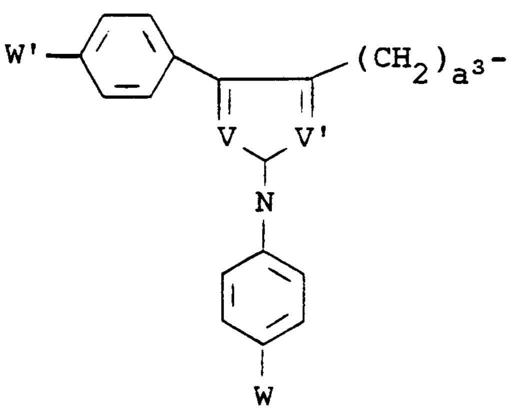

wherein $a^3$ is 1, 2 or 3;

W and W', are identical or different, and each is hydrogen, fluorine or chlorine, and

one of V and V' is nitrogen and the other is a methyne residue optionally substituted by a phenyl group, and

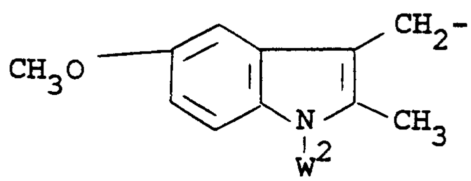

wherein $W^2$ is p-chlorobenzoyl or cinnamoyl);

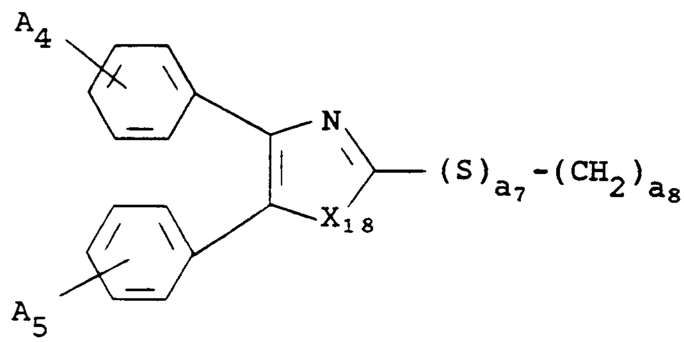

(wherein $A_4$ and $A_5$ are the same or different and are H, OH, lower alkoxy or halogen;
$X_{15}$ is O, S or NH;
$a_7$ is 0 or 1;
$a_8$ is 0 or an integer of 1-6);

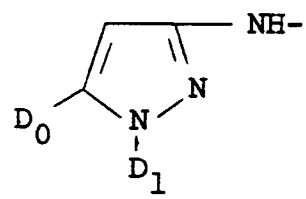

(wherein $D_0$ is H or alkyl;
$D_1$ is H or lower alkyl);

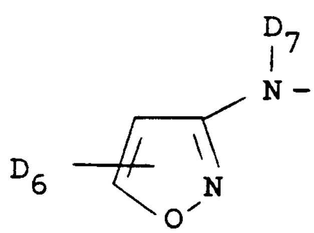

(wherein $D_6$ is H, 1-10C alkyl, 3-10C cycloalkyl, phenyl, 2-10C alkenyl (optionally substituted by phenyl) or phenyl(1-5C)alkyl (optionally ring-substituted by a 1-5C alkoxy);
$D_7$ is H or 2-6C alkanoyl);

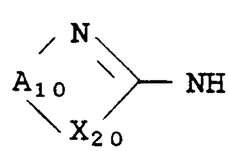

(wherein $A_{10}$ is a group of formula (a)-(c):

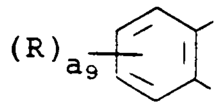

(a)

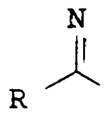

(b)

or

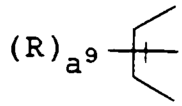

(c)

and $X_{20}$ is O, S or NH);

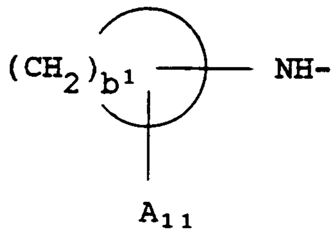

(wherein $A_{11}$ is H or 1-5C alkyl;
$b_1$ is 3-10);

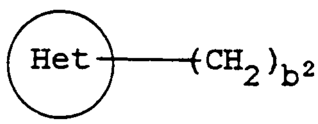

(wherein ring Het is a group of formula (A) or (B):

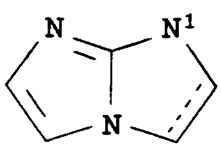

(A)

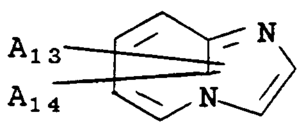

(B)

the dotted line represents an optional double bond; $A_{13}$, $A_{14}$ are H, 1-5C alkyl, halogen or OH);

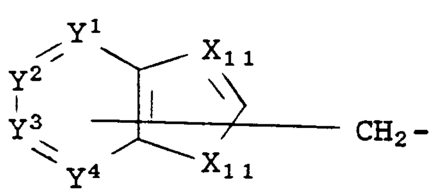

(wherein $X_{11}$ are both N or one is N and the other is CH; one of $Y^1$-$Y^4$ is N and the rest is CH);

and the other of $R^5$ and $R^6$ is H, halogen, $C_1$-$C_{30}$ alkyl, amino, alkylamino, dialkylamino, uriedo ($NH_2CO-N(R^{38})$- where $R^{38}$ is H, alkyl, benzyl, phenyl optionally substituted with Cl or $CH_3$); alkenylamino, cycloalkylamino, aryloxy, pyridinium, guanidinium, ammonium, di-and tri-lower alkanolammonium, hydroxy, arylalkyl, alkoxy, alkylaryloxy, $-CH_2CO_2H$, $-CH_2PO_3H_2$, $-CH(PO_3H_2)(OH)$, $-CH_2CO_2C_2H_5$, $-CH_2CH(PO_3H_2)_2$,

a hydrocarbyl radical as defined herein, a heterocyclic radical as defined herein, alkanoyl, an $R^6$ or $R^5$ radical as defined herein, a prodrug ester (such as (1-alkanoyloxy)alkyl, for example $t\text{-}C_4H_9CO_2CH_2$-, $CH_3CO_2CH_2$-);

at least one of $R^5$ and $R^6$ being a lipophilic group, or $R^5$ and $R^6$ can be joined to form a carbocyclic ring containing 3 to 12 carbons or a heterocyclic ring containing N, O and/or S atoms, such as of the formula

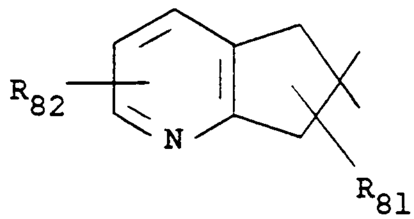

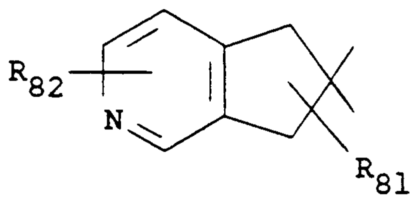

or

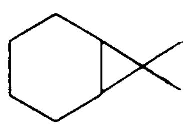

wherein $R_{81}$ and $R_{82}$ are each one or more substituents selected from H, optionally substituted saturated or unsaturated 1-6C alkyl, optionally substituted aryl, optionally substituted benzyl, amido, OH, halogen, optionally substituted amino, amido, COOH, carbonyl, carboxylate, alkoxy and $NO_2$.

In another aspect of the present invention, a hypocholesterolemic or hypolipemic composition is provided formed of a bisphosphonate squalene synthetase inhibitor of structure I and a pharmaceutically acceptable carrier therefor.

The various preferred bisphosphonate compounds of structure I which may be employed in the method of the invention and/or hypocholesterolemic composition of the invention are outlined below.

a) methylene diphosphonic acids and salts and esters as disclosed in U.S. Patent Nos. 3,299,123, 3,414,393 and 3,518,200 all to Fitch et al (all assigned to Monsanto), U.S. Patent Nos. 3,463,835, 3,471,406, 3,892,676 and 4,440,646 all to Budnick (all assigned to Plains Chemical Development Co.) (disclosed for use as surfactants, metal ion sequestering and deflocculating agents for detergents, gasoline additives, dry cleaning agents) having the formula

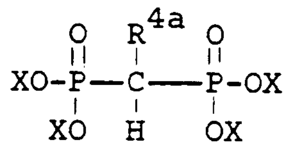

wherein $R^{4a}$ is an aliphatic hydrocarbyl (for example, alkyl, aralkyl), alicyclic (for example, cycloalkyl), aryl, alkylaryl of from 5 to 30 carbon atoms and carbon containing heterocyclics (such as those set out above with respect to $R^4$ and $R^5$) (any of $R^{4a}$ being optionally substituted with OH, halo, alkoxy, ester, ether, nitro, sulfonyl, amido, amino, carboxyl or nitroso); and

X is H, alkali metal, alkaline earth metal, aluminum, ammonium, amine and aliphatic hydrocarbyl, aryl, alkylaryl of from 1 to 30 carbons;

b) alkylenediphosphonic acids and/or salts disclosed in U.S. Patent Nos. 3,297,578 to Crutchfield et al, and 3,346,487 to Irani et al (both assigned to Monsanto) (used in detergents) and having the formula

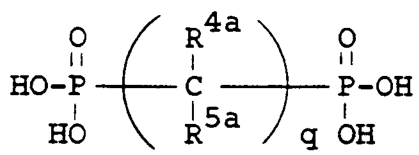

wherein $R^{4a}$ is H or $C_1$-$C_4$ alkyl, and $R^{5a}$ is H, OH or $C_1$-$C_4$ alkyl;

c) substituted methylene diphosphonic acids esters, or salts thereof as disclosed in U.S. Patent Nos. 3,404,178 and 3,422,021 each to Roy (both assigned to Procter & Gamble) (used in detergents) having the formula

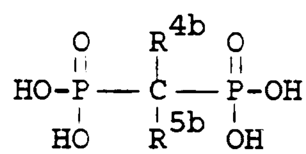

wherein $R^{4b}$ and $R^{5b}$ are each selected from H, methyl, benzyl or carboxymethylene ($CH_2CO_2H$), at least one of $R^{4b}$ and $R^{5b}$ being other than H;

d) alkyldiphosphonic acids, esters or salts as disclosed in U.S. Patent No. 3,609,075 to Barbera (assigned to Procter & Gamble) having the formula

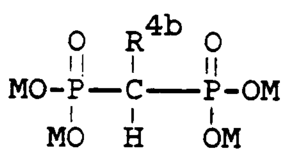

wherein $R^{4b}$ is alkyl of from 12 to 30 carbons and M is H, $C_1$ to $C_8$ alkyl, alkali metal or ammonium;

e) diphosphonates as disclosed in U.S. Patent Nos. 3,488,419 to McCune et al, 3,683,080 to Francis and 3,678,154 to Widder et al, (disclosed for use in compositions for inhibiting deposition and mobilization of calcium phosphate, arthritis, atherosclerosis) (not related to cholesterol biosynthesis inhibition or cholesterol lowering) having the formula

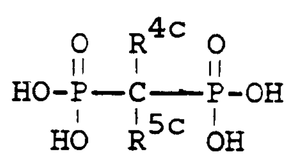

wherein $R^{4c}$ is H, $C_1$ to $C_{20}$ alkyl, $C_2$ to $C_{20}$ alkenyl, aryl, phenylethenyl, benzyl, halo, amino, substituted amino (for example, dimethylamino, diethylamino, N-hydroxy-N-ethylamino, acetylamino), $-CH_2COOH$, $-CH_2PO_3H_2$, $-CH(PO_3H_2)(OH)$ or $-CH_2CH(PO_3H_2)_2$,

$R^{5c}$ is H, lower alkyl, amino, benzyl, halo, OH, $-CH_2CO_2H$, $-CH_2PO_3H_2$, $-CH_2CH_2PO_3H_2$,

at least one of $R^{4c}$ and $R^{5c}$ being a lipophilic group;

[The following additional patents disclose type e) diphosphonate compounds:

U.S. Patent Nos. 4,330,530 to Baker (disclosed for use with gold salts for treating arthritis); 4,067,971 to Francis (disclosed for use in hypoxias and ischemic tissue diseases), 4,254,114 to Triebwasser (disclosed for use in control of pyrophosphate microorganisms), 4,137,309 to Van Duzee (disclosed for

use in sickle cell anemia), European Patent Application 88462A2 (disclosed for use with steroids for anti-inflammatory utilities)];

f) methanecycloalkylhydroxydiphosphinates as disclosed in U.S. Patent No. 3,553,314 to Francis (assigned to Procter & Gamble) [disclosed for use in oral compositions for calculus retardation and for treating deposition and mobilization of calcium phosphate including atherosclerosis (unrelated to cholesterol inhibition or lowering)] having the formula

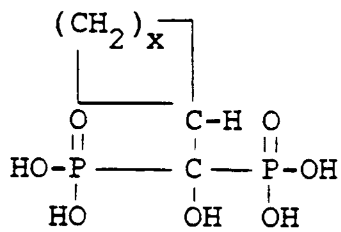

wherein x is 1 to 7 so that the ring may contain 4 to 10 carbons, including salts thereof such as alkali-metal, alkaline earth metal, non-toxic heavy metal, ammonium or low molecular weight substituted ammonium.

[The following additional patents disclose type f) compounds: U.S. Patent Nos. 3,959,458 to Agricola et al, 4,025,616 and 3,934,002 both to Haefele (all relating to therapeutic substances for use in toothpaste compositions), and U.S. Patent No. 3,584,125 to Francis (relating to substances for inhibiting anomalous deposition and mobilization of calcium phosphates in animal tissue, arthritis, atherosclerosis (unrelated to cholesterol inhibition or lowering)); GB 1,453,667 (for containing radioactive P for treatment of tumors) (all assigned to Procter & Gamble)];

g) alkyldiphosphonates as disclosed in U.S. Patent No. 4,113,861 to Fleisch (assigned to Procter & Gamble) (disclosed or use in treatment of diabetes) having the formula

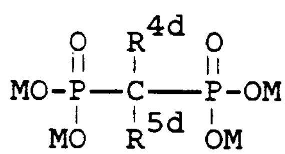

wherein $R^{4d}$ is a $C_2$ or higher hydrocarbyl group (preferably containing 6 to 13 carbons) such as unsubstituted or substituted alkyl, cycloalkyl, alkenyl, alkynyl or carbocyclic group (cycloalkyl)

$R^{5d}$ is H, OH or $NH_2$,

M is H, metal ion (such as alkali metal),

alkyl or aryl;

i) polyphosphonates as disclosed in U.S. Patent No. 4,761,406 to Flora et al (assigned to Procter & Gamble) (disclosed for treating or preventing osteoporosis) having the formula

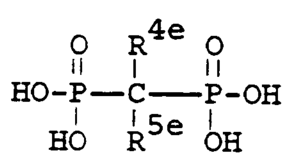

wherein $R^{4e}$ is $R^7$-X-$(CH_2)_a$-

wherein $R^7$ is H or a nitrogen-containing 6-membered aromatic ring (such as pyridyl, indanyl, hexahydroindanyl, picolyl);

X is -NH-, oxygen or a single bond;

"a" is 0 to 7;

$R^{5e}$ is H, Cl, amino or OH;

j) bisphosphonates as disclosed in Derwent No. 85-223756 (Akad Wissenschaft DDR) and DE 3804686

(Henkel) (disclosed for use in anticancer compositions) having the formula

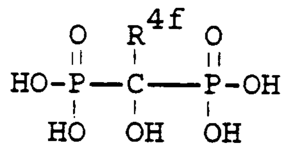

wherein $R^{4f}$ is $C_6$ to $C_{17}$ alkyl (Akad Wissenschaft) and $C_1$ to $C_9$ alkyl (Henkel) containing amino, carboxylate and other substituents);

k) bisphosphonates as disclosed in DE 3,425,746 (Amersham Buchler) (disclosed for use in diagnosis and treatment of bone tumors, and other diseases of the skeletal system) having the formula

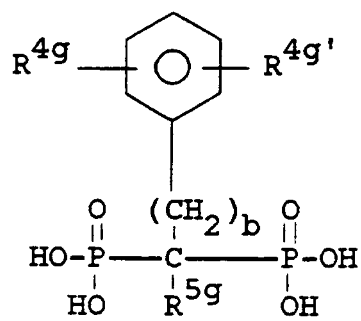

wherein $R^{4g}$ and $R^{4g'}$ are independently H, alkyl, aryl, OH, alkoxy, aryloxy, amino, alkyl- or arylamino, carboxylalkyl, mercapto, alkyl- or arylthio, halo, nitro, cyano, sulfonic or sulfonamide, and $R^{5g}$ is H, alkyl, aryl, OH, halo, amino, $PO_3H_2$ (alkyl has 1 to 6 carbons and is branched or unbranched, aryl has 6 to 14 carbons and either may be substituted);

l) oxa-alkane diphosphonic acids as disclosed in U.S. Patent No. 4,892,679 to Blum et al (assigned to Henkel Kommanditgesellschaft) (disclosed for use as metal ion complexing agents and in diseases of calcium and phosphate metabolism) having the formula

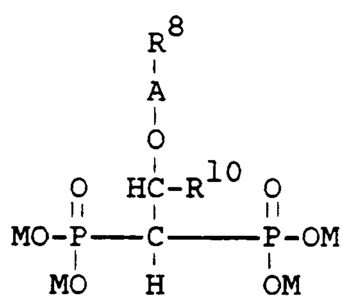

wherein A is

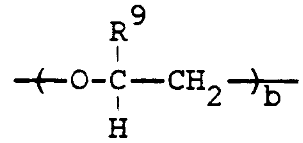

where b is 1 to 20,

$R^8$ is $C_1$-$C_{10}$ alkyl, optionally substituted $C_6$-$C_{10}$ aryl, phenylalkyl, naphthylalkyl,

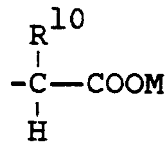 , 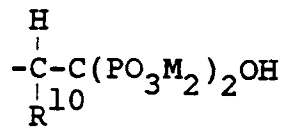

$R^9$ and $R^{10}$ are independently H or $CH_3$; and

M is H or a monovalent cation;

m) dihydroxyalkane diphosphonic acids as disclosed in U.S. Patent No. 4,536,348 to Blum (assigned to Henkel) (disclosed for use as complexing and sequestering agents and in diseases of calcium and phosphate metabolism) having the formula

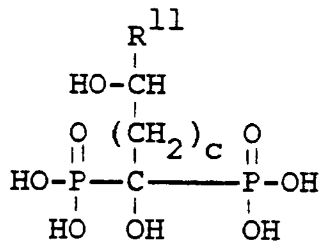

wherein $R^{11}$ is H, phenyl, phenyl substituted with halo, $C_1$ to $C_6$ alkyl or OH and c is 1 to 9;

n) 3-oxo-propene-1,1-diphosphonic acids as disclosed in European Patent 0301352A2 (disclosed for use as a calcium complexing agent and in diseases of calcium and phosphate metabolism and tartar prevention) having the formula

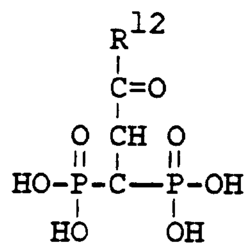

wherein $R^{12}$ is tert-alkyl $CR^{13}$, $R^{14}$, $R^{15}$ (wherein $R^{13}$ and $R^{14}$ are independently $C_1$-$C_3$alkyl and $R^{15}$ is $C_1$-$C_{10}$ alkyl); cycloalkyl; aryl; or heteroaryl; or cycloalkyl, aryl or heteroaryl substituted with halo, $C_1$-$C_4$alkyl, alkoxy or dialkylamino;

o) lipophilic bisphosphonates as disclosed in WO88/00829 (Leo Pharmaceutical Products) (disclosed for use for nasal administration in diseases involving calcium metabolism and arthritis) having the formula

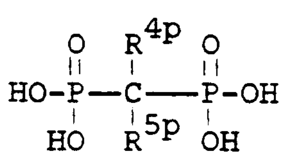

wherein $R^{4p}$ is alkyl, phenoxy, 4-Cl-$C_6H_4$-S-$CH_2$- and $R^{5p}$ is H or OH;

p) methylene bisphosphonic acids as disclosed in WO86/00902 (Leo Pharmaceutical Products) (disclosed for use in various diseases involving calcium phosphate deposition or resorption, including atherosclerosis, and prevention of dental calculus) having the formula

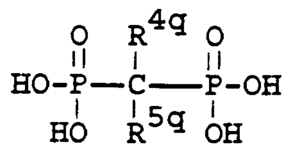

wherein $R^{4q}$ is $R^{16}$-$(QCH_2CH_2)_dO$- wherein $R^{16}$ is a straight or branched, saturated or unsaturated aliphatic or alicyclic $C_1$-$C_{10}$ hydrocarbon radical, an aryl or an aryl-$C_1$-$C_4$-alkyl radical, $R_1$ if desired being unsubstituted or substituted with straight or branched $C_1$-$C_4$-alkyl, amino, $C_1$-$C_4$-alkylamino, di-($C_1$-$C_4$-alkyl)-amino, carboxy, $C_1$-$C_4$-alkoxycarbonyl, hydroxy, $C_1$-$C_4$-alkoxy, phenoxy, mercapto, $C_1$-$C_4$-alkylthio, phenylthio, halogen, trifluoromethyl;

$R^{5q}$ is hydrogen, $C_1$-$C_8$-alkyl, aryl-$C_1$-$C_4$-alkyl or halogen;

Q is O or S, and d is 0, 1 or 2; with the proviso that $R^{5q}$ cannot be hydrogen or methyl if d = O and $R^{1b}$ is methyl,

including double esters thereof;

q) cyclic diphosphonic acids as disclosed in U.S. Patent No. 4,687,768 and European Patent Applications 0304961 and 0304962 all to Benedict et al and assigned to Procter & Gamble (disclosed for treating diseases characterized by abnormal calcium and phosphate metabolism) having the formula

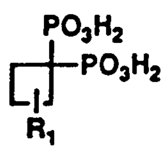

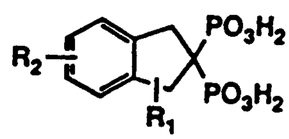

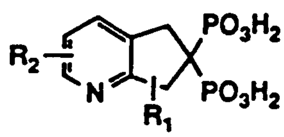

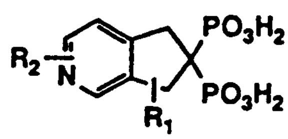

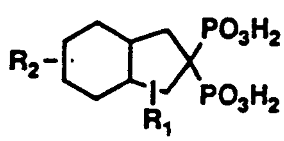

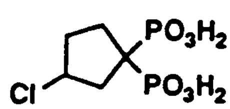

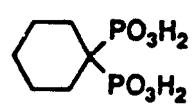

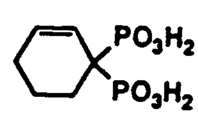

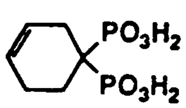

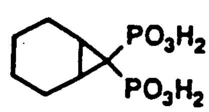

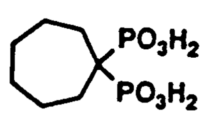

wherein $R_1$ represents one or more substituents including alkyl, alkenyl, aryl, benzyl, hydroxy, halogen, amino, amido, carboxy, carbonyl, carboxylate, alkoxy and combinations thereof, and

$R_2$ represents hydrogen, nitro, or any of the groups defined under $R_1$;

wherein the above $R_1$ and/or $R_2$ groups may optionally include substituents such as alkyl, alkenyl, aryl, halogen, hydroxy or cycloalkyl;

r) bisphosphonates as disclosed in U.S. Patent No. 4,515,766 to Castronovo et al (assigned to Mass. Gen. Hosp.) (disclosed for use in forming radiolabeled compounds for scanning for calcium deposits) having the formula

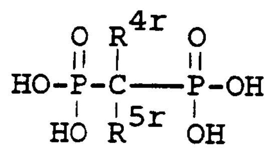

wherein $R^{4r}$ is aryl, and $R^{5r}$ is H, alkyl, alkenyl, amino, benzyl, OH, $-CH_2PO_3H$, $-CH_2CH_2PO_3O_2$.

s) bisphosphonates as disclosed in Derwent No. 79-00757C/01 (Japanese Patent No. 55-4147-925) (disclosed for use as herbicides) having the formula

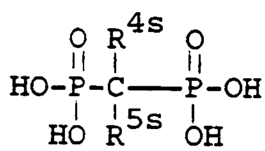

wherein $R^{4s}$ and $R^{5s}$ are independently H, halo, alkyl or cycloalkyl, at least one of $R^{4s}$ and $R^{5s}$ being a lipophilic group;

t) diphosphonic acids as disclosed in U.S. Patent Nos. 4,836,956 and 4,818,774 each to Kem (each assigned to Occidental Chemical Corp) (disclosed for use in extraction of uranium and other metals from water) having the formula

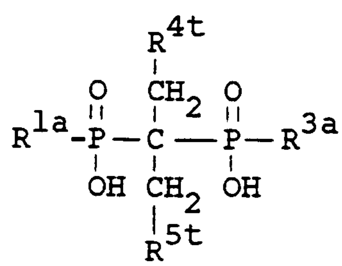

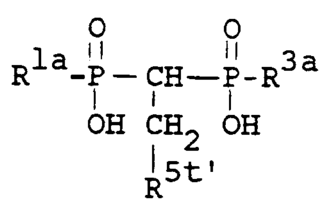

wherein $R^{1a}$ and $R^{3a}$ are the same or different and are alkyl and alkylaryl groups having from 1 to about 18 carbon atoms or hydroxyl;

$R^{4t}$ is independently selected from substituted or unsubstituted alkyl or alkylaryl groups having 1 to about 18 carbon atoms or hydrogen;

$R^{5t}$ is independently selected from alkyl or alkylaryl groups having 1 to about 18 carbon atoms; provided that the sum of carbon atoms of the R groups is at least 15; and

$R^{5t'}$ is independently selected from substituted and unsubstituted alkyl or alkylaryl groups having 1 to about 18 carbon atoms or a polymeric group; provided that the sum of the carbon atoms of the $R^1$, $R^{1a}$, $R^{3a}$ and $R^{5t'}$ groups is a least 16.

Substituted alkyl and alkylaryl groups include alkyl and alkylaryl groups substituted with moieties, such as fluoro, chloro, bromo, iodo and hydroxyl groups;

u) bisphosphonate acids as disclosed in European Patent Application 185589A (Rhone-Poulenc) (used for treatment of Paget's disease and osteoporosis) having the formula

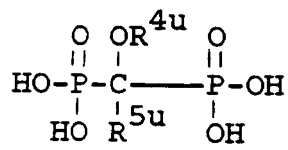

wherein $R^{4u}$ is $C_2$ or $C_3$ alkyl and $R^{5u}$ is H or alkyl; or $R^{4u}$ is $C_{1-3}$ alkyl and $R^{5u}$ is $C_{1-2}$alkyl.

v) methylene diphosphonic acids as disclosed in U.S. Patent Nos. 4,746,654 and 4,876,248 both to Breliere (both assigned to Sanofi) (disclosed for use as anti-inflammatory agents) having the formula

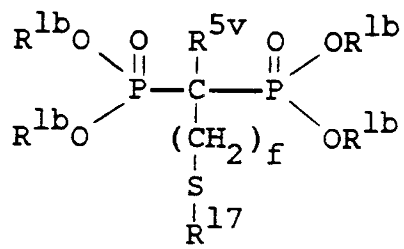

wherein $R^{1b}$ is hydrogen or a straight or branched lower alkyl group having from 1 to 4 carbon atoms;

$R^{17}$ is hydrogen, an alkyl group which is unsubstituted or substituted by a hydroxyl group, a thiol group, one or more halogen atoms, an alkoxycarbonyl group or a

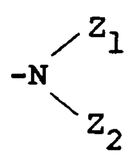 group,

where $Z_1$ and $Z_2$, considered independently of one another, are hydrogen or a lower alkyl group, a phenyl group which is unsubstituted or has one or more halogen, nitro group, lower alkyl group, lower alkoxy group, trifluoromethyl, $NH_2$ group, COOH group or COOalkyl group, a

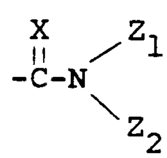 group,

where X is oxygen or sulfur and $Z_1$ and $Z_2$ are as defined above, a heterocyclic radical with 5 or 6 members, containing 1 or 2 heteroatoms chosen from amongst nitrogen and sulfur, or, a heterocyclic radical with 5 members fused to a benzene ring and having the formula

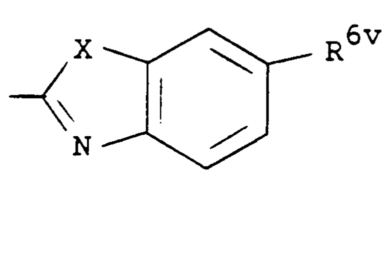

where X is oxygen, an NH group or sulfur and $R^{6v}$ is hydrogen or a halogen atom, preferably chlorine, $R^{5v}$ is hydrogen or a hydroxyl group, and f is an integer between 0 and 10, with the proviso that f cannot be 0 if $R^{5v}$ is OH;

w) bisphosphonic acids as disclosed in European Patent Application 336851A (Sanofi) (disclosed for use with sodium lauryl sulfate (for oral availability) for treating rheumatism and arthritis) having the formula

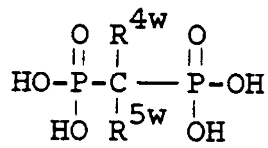

wherein $R^{4w}$ is halo, $C_{1-5}$ alkyl (optionally substituted with Cl, OH, $NH_2$ or dialkylamino), phenoxy, phenyl, thio, phenylthio, chlorophenylthio, pyridyl, 4-thiomorpholinyl and $R^{5W}$ is H, halo, OH, $NH_2$, dialkylamino;

x) diphosphonic acids as disclosed in U.S. Patent No. 4,503,049 to Biere et al (assigned to Schering A.G.) (disclosed for use as anti-inflammatory and antiarthritic agents and for diseases involving calcium) having the structure

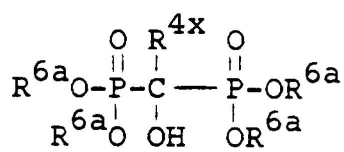

wherein $R^{6a}$ is hydrogen, an alkali metal atom, an alkaline earth metal atom, or alkyl of 1-4 carbon atoms, and $R^{4x}$ is the residue of a carboxylic acid containing an aromatic or heteroaromatic group and being of the formula

arylCOOH,

diphosphonic acid derivatives of the formula

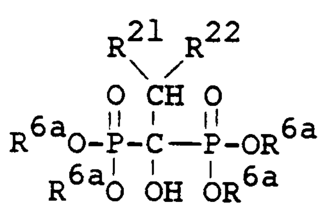

wherein $R^{6a}$ is as defined above;

$R^{21}$ is hydrogen, methyl, or ethyl; and

$R^{22}$ is phenyl substituted in the para-position by isobutyl, cyclohexyl, alkoxy, or 1-pyrrolinyl and, optionally substituted additionally in the meta-position by fluorine or chlorine; or phenyl substituted in the meta-position by benzoyl or phenoxy, or phenyl substituted in the ortho-position by 2,4-dichlorophenoxy or 2,6-dichlorophenylamino;

diphosphonic acid derivatives of the formula

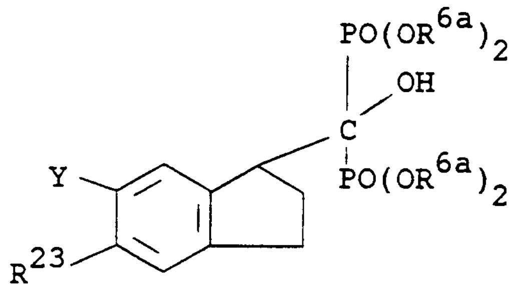

wherein $R^{6a}$ is as defined above;

$R^{23}$ is cyclohexyl or cyclopentylmethyl; and Y is hydrogen or chlorine;

diphosphonic acid derivatives of the formula

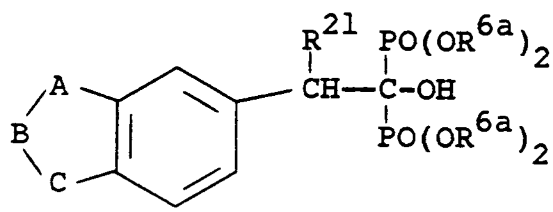

wherein $R^{6a}$ and $R^{21}$ are as defined above, and

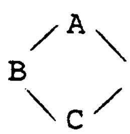

is

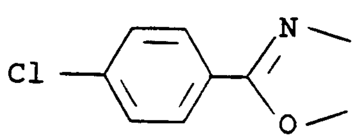

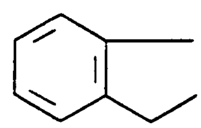

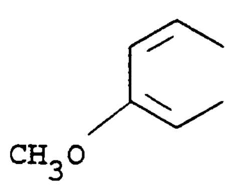

or

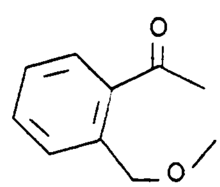

;

diphosphonic acid derivatives of the formula

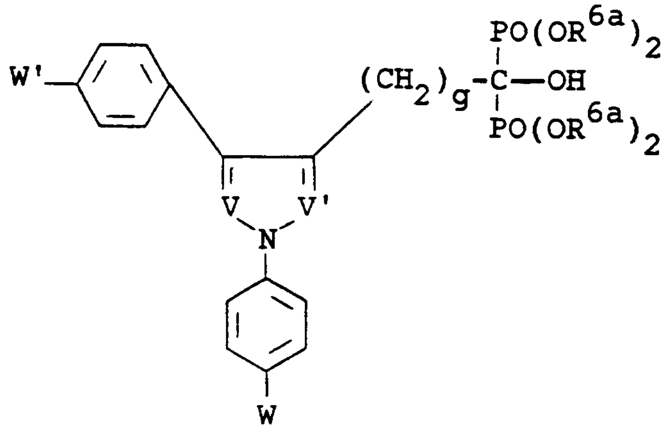

wherein g is 1, 2, or 3;

$R^{6a}$ is as defined above;

W and W', are identical or different, and each is hydrogen, fluorine or chlorine, and

one of V and V' is nitrogen and the other is a methyne residue optionally substituted by a phenyl group; and

diphosphonic acid derivatives of the formula

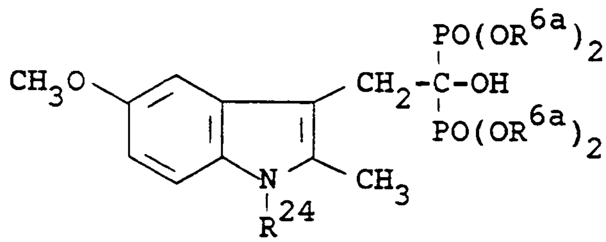

wherein $R^{6a}$ is as defined above, and

$R^{24}$ is p-chlorobenzoyl or cinnamoyl; or, throughout, when $R^{6a}$ is H, a physiologically acceptable salt thereof with an organic base;

y) diphosphonic acids as disclosed in U.S. Patent No. 4,473,560 to Biere et al (assigned to Schering AG) (disclosed for use as anti-inflammatory and antiarthritic agents, and for diseases involving calcium, such as Paget's disease, osteoporosis, and ectopic calcification) having the formula

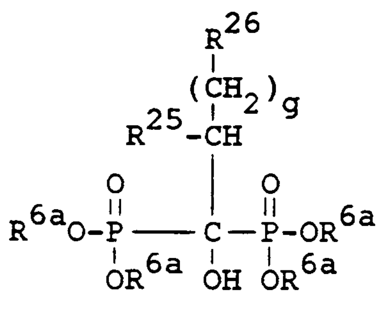

wherein g is 0, 1 or 2;

$R^{25}$ is H or $C_1$-$C_4$ alkyl;

$R^{6a}$ is H, alkali metal, alkaline earth metal, or $C_1$-$C_4$ alkyl; and

$R^{26}$ is phenyl optionally substituted by F atom(s), Cl atom(s), alkyl group(s) of 1-4 carbons, alkoxy group(s) of 1-4 carbons; naphthyl; biphenyl; or thienyl; or salts thereof;

z) bisphosphonates as disclosed in Japanese Patent JK 73-54,278 (used in peroxide bleaching solutions)

having the formula

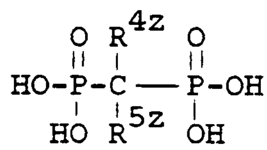

wherein $R^{4z}$ is H or alkyl and

$R^{5z}$ is H, OH or alkyl, at least one of $R^{4z}$ and $R^{5z}$ being a lipophilic group;

aa) alkylenepolyphosphonic acids as disclosed in U.S. Patent No. 4,276,089 to Moran (assigned to Union Chimique et Industrielle de l'Ouest S.A.) (disclosed for use with polyamines as corrosion inhibitors) having the structure

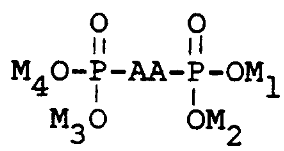

wherein AA represents a bivalent alkylene group which is a straight and saturated $C_1$-$C_{10}$ hydrocarbon chain, each carbon of which may be optionally substituted by at least one of OH, $C_1$-$C_4$ alkyl and phosphonic group

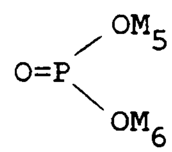

and $M_1$ to $M_6$ may be the same or different and are H, $C_1$-$C_4$ alkyl, $NH_4^+$ or a metal cation;

bb) bisphosphonates as disclosed in DD 237,252A, Derwent No. 86-291914/45 (assigned to Veb Chem Bitterfeld) (disclosed for use as a plant growth regulator) having the formula

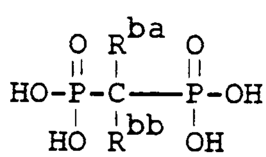

wherein $R^{ba}$ is H, lower alkyl, pyrrolidino or piperidino and

$R^{bb}$ is H, OH or lower alkyl, at least one of $R^{ba}$ and $R^{bb}$ being a lipophilic group;

cc) bisphosphonates as disclosed in Japanese Patent No. J-6-3,295,595A, Derwent No. 89-019688/03 (assigned to Yamanouchi Pharm KK) (disclosed or use as anti-inflammatory, and analgesic agents, and for bone abnormalities due to rheumatism, arthritis and osteoporosis), having the formula

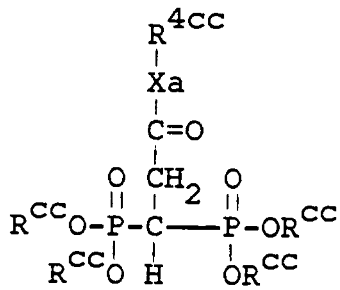

wherein $R^{cc}$ is H or lower alkyl;

Xa is NH or a bond; and

$R^{4cc}$ is phenyl or pyrrolyl each optionally substituted with alkyl;

dd) bisphosphonates as disclosed in USSR SU 862,439 (disclosed for use as collecting agents for the flotation of tin containing ore) having the formula

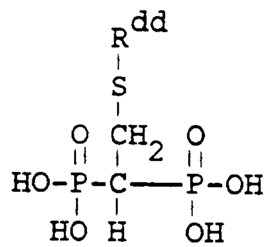

wherein $R^{dd}$ is a $C_3$-$C_{10}$ aliphatic (such as alkyl), aromatic (such as phenyl) or arylaliphatic (such as benzyl) hydrocarbyl group;

ee) substituted aminomethylenebis(phosphonic acid) derivatives as disclosed in European Patent Application 337706 (Yamanouchi Pharmaceutical Co.) (disclosed for use in bone resorption-inhibitory and anti-inflammatory effects) having the formula

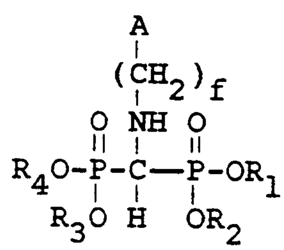

wherein $R_1$-$R_4$ is H or alkyl, f is o to 4 and A is $C_5$-$C_8$ cycloalkenyl, bicycloheptyl, bicycloheptenyl, saturated $C_4$-$C_7$ heterocyclyl containing O, S, SO or $SO_2$;

ff) araliphatyl aminoalkyldiphosphonic acids as disclosed in European Patent Application 320455 (Ciba-Geigy) (disclosed for use in disorders of calcium metabolism) having the formula

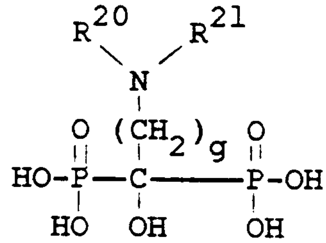

wherein $R^{20}$ is arylaliphatic residue, $R^{21}$ is H or aliphatic residue, and $(CH_2)_g$ is a divalent aliphatic residue where g is 1 to 6;

gg) azabicycloheptanes as disclosed in European Patent Application 317506 (Ciba-Geigy) (disclosed for use as calcium metabolism modulators) having the formula I wherein $R^5$ is

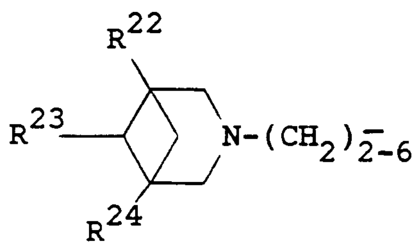

(wherein $R^{22}$ is H, $C_1$-$C_{20}$alkyl, alkoxy, aryl;

$R^{23}$ is H, $C_1$-$C_{20}$alkyl, alkoxy, aryl, halo, carboxyl;

$R^{24}$ is H, $C_1$-$C_{20}$alkyl, alkoxy);

hh) 3-oxopropylidene-1,1-diphosphonates as disclosed in Japanese Patent Application 87-271,433 (Yamanouchi Pharmaceutical Co.) (disclosed for use as inflammation inhibitors, analgesics, antipyretics) having the formula I wherein $R^5$ is

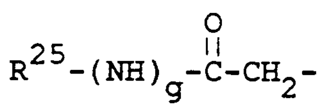

wherein $R^{25}$ is (alkyl-substituted)pyrrolyl or phenyl and g is 0 or 1;

ii) (aminomethylene)diphosphonic acids as disclosed in Japanese Patent 63150290 (Ciba-Geigy) having the structure

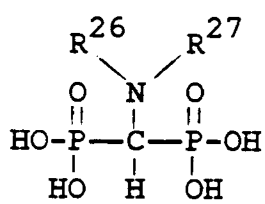

wherein $R^{26}$ is (benzo- or cyclohexeno-fused) 5-membered heteroaryl containing 2 to 4 N atoms or 1-2 N atoms plus an O or S atom with optional substituents such as alkyl or halo;

$R^{27}$ is K or alkyl, but $R^{27}$ is alkyl when $R^{26}$ is (alkyl and/or halo-substituted) pyrazol-3-yl or isoxazol-3-yl;

jj) alkylenediphosphonic acids as disclosed in Japanese Patent 63150291 (Ciba-Geigy) having the formula

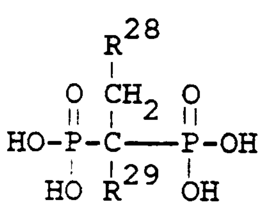

wherein $R^{28}$ is a 5-membered heteroaryl containing 2-4 N atoms or 1-2 N atoms plus an O or S atom optionally substituted with alkyl or halo, and

$R^{29}$ is H, OH, $NH_2$, alkylthio or halo;

kk) azacycloalkylalkanediphosphonic acids as disclosed in EP 272208 (Ciba-Geigy) (disclosed for use as regulators of calcium metabolism) having the formula

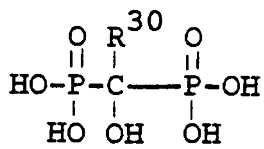

wherein $R^{30}$ is aromatic-substituted mono- or biazacyclylalkyl (alkyl bonds with N in heterocycle);
ll) 2-substituted-2-oxoethylene-1,1-diphosphonic acids as disclosed in Japanese Patent 63185993 (Yamanouchi Pharmaceutical Co.) (disclosed for use in bone disorders, anti-inflammatories) having the structure

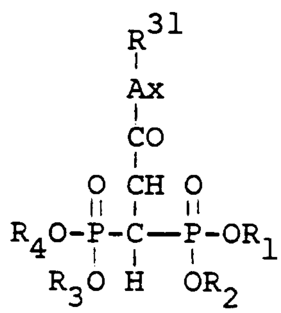

wherein $R_1$-$R_4$ is H or lower alkyl, Ax is phenyl, naphthyl, mono- or bicyclic N-containing heterocycles, and

$R^{31}$ is H, halo, lower alkoxy, lower alkyl;

mm) ureidoalkylbisphosphonic acids as disclosed in Japanese Patent 63088192 (Fujisawa Pharmaceutical Co.) (disclosed for use as bone absorption inhibitors) having the formula

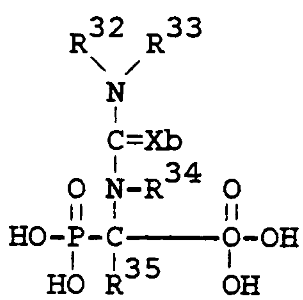

wherein $R^{32}$ is optionally substituted aryl, optionally substituted aralkyl, or optionally substituted alkyl,

$R^{33}$ is H or aryl,

$R^{34}$ and $R^{35}$ are H or alkyl, and

Xb is O or S;

nn) heterocyclicalkyl diphosphonic acids as disclosed in DE 3640938 (Boehringer Mannheim) (for use in treating calcium metabolic disorders) such as 1-(decahydroquinolin-3-yl)methane-1-hydroxy-1,1-diphosphonic acid, di Na salt;

oo) 1-aminoalkyl-1,1-bisphosphonic acids as disclosed in DD 222598 (Akademie der Wissenschaften) having the formula

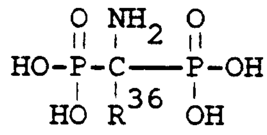

wherein $R^{36}$ is $C_2$-$C_{12}$ alkyl.

pp) methylenediphosphonic acids as disclosed in EP 151072 (Sanofi) (disclosed for use in anti-inflammatory and antiarthritic compositions) having the formula

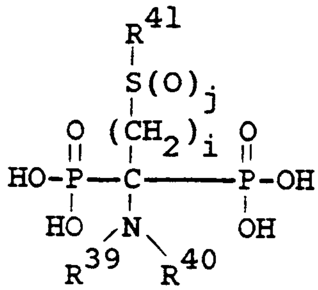

wherein $R^{39}$ is H, alkyl or $CONH_2$,

$R^{40}$ is H, alkyl, benzyl, optionally substituted phenyl (such as with Cl or $CH_3$), or $R^{39}$, $R^{40}$ are $(CH_2)_{4,5}$, j is 0, 1 or 2, i is 1 to 5,

$R^{41}$ is alkyl, cycloalkyl, optionally substituted phenyl or heterocyclyl;

qq) N-(unsubstituted or substituted pyridyl)amino-methylene diphosphonic acids as disclosed in U.S. Patent No. 4,447,256 (Nissan Chemical Industries) (disclosed for use as herbicides) having the formula

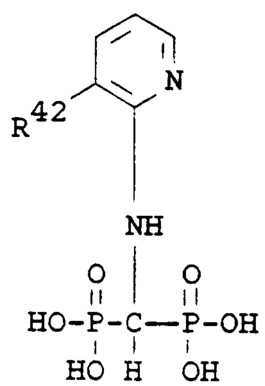

wherein $R^{42}$ is H, alkyl or halo;

rr) pyridylaminomethylenediphosphonates as disclosed in Japanese Patent 55089210 (Nissan Chemical Industries) (disclosed for use as herbicides) having the formula

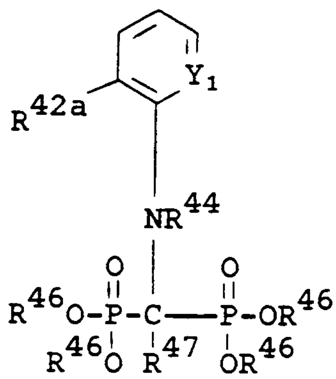

wherein $R^{46}$ is OH, halo, phenoxy or alkylamino,

$R^{47}$ is H or halo,

$R^{44}$ is H or aliphatic acyl (such as alkanoyl or alkenoyl),

$R^{42a}$ is alkyl, and

$Y_1$ is N, NO or $NR^{43}Y_2$, wherein $R^{43}$ is alkyl and $Y_2$ is halo;

ss) pyridylbisphosphonates as disclosed in Japanese Patent 55098105 (Nissan Chemical Industries) (disclosed for use as herbicides) having the formula

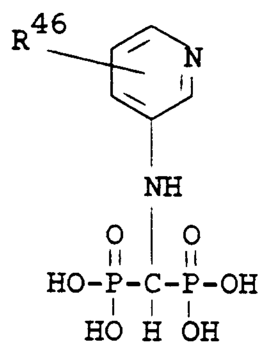

wherein $R^{46}$ is H, halo or alkyl;

tt) bisphosphonates as disclosed in Japanese Patent 55089293 (Nissan Chemical Industries) (disclosed for use as herbicides) having the formula

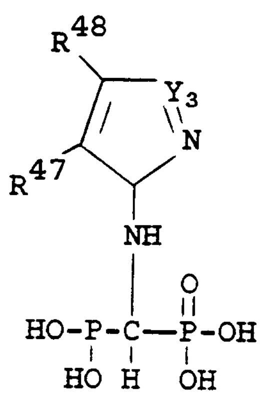

wherein $Y_3$ is O or NH, $R^4$ is H, alkyl or halo, and $R^{48}$ is H or alkyl;

uu) diphosphonic acids as disclosed in Japanese Patent 54144383 (Nissan Chemical Industries) (disclosed for use as herbicides) having the formula

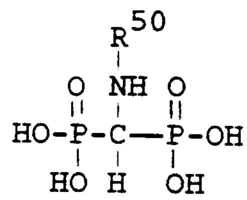

wherein $R^{50}$ is

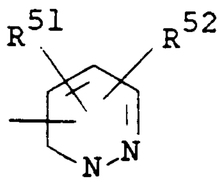

,

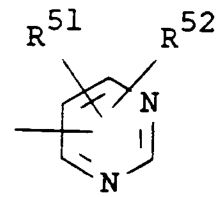

or

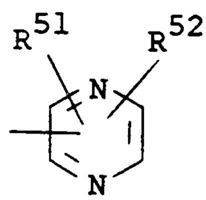

wherein $R^{51}$ and $R^{52}$ are H, halo, alkyl or hydroxy;

vv) diphosphonates as disclosed in Japanese Patent 54037829 (Nissan Chemical Industries) (disclosed for use as herbicides) having the formula

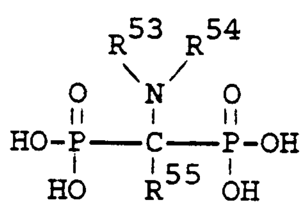

wherein $R^{53}$ is H, alkyl, alkenyl, benzyl or ethylcarbonyloxymethyl,

$R^{54}$ is H, alkyl, alkenyl or cyclohexyl,

$R^{55}$ is H, alkyl, alkenyl or ethylcarbonyloxymethyl;

ww) disphosphonates as disclosed in UK 1508772 and DE 2625767 (Benckiser-Knapsack) (Shell) having the formula

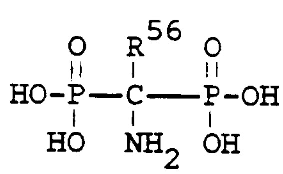

wherein $R^{56}$ is $C_5$-$C_{12}$ alkyl, aryl or aralkyl;

xx) 1-aminoalkylidene-1,1-diphosphonic acids as disclosed in DE 2115737 (Henkel) (disclosed for use as water softeners) having the formula

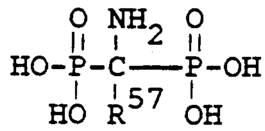

wherein $R^{57}$ is $CH_3$, propyl, n-heptyl, n-$C_{11}H_{23}$, n-$C_{15}H_{31}$, i-propyl;

yy) 1-amino-alkylidenediphosphonic acids as disclosed in DE 2048912 and DE 2048913 (Henkel) (disclosed for use as water softeners) having the formula

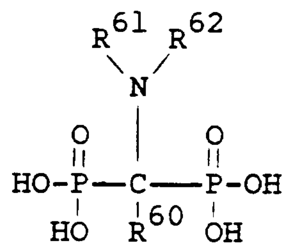

wherein $R^{60}$ is H, $CH_3$, n-nonyl, phenyl, benzyl or $CH_2CO_2H$, $R^{61}$ and $R^{62}$ are H or $CH_3$;

zz) 1-piperidinoalkane-1,1-diphosphonic acids as disclosed in Japanese Patent 53059674 (Nissan chemical Industries) (disclosed for use as herbicides) having the formula

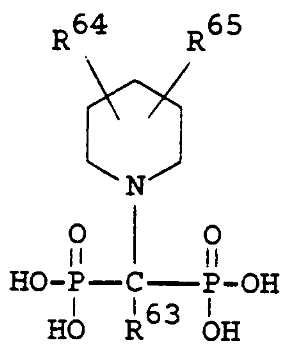

wherein $R^{63}$ is H or $CH_3$,

$R^{64}$ is alkyl, and

$R^{65}$ is H or alkyl;

aaa) heterocyclically substituted alkane-1,1-diphosphonic acids as disclosed in EP 170228A (Boehringer Mannheim) (disclosed for use as anti-inflammatory agents) having the structure

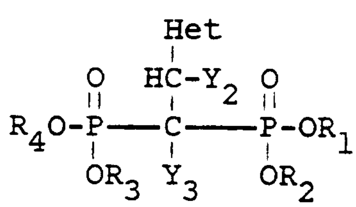

wherein Het is a heteroaromatic 5-membered ring with 2 or 3 heteroatoms, optionally partially hydrogenated and optionally substituted by one or more alkyl, alkoxy, phenyl, cyclohexyl, cyclohexylmethyl, halo or amino, with 2 adjacent alkyl optionally together forming a ring (Het cannot be pyrazole),

$Y_2$ is H or lower alkyl, and

$Y_3$ is H, OH, amino or alkylamino;

bbb) diphosphonic acids as disclosed in U.S. Patent No. 4,687,767 (Boehringer Mannheim) (disclosed for

use in calcium metabolism disturbances) having the formula

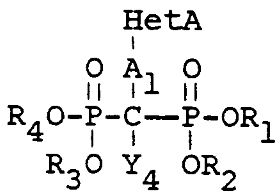

wherein HetA is imidazole, oxazole, isoxazole, thiazole, pyridine, 1,2,3-triazole, 1,2,4-triazole or benzimidazole, the above heterocyclics optionally substituted by alkyl, alkoxy, halo, OH, carboxyl, amino optionally substituted by alkyl or alkanoyl or a benzyl optionally substituted by alkyl, nitro, amino or aminoalkyl, $A_1$ is a straight-chained or branched saturated or unsaturated hydrocarbon chain containing 2 to 8 carbons, $Y_3$ is H, OH, alkanoyl;

bbb') diphosphonic acids as disclosed in U.S. Patent No. 4,666,895 (Boehringer Mannheim) (disclosed for use in calcium metabolism disturbances) having the formula

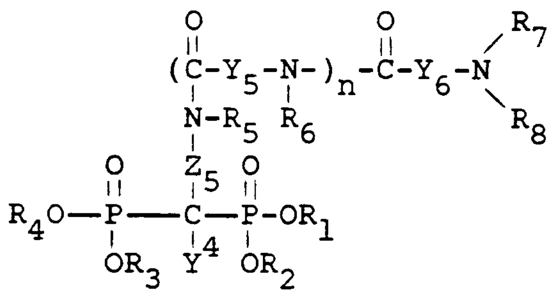

wherein $Y_4$ is H or OH,

$R_1$-$R_8$ are independently H or lower alkyl, whereby $R_7$ and $Y_6$, or $R_6$ and $Y_5$, or $R_5$ and $Z_5$, together with the nitrogen atoms to which they are attached can form a 5- or 6-membered ring,

$Y_6$ and $Y_5$ which can be the same or different are $C_1$-$C_6$ alkylene chains optionally substituted by aromatic or heteroaromatic radicals,

$Z_5$ is $C_1$ to $C_6$ alkylene which can include heteroatoms and optionally substituted by aromatic or heteroaromatic,

n is 0, 1 or 2;

ccc) omega amino-alkane-1,1-diphosphonic acids as disclosed in DE 623397 (Boehringer Mannheim) (disclosed for use in disorders of calcium metabolism) having the formula

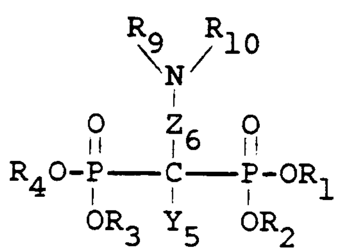

wherein $R_1$-$R_4$ are H or $C_1$-$C_4$ alkyl,

$Z_6$ is $C_1$-$C_6$ alkylene,

$R_9$ is saturated or unsaturated $C_1$-$C_9$ alkyl optionally substituted by phenyl or cyclohexyl,

$R_{10}$ is cyclohexyl, cyclohexylmethyl, benzyl or saturated or unsaturated $C_4$-$C_{18}$ alkyl optionally substituted by phenyl or optionally esterified or etherified OH, and

$Y_5$ is H, OH, or mono- or di($C_1$-$C_6$ alkyl)amino;

ddd) aminocycloalkane diphosphonates as disclosed in DE 3,540,150 (Boehringer Mannheim) (disclosed for use in calcium metabolic disorders) having the structure

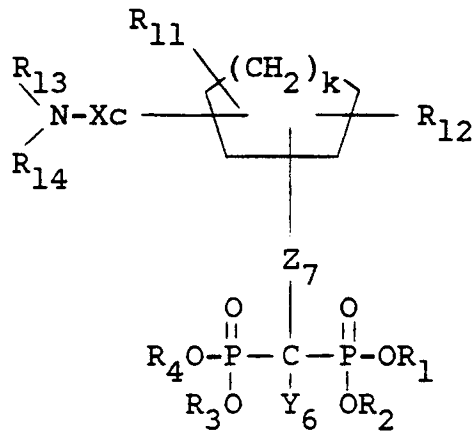

wherein $R_1$-$R_4$ are H or alkyl,

Xc is a bond or alkylene,

$R_{13}$, $R_{14}$ are H, acyl, alkyl or aralkyl, $R_{11}$, $R_{12}$ are H, alkyl or

$R_{11}$, $R_{12}$ are together ($CH_2$),

$Z_7$ is a bond or (amino)alkylene,

$Y_6$ is H, OH, amino and k is 1 to 3;

eee) amino-oxa-alkane-diphosphonic acids as disclosed in DE 822650 (Boehringer Mannheim) (disclosed for use in calcium metabolism disorders) having the structure

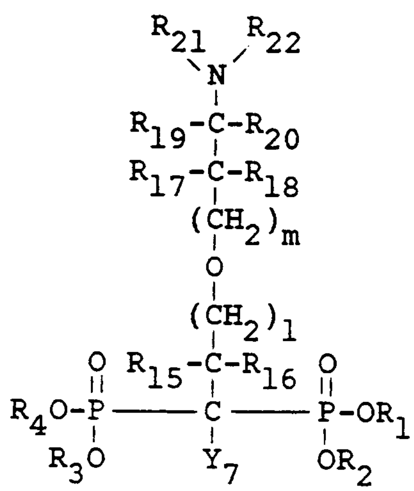

$R_{21}$ and $R_{22}$ are independently H, saturated or unsaturated 1-9C alkyl (optionally substituted by OH, 1-5C alkoxy, 1-5C alkylthio, Aryl or 5-7C cycloalkyl) 5-7C cycloalkyl or phenyl;

Aryl is phenyl optionally substituted by 1-5C alkyl, 1-5C alkoxy, OH or halogen;

$R_{19}$ is H, 1-5C alkyl (optionally substituted by OH, 1-5C alkoxy, 1-5C alkylthio, SH, phenyl, 3-indolyl or 4-imidazolyl), or phenyl optionally substituted by OH or 1-5C alkoxy;

$R_1$-$R_4$, $R_{20}$, $R_{18}$, $R_{16}$ are independently H or 1-5C alkyl;

$R_{15}$ and $R_{17}$ are independently H, 1-5C alkyl, or phenyl optionally substituted by OH or 1-5C alkoxy;

$Y_7$ is H, OH or $NR_{23}R_{24}$;

$R_{23}$ and $R_{24}$ are independently H or 1-5C alkyl;

m and l are independently 0 or 1;

or $NR_{21}R_{22}$ is a 4-9C mono- or bicyclic ring system which is partially or totally hydrogenated and is optionally substituted by OH, 1-5C alkyl or 1-5C alkoxy, where monocyclic rings may also contain an O,

N or S atom;

or $R_{21}$ + $R_{20}$ forms a 5- or 6-membered ring, optionally fused with another 6-membered ring;

or $R_{21}$ + $R_{18}$ forms a 5- or 6-membered ring;

or $R_{20}$ + $R_{19}$, $R_{19}$ + $R_{18}$, $R_{18}$ + $R_{17}$ and/or $R_{15}$ + $R_{16}$ forms a 5- or 6-membered ring.

fff) diphosphonic acids as disclosed in DE 640938 (Boehringer Mannheim) (disclosed for use in calcium metabolism disorders) having the structure

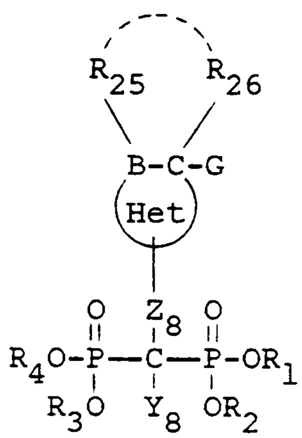

wherein Het is a hydrogenated or partially hydrogenated heterocycle containing 1 or 2 N atoms, each optionally substituted with alkyl, benzyl or cyclohexylmethyl;

B is N or CH and the bond connnecting B to the C atom to which G is attached can be a single or a double bond;

$R_{25}$ and $R_{26}$ are independently H or lower alkyl, or together form a 3-5C alkylene chain, and this ring fused with Het may contain up to 3 double bonds;

$R_1$ to $R_4$ are H or lower alkyl;

$Z_8$ is a single bond or optionally branched 1-6C alkylene which may not be attached to a heteroatom;

$Y_8$ is H, OH or amino;

G is H; and provided that, if $Y_8$ is a single bond, Het is not a pyrrolidine ring which is 2-substituted by $Y_8$;

ggg) methylenediphosphonic acids as disclosed in EP 243173A (Fujisawa Pharm KK) (disclosed for use in abnormal bone metabolism) having the formula

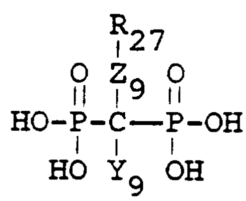

wherein $R_{27}$ is aryl or heterocyclyl both optionally substituted by one or more of lower alkyl, lower alkoxy, lower alkylthio, halo(lower)alkyl, acyl, acylamino or halo or $R_{27}$ is lower alkyl substituted by heterocyclyl which is optionally substituted by acyl;

$R_{27}$-$Z_9$- is $R_{27}$-NHC(=$X_9$)-; $R_{27}$-C(=O)NH-; or $R_{27}$-$SO_2$-NH-;

$X_9$ is O or S;

$Y_9$ is H or lower alkyl, provided that when $R_{27}$ is lower alkyl then $R_{27}$-$Z_9$- is $R_{27}$NHC(X)- or $R_{27}SO_2NH$-;

hhh) diphosphonic acids as disclosed in Japanese Patent 261275 (Fujisawa Pharm) (disclosed for use in bone disorders) having the formula

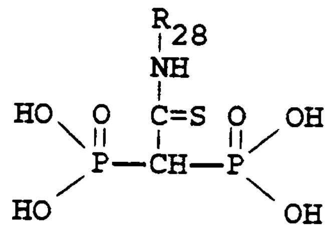

wherein $R_{28}$ is phenyl, pyridyl or quinolyl substituted by lower alkylsulphonylamino halo-lower alkylsulphonylamino and mono- or di-lower alkylamino;

hhh) diphosphonic acids as disclosed in Japanese Patent 259896 (Fujisawa Pharm) (disclosed for use as anti-inflammatory agents) having the formula

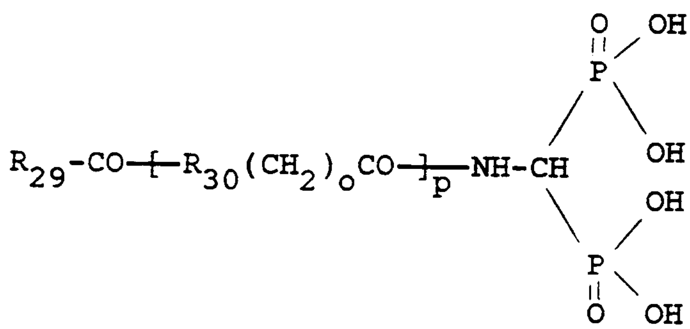

wherein $R_{29}$-CO- is a residue of pharmaceutically active compound $R_{29}$-COOH;

$R_{30}$ = -NH- or -O-;

p = 0 or 1;

o = 1-10;

$R_{29}$-COOH is an anti-inflammatory agent, e.g. diclofenac, ibuprofen, mefenamic acid, aspirin, naproxen, ketoprofen, indomethacin or sulindac; antioncotic, e.g. methotrexate; or hormone, e.g. calcitonin or insulin-like growth factor;

iii) N-aralkyl-amino-alkane-diphosphonic acids as disclosed in EP 371921A (Ciba-Geigy) (disclosed for use in calcium metabolism disorders) having the formula

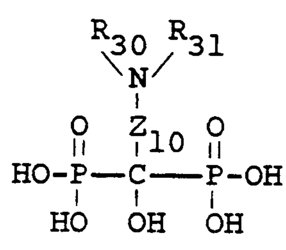

wherein $Z_{10}$ is $C_2$-$C_4$ aliphatic hydrocarbyl (e.g. alkylene),

$R_{30}$ is phenyl substituted $C_4$-$C_7$ aliphatic hydrocarbyl,

$R_{31}$ is $C_1$-$C_4$ aliphatic hydrocarbyl;

jjj) N-aralkylamlno-1-hydroxyalkane-1,1-diphosphonic acids as disclosed in EP 320455A (Ciba-Geigy) (disclosed for use in calcium metabolism disorders) having the formula

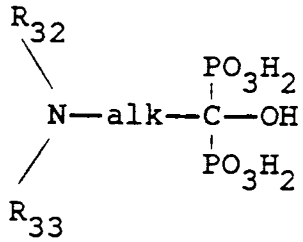

wherein $R_{32}$ ia an aromatically-substituted aliphatic group;

$R_{33}$ is H or monovalent aliphatic group;

alk is a divalent aliphatic group;

provided that when $R_{32}$ is mono-substituted by phenyl, $R_{33}$ is H or when $R_{32}$ has 2 or 3C in the aliphatic portion, $R_{33}$ is an aliphatic group with at most 3C;

kkk) azabicycloalkyl-1-hydroxyalkane-1,1-diphosphonic acids as disclosed in EP 317505A (Ciba-Geigy) (disclosed for use in calcium metabolism disorders) having the structure

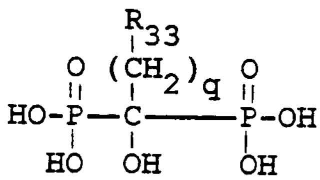

wherein $R_{33}$ is an N-bonded azabicycloalkyl group with 3 to 8-membered rings, such as 3-azabicyclo-(3,1,1,)hept-3-yl, 1,5-dimethyl-3-azabicyclo(3,1,1)-hept-3-yl, 3-azabicyclo(3,2,1)oct-3-yl, 3-azabicyclo-(3,2,2)non-3-yl or 3-azabicyclo(4,2,2)dec-3-yl, and $(CH_2)_q$ is lower alkylene (2 to 4 carbons);

lll) azacycloalkyl-substituted 1-hydroxyalkane-1,1-diphosphonic acids as disclosed in EP 272208A (Ciba-Geigy) (disclosed for use in calcium metabolism disorders) having the formula

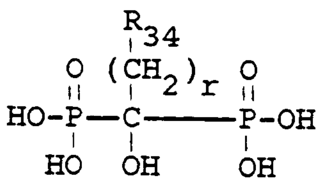

wherein $R_{34}$ is an N-bonded, aryl-substituted mono- or diazacycloaliphatic group, such as 3-$R_{35}$-pyrrolidino, 3- or 4-$R_{35}$-piperidino or 6-$R_{35}$-3-azabicyclo(3,1,1)hept-3-yl, wherein $R_{35}$ is phenyl optionally substituted by $C_1$-$C_4$ alkyl, $C_1$-$C_4$ alkoxy or halogen;

mmm) heteroarylaminomethane diphosphonic acids as disclosed in EP 274346A (Ciba-Geigy) (disclosed for use in calcium metabolism disorders) having the formula

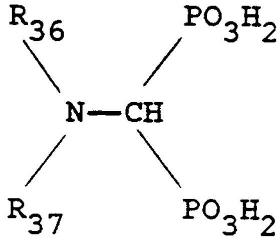

wherein $R_{36}$ is a 5 membered heteroaryl with 2-4 N or with 1-2 N plus an O or S atom, optionally fused to a benzo or cyclohexeno ring;

$R_{36}$ can be C substituted by lower alkyl, phenyl (optionally substituted by lower alkyl, alkoxy and/or halo), lower alkoxy, OH, di(lower alkyl)amino, lower alkylthio and/or halo, and/or N substituted by lower alkyl or phenyl (lower) alkyl (optionally substituted by lower alkyl, lower alkoxy and/or halo);

$R_{37}$ is H or lower alkyl; provided $R_{37}$ is not H if $R_{36}$ is optionally alkyl and/or halo substituted 3-pyrazolyl or 3-isoxazolyl;

examples of $R_{36}$ groups are 2-thiazolyl (optionally substituted by 1 or 2 1-4 C alkyl or phenyl); imidazol-2-yl or benzimidazol-2-yl (optionally 1-substituted by 1-4 C alkyl or phenyl(1-4 C) alkyl); or unsubstituted 2-benzoxazolyl or 2-benzothiazolyl;

nnn) N-substituted aminoalkanediphosphonic acids as disclosed in EP 387194A (Ciba-Geigy) (disclosed for use in calcium metabolism disorders) having the formula

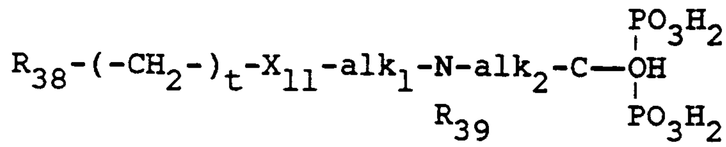

wherein $R_{38}$ is an aromatic residue (such as phenyl, pyridyl or pyrimidyl);

t is 0-3;

$X_{11}$ is O, S (optionally oxidized) or amino (optionally substituted by aliphatic group);

$alk_1$ and $alk_2$ are divalent aliphatic groups;

$R_{39}$ is H or monovalent aliphatic group;

ooo) 2-heteroarylethane-1,1-diphosphonic acids as disclosed in Australian Patent 8781453A (Ciba-Geigy) (disclosed for use in calcium metabolism disorders) having the formula

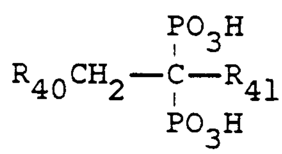

wherein $R_{40}$ is a 5 membered heteroaryl containing either 2-4N atoms or 1-2N atoms and an O or S atom, optionally (a) C-substituted by lower alkyl, aryl, lower alkoxy, OH, di(lower alkyl)amino, lower alkylthio and/or halogen and/or (b) N-substituted by lower alkyl or aryl(lower)alkyl, where aryl in (a) and (b) is phenyl optionally substituted by lower alkyl, lower alkoxy and/or halogen;

$R_{41}$ is H, OH, $NH_2$, lower alkylthio or halogen;

examples of $R_{40}$ groups include imidazolyl, 1,2,4-triazolyl or thiazolyl, optionally C-substituted and/or N-substituted;

ppp) 1-aminoalkane-1,1-diphosphonates as disclosed in U.S. Patent Nos. 3,846,420 and 3,979,385 (Henkel) (disclosed for use as metal ion complexing agents) having the formula

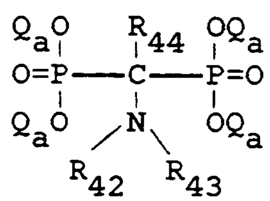

wherein $R_{44}$ is hydrogen, lower alkyl and phenyl; $R_{42}$ and $R_{43}$ are independently hydrogen, alkyl having one to 22 carbon atoms, cycloalkyl having five to six carbon atoms, phenyl, alkylphenyl having seven to 18 carbon atoms, phenylalkyl having seven to 18 carbon atoms and together with the nitrogen atom, piperidino, pyrrolidino and morpholino, and $Q_a$ is hydrogen, alkali metal, ammonium, pyridinium, guanidinium and mono-, di-, and tri-lower-alkanol-ammonium, with the proviso that at least one of $R_{42}$, $R_{43}$ and $R_{44}$ is other than hydrogen;

qqq) 1-aminoalkane-1,1-diphosphonic acids as disclosed in U.S. Patent No. 3,899,496 (Henkel) having the formula

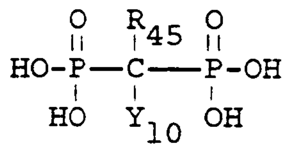

wherein $R_{45}$ represents a hydrogen atom or an alkyl residue with 1 to 12 carbon atoms, a phenyl group, a cyclohexyl group, a phenylalkyl group with 7-12 carbon atoms, a piperidinyl group, a carboxyalkyl group with 2-12 carbon atoms, or a carbalkoxy alkyl group with 3-12 carbon atoms; and

$Y_{10}$ represents an $NH_2$ group, a piperidino group, a morpholino group or a $NR_xR_y$ group, wherein $R_x$ and $R_y$ represent alkyl residues with 1 to 4 carbon atoms;

rrr) aminophosphonic acids as disclosed in U.S. Patent No. 3,303,139 (Henkel) (disclosed for use as metal ion complex formers) having the formula

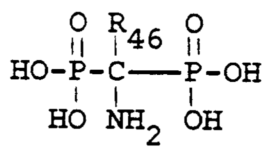

wherein $R_{46}$ is a saturated or unsaturated aliphatic radical having 1-10 carbon atoms or a phenyl- or benzyl-radical;

sss) 3-alkyl-3-oxo-1-amino-propane-1,1-diphosphonic acids as disclosed in DE 3611522A (Henkel) (disclosed for use in inhibiting growth of bacteria) having the formula

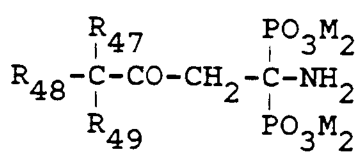

wherein $R_{47}$ is optionally branched 1-8C alkyl;

$R_{48}$ and $R_{49}$ are each methyl or ethyl;

M is H or a cation of a water-soluble base;

ttt) N-heterocyclic propylidene-1,1-bis-phosphonic acids as disclosed in WO 89/09775 (PCT/DK89/00071) (Leo Pharmaceutical Products) (disclosed for use in calcium metabolism disorders) having the formula

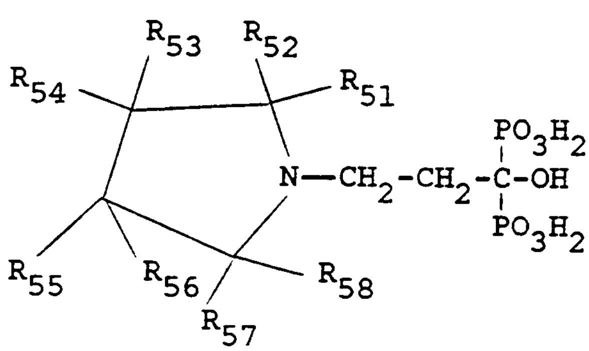

in which $R_{51}$-$R_{58}$ can be the same or different and stand for hydrogen or a straight or branched alphatic $C_1$-$C_{10}$ hydrocarbon radical; and

$R_{53}$ when taken together with either $R_{51}$ or $R_{55}$ can form a saturated aliphatic 5-, 6- or 7-membered ring, which may be substituted with one or more $C_1$-$C_4$-alkyl radicals;

uuu) thiomorpholinylmethylene-bisphosphonic acids as disclosed in WO 8703-598A (Leo Pharmaceuticals) (disclosed for use in reducing bone resorption and stimulating bone alkaline phosphatase) having the formula

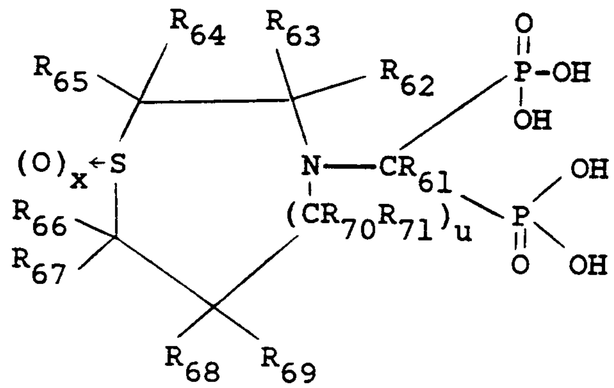

wherein $R_{61}$-$R_{71}$ are independently H, straight, branched or alicyclic 1-10C hydrocarbyl, aryl or aryl-(1-4C)alkyl;

x is 0 or 1;

u is 0, 1 or 2 or

$R_{62}$ + $R_{64}$ may complete a 5- to 7-membered saturated aliphatic ring, optionally substituted by one or more 1-4C alkyl groups;

vvv) 1-aminoalkane-1,1-diphosphonic acids as disclosed in U.S. Patent No. 4,100,167 (Nalco Chemical) (disclosed for use as anti-corrosives) having the formula

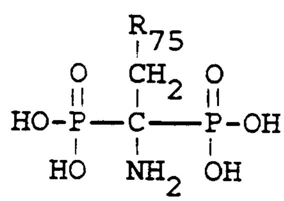

wherein $R_{75}$ is alkyl, aryl, arylalkyl (including phenyl substituted with a sulfonic acid group, halo or pyridyl);

www) heterocycle-substituted diphosphonic acids as disclosed in EP 274158A (Norwich Eaton) (disclosed for use in calcium or phosphate metabolism disorders and as antiplague agents, herbicides) having the formula

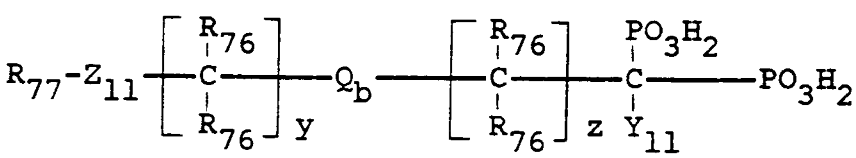

wherein $Z_{11}$ is a N-containing 6-membered ring heterocycle moiety selected from piperidinyl, diazinyl or triazinyl;

$Q_b$ is a covalent bond, O, S or $NR_{76}$;

y, x and y + x are integers of 0-10;

$Y_{11}$ is H, halogen, 1-6C alkyl, phenyl, benzyl, hydroxy or its ester derived from a 1-6C carboxylic

acid, unsubstituted amino or its amide derived from a 1-6C carboxylic acid, amino substituted with one alkyl having 1-6C or its amide derived from a 1-6C carboxylic acid, di(1-6C alkyl)amino, tri(1-6C alkyl)-ammonium, $CO_2H$ or its salts or esters derived from 1-6C alcohols, or its amide optionally substituted with one or two 1-6C alkyl groups, except when n = 0 and $Q_b$ = O, S or N, then $Y_{11}$ is H, 1-6C alkyl, phenyl, benzyl, or $Co_2H$ or its salts or the esters derived from 1-6C alcohols or its amide optionally substituted with one or two 1-6C alkyl groups;

$R_{76}$ is H, methyl, ethyl or propyl;

$R_{77}$ is one or more substituents selected from H, halogen, 1-3C alkyl, unsubstituted amino and its amide derived from a 1-3C carboxylic acid, mono(1-3C alkyl) amino and its amide derived a 1-3C carboxylic acid, di(1-3C alkyl)amino, tri(1-3C alkyl)ammonium, hydroxy or its ester derived from a 1-3C carboxylic acid, ether having 1-3C, $CO_2H$ and its salts and esters derived form 1-3C alcohols, its amide optionally substituted with one or two 1-3C alkyl groups and $NO_2$;

xxx) N-heterocyclyl thio alkane diphosphonic acids as disclosed in EP 230068A (Norwich Eaton) (disclosed for use in calcium and phosphate metabolism disorders) having the structure

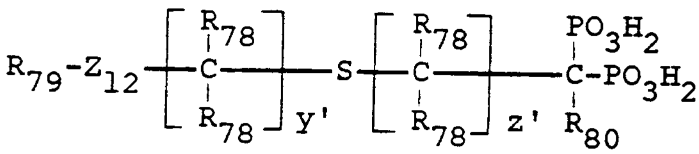

wherein $Z_{12}$ is a 6-membered aromatic ring containing ≧ 1 N atom(s); where:

the ring is optionally substituted by (optionally substituted, optionally unsaturated) 1-6C alkyl, (optionally substituted) aryl, (optionally substituted) benzyl, OH, halogen, carbonyl, alkoxy, $NO_2$, $CONH_2$, (optionally substituted) $NH_2$ and/or carboxylate;

$R_{78}$ is H or (optionally substituted, optionally unsaturated) 1-4C alkyl;

$R_{79}$ is H, (optionally substituted, optionally unsaturated) 1-6C alkyl, (optionally substituted) aryl, (optionally substituted) benzyl, OH, halogen, carbonyl, alkoxy, $NO_2$, $CONH_2$, (optionally substituted) amino or carboxylate;

$R_{80}$ is H, (optionally substituted) $NH_2$, $CONH_2$, OH, alkoxy, halogen, carboxylate, (optionally substituted, optionally unsaturated) 1-6C alkyl;

examples of $Z_{12}$ are pyridine, pyridazine, pyrimidine or pyrazine ring;

y' + z' is 0 to 5;

zzz) cyclic diphosphonic acids as disclosed in EP 304962A (Procter & Gamble) (disclosed for use in calcium and phosphate metabolism disorders) having the formula

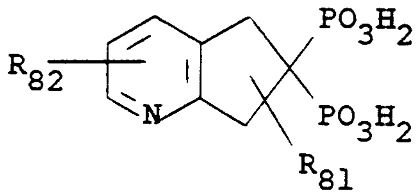

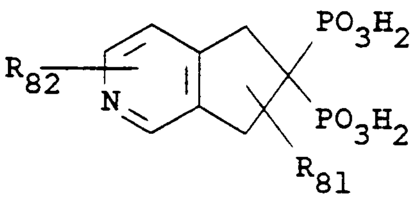

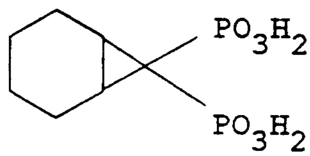

$R_{81}$ and $R_{82}$ are each one or more substituents selected from H, optionally substituted saturated or unsaturated 1-6C alkyl, optionally substituted aryl, optionally substituted benzyl, amimo, OH, halogen, optionally substituted amino, amido, COOH, carbonyl, carboxylate, alkoxy and $NO_2$;

a[4]) heterocyclyl-alkane-diphosphonic acids as disclosed in Australian Patent 8551534A (Procter & Gamble) (disclosed for use in resorption of bone tissue) having the formula

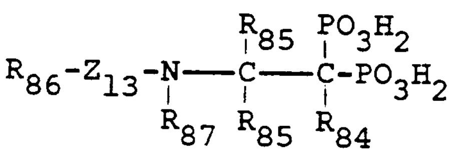

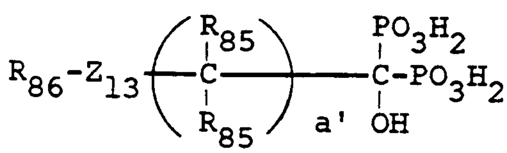

wherein $Z_{13}$ is pyridine, pyridazine, pyrimidine or pyrazine ring; this ring is optionally substituted by optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{84}$ is H, optionally substituted $NH_2$, amido, OH, alkoxy, halogen, carboxylate, optinally substituted optionally unsaturated 1-6C alkyl, optionally substituted aryl or optionally substituted benzyl;

$R_{85}$ is H or optionally substituted optionally unsaturated 1-4C alkyl;

$R_{86}$ is one or more of H, optionally substituted optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{87}$ is H, optionally substituted optionally unsaturated 1-4C alkyl or acyl;

a' is 1-5;

b[4]) geminal diphosphonates as disclosed in EP 186405A (Procter & Gamble) (disclosed for use in calcium and phosphate metabolism disorders) having the strucuture

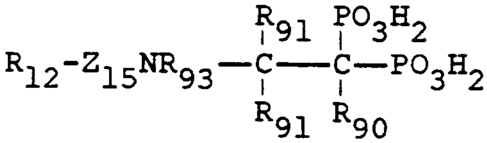

or

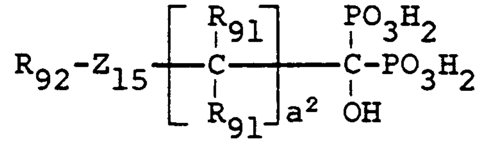

wherein $Z_{15}$ is a 6 membered aromatic ring containing 1 or more N atoms such as pyridine, pyridazine, pyrimidine or pyrazine which ring may be substituted with one or more optionally substituted, optionally unsaturated 1-6C alkyl, optionally substituted aryl, optionally substituted benzyl, OH, halogen, carbonyl, alkoxy, $NO_2$, amido, optionally substituted amino or carboxylate;

$R_{90}$ is H, optionally substituted amino, amido, OH, alkoxy, halogen, carboxylate, optionally substituted, optionally unsaturated 1-6C alkyl, optionally substituted aryl or optionally substituted benzyl;

$R_{91}$ is H or optionally substituted optionally unsaturated 1-4C alkyl;

$R_{92}$ is H or one or more substituents selected from optionally substituted, optionally unsaturated 1-6C alkyl, optionally substituted aryl, optionally substituted benzyl, OH, halogen, carbonyl, alkoxy, $NO_2$, amido, optionally substituted amino or carboxylate;

$R_{93}$ is H, optionally substituted, optionally unsaturated 1-4C alkyl or acyl;

$a^2$ is 1 to 5;

$C^4$) diphosphonic acid derivatives as disclosed in U.S. Patent No. 4,503,049 (Schering A.G.) (disclosed for use as anti-inflammatory agents) having the structure

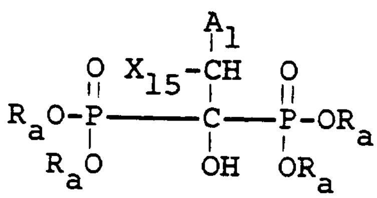

wherein $R_a$ is hydrogen, an alkali metal atom, an alkaline earth metal atom, or alkyl of 1-4 carbon atoms,

$X_{15}$ is hydrogen, methyl, or ethyl, and

$A_1$ is phenyl substituted in the para-position by isobutyl, cyclohexyl, alkoxy, or 1-pyrrolinyl and, optionally substituted additionally in the meta-position by fluorine or chlorine, or phenyl substituted in the meta-position by benzoyl or phenoxy, or phenyl substituted in the ortho-position by 2,4-dichlorophenoxy or 2,6-dichlorophenylamino;

diphosphonic acid derivatives of the sturcture

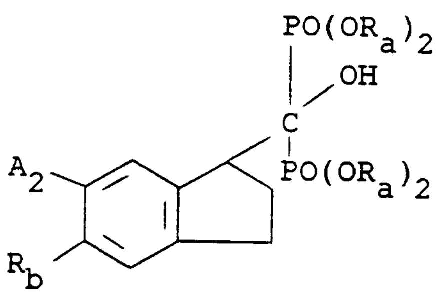

wherein $R_a$ is as defined above;

$R_b$ is cyclohexyl or cyclopentylmethyl; and

$A_2$ is hydrogen or chlorine;

diphosphonic acid derivatives of the formula

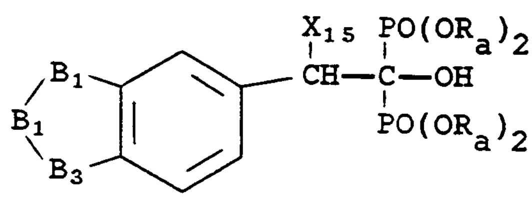

wherein $R_a$ and $X_{15}$ are as defined above, and

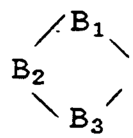 is

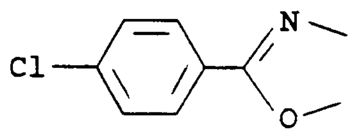 ; 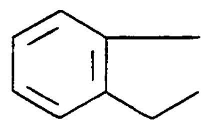

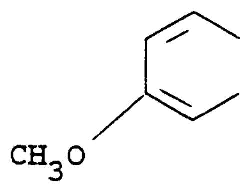 or 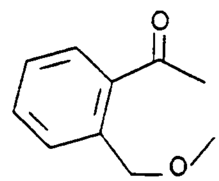 ;

diphosphonic acid derivatives of the formula

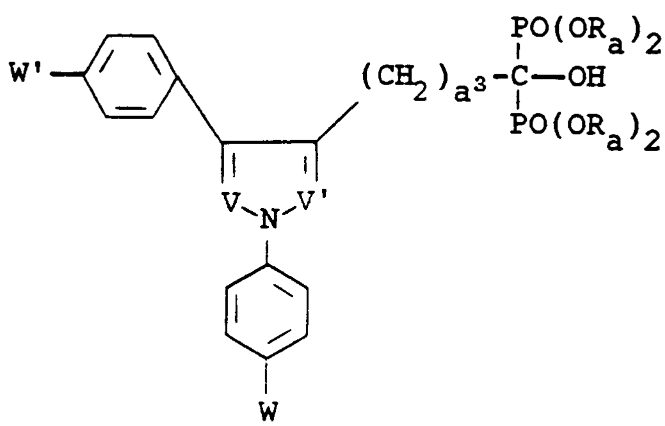

wherein $a^3$ is 1, 2 or 3,

$R_a$ is as defined above,

W and W' are identical or different, and each is hydrogen, fluorine or chlorine, and

one of V and V' is nitrogen and the other is a methyne residue optionally substituted by a phenyl group; and diphosphonic acid derivatives of the formula

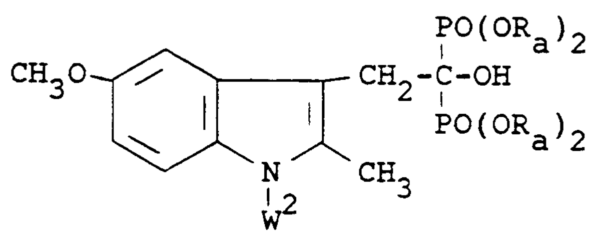

wherein $R_a$ is as defined above, and

$W^2$ is p-chlorobenzoyl or cinnamoyl; or throughout, when $R_a$ is H, a physiologically acceptable salt

thereof with an organic base;

d[4]) methylenediphosphonic acids as disclosed in U.S. Patent No. 4,876,247 (Sanofi) (disclosed for use as an antirheumatic) having the formula

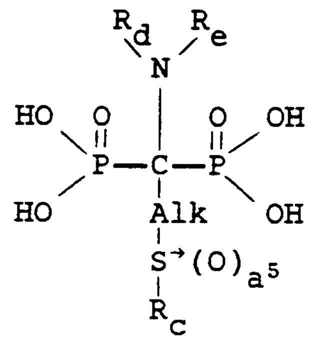

wherein $R_c$ represents:

a $C_1$-$C_6$ alkyl group,

a $C_5$-$C_7$ cycloalkyl group,

a phenyl group optionally monosubstituted or poly-substituted by a halogen, a $C_1$-$C_6$ alkyl group or a trifluoromethyl group, or

a 5-membered or 6-membered heterocycle containing 1 or 2 heteroatoms chosen from nitrogen and sulfur,

Alk denotes a linear or branched $C_1$-$C_6$ alkylene group,

$R_d$ represents hydrogen, a $C_1$-$C_6$ alkyl group or a -$CONH_2$ group,

$R_e$ represents hydrogen, a $C_1$-$C_6$ alkyl group, a benzyl group or a phenyl group optionally substituted by chlorine or methyl groups; or alternatively

$R_d$ and $R_e$ taken together, represent a

$$(CH_2)_{a^4}$$

group, in which $a^4$ is 4 or 5, and

$a^5$ represents 0 or the integer 1 or 2.

e[4]) aminomethylene-bisphosphonic acids as disclosed in EP 337706A (Yamanouchi Pharm) (disclosed for use in inhibiting bone resportion, and for its anti-inflammatory, antirheumatic and analgesic activities) having the structure

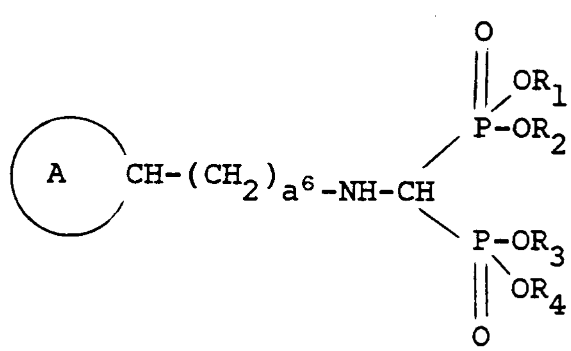

wherein $R_1$-$R_4$ is H or 1-5C alkyl;

$a^6$ is 0-4;

Ring A is 5-8-cycloalkenyl, bicycloheptyl, bicycloheptenyl or 4-7C saturated heterocyclyl containing O, S, SO or $SO_2$;

f[4]) diphenylazolediphosphonic acids as disclosed in Japanese Patent 210445 (Yamanouchi Pharm)

(disclosed for use as anti-inflammatory, antipyretic and analgesic agents) having the structure

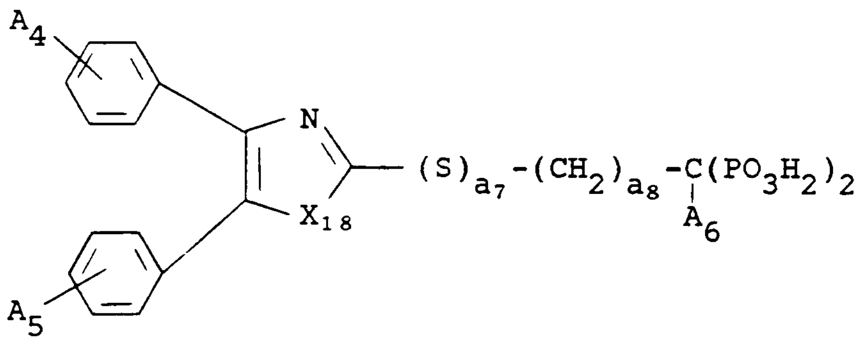

wherein $A_4$ and $A_5$ are the same or different and are H, OH, lower alkoxy or halogen;

$A_5$ is H or OH:

$X_{18}$ is O, S or NH;

$a_7$ is 0 or 1;

$A_5$ is 0 or an integer of 1-6;

g[4]) (pyrazolylamino)methylene-bis-(phosphonic acids) as disclosed in Japanese Patent 086857 (Yamanouchi Pharm) (disclosed for use as bone resorption inhibitors) having the formula

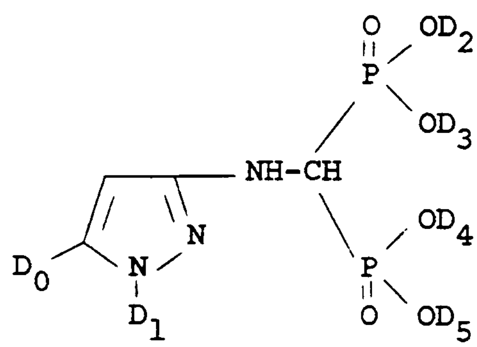

(wherein $D_0$ is H or alkyl;

$D_1$ to $D_5$ is H or lower alkyl);

h[4]) isoxazolyl-containing bisphosphonic acids as disclosed in EP 282320A (Yamanouchi Pharm) (disclosed for use as bone resorption inhibitors and in arthritis) having the formula

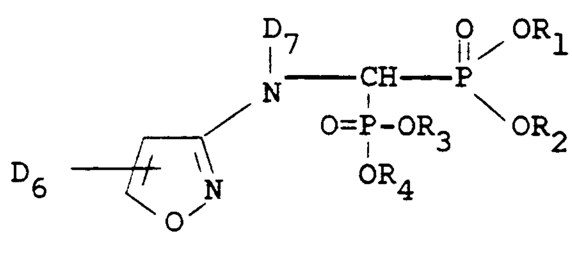

wherein $D_6$ is H, 1-10C alkyl, 3-10C cycloalkyl, phenyl, 2-10C alkenyl (optionally substituted by phenyl) or phenyl(1-5C)alkyl (optionally ring-substituted by a 1-5C alkoxy);

$D_7$ is H or 2-6C alkanoyl; and

$R_1$-$R_4$ is H or 1-5C alkyl;

provided that when $D_6$ is methyl, ethyl, isopropyl or tert-butyl, at least one of $D_7$, $R_1$, $R_2$, $R_3$ or $R_4$ is other than H;

i[4]) azole-amino methylene bisphosphonic acids as disclosed in EP 282309A (Yamanouchi Pharm)

(disclosed for use as bone resorption inhibitors) having the sturcture

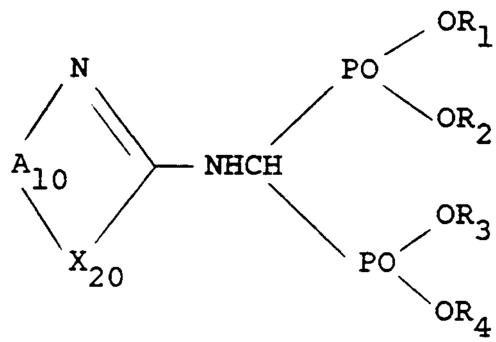

wherein $A_{10}$ = a group of formula (a)-(C):

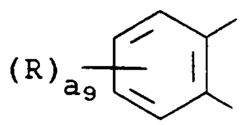

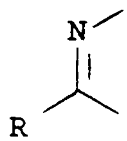

or

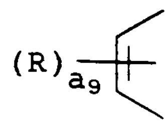

(a) (b) (c)

R is H, halogen, 1-5C alkyl or phenyl;
$a_9$ is 1 or 2;
$X_{20}$ is O, S or NH;
$R_1$-$R_4$ is H or 1-5C alkyl;

j[4]) cycloalkyl:amino-methylene-bis:phosphonic acids having the formula

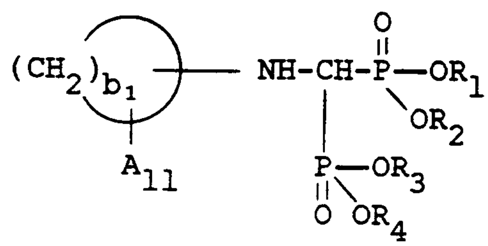

wherein $A_{11}$ and $R_1$ - $R_4$ are H or 1-5C alkyl;
$b_1$ is 3-10; provided that $A_{11}$ is 1-5C alkyl when $R_1$ - $R_4$ is H and $b_1$ is 5 or 6;

k[4]) heterocyclic bisphosphonic acids as disclosed in EP 354806A (Yamanouchi Pharm) (disclosed for use in bone resorption) having the formula

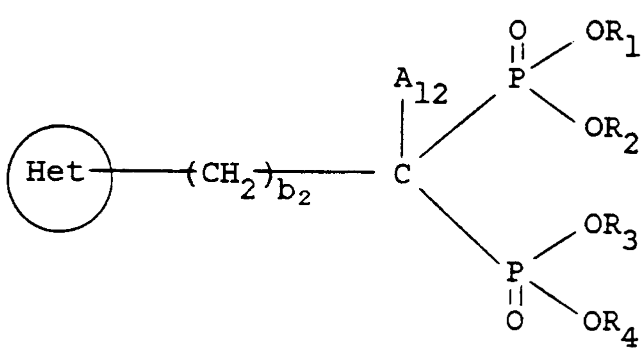

wherein ring Het is a group of formula (A) or (B):

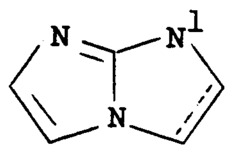

(A)

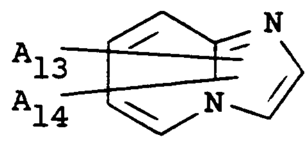

(B)

the dotted line represents optionally double bond;

$A_{13}$, $A_{14}$ are independently H, 1-5C alkyl, halogen or OH;

$A_{12}$ is H or OH;

$R_1$, $R_2$, $R_3$, $R_4$ are H or 1-5C alkyl;

$b_2$ is 0 or 1; provided that $b_2$ is 1 when ring Het is (A); and

$A_{12}$ is OH when ring Het is (B).

$l^4$) imidazo- or pyrrolo-pyridine substituted bisphosphonic acids as disclosed in Japanese Patent 200462 (Yamanouchi Pharm) (disclosed for use as bone resorption inhibitors) having the structure

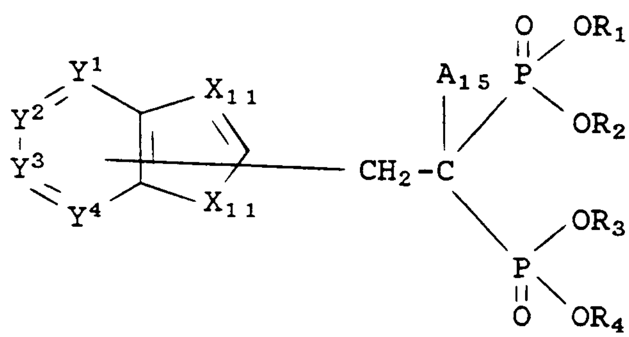

wherein $A_{15}$ is H or OH;

$R_1$-$R_4$ is H or lower alkyl;

$X_{11}$ is both N or one is N and the other is CH;

one of $Y^1$-$Y^4$ is N and the rest are CH.

In another aspect of the present invention, a pharmaceutical composition is provided for inhibiting cholesterol biosynthesis and thereby reducing plasma (or serum) cholesterol by inhibiting the enzyme squalene synthetase wherein bisphosphonate compounds as described above are employed or compounds as disclosed in U.S. Patent Nos. 4,309,364 and 4,416,877 and UK Patent 2,079,285A, all to Bentzen et al and assigned to Symphar S.A. (hereinafter referred to as the Symphar patents) are employed. Thus, the pharmaceutical compositions of the invention inhibit squalene synthetase by containing compounds disclosed in the Symphar patents including diphosphonates of the formula

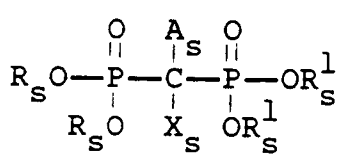

wherein $R_s$ and $R_s^1$ are the same or different and are H, methyl or ethyl, wherein at most only one of $R_s$ and $R_s^1$ is methyl or ethyl;

$X_s$ is H, OH, $OCOCH_3$ or $NH_2$;

$A_s$ is t-butyl; $Y_s$-$C_6H_4$-; $Y_s$-$C_6H_4$-O-$C(CH_3)_2$-; $Y_s$-$C_6H_4$-$C(CH_3)_2$-; $Y_s$-$C_6H_4$-CO-$C_6H_4$; $Y_s$-$C_6H_4$-$(CH_2)_{ns}$- and $Y_s$-$C_6H_4$-O-$(CH_2)_{ns}$-

where ns is 1 to 6 and

$Y_s$ is H, $CH_3$, halogen, $OC_{1-20}$ alkyl.

It will be appreciated that $C_6H_4$ in the above groups represents a phenylene group.

Examples of compounds of the Symphar type suitable for use in the preparation of the pharmaceutical compositions for inhibiting squalene synthetase include

tetramethyl 1(p-chlorophenyl)methane-1-hydroxy 1,1-diphosphonate,
tetramethyl 2,2-dimethyl 2-(p-chlorophenoxy)ethane 1-hydroxy-1,1-diphosphonate,
tetramethyl 1-[4(4'-chlorobenzoyl)phenyl]-methane 1-hydroxy 1,1-diphosphonate,
dimethyl 1[(dimethoxyphosphinyl)p-chlorobenzyl]phosphate,
dimethyl [1(dimethoxyphosphinyl) 2,2-dimethyl 2-phenyl]-ethyl phosphate,
dimethyl [1(dimethoxyphosphinyl)2,2-dimethyl 2(p-chlorophenyl] ethyl phosphate,
tetramethyl 4-phenylbutylidene, 1,1-diphosphonate.

More preferred are compounds of formula I wherein one of $R^5$ and $R^6$ is a lipophilic group which is optionally substituted alkyl, optionally substituted alkenyl or optionally substituted aryl, and the other of $R^5$ and $R^6$ is hydrogen, lower alkyl or halogen.

In the pharmaceutical compositions of the invention bisphosphonate compounds are used which inhibit cholesterol biosynthesis by inhibition of de novo squalene production. These bisphosphonate compounds (which are described in detail hereinbefore) inhibit the squalene synthetase enzyme and, in addition, some of these compounds inhibit other enzymes in the pathway from isopentenyl diphosphate to squalene, that is, farnesyl diphosphate synthetase and isopentenyl diphosphate-dimethylallyl diphosphate isomerase.

Thus, the pharmaceutical compositions of the invention are useful in treating atherosclerosis to inhibit progression of disease and in treating hyperlipidemia to inhibit development of atherosclerosis. In addition, they may be employed to increase plasma high density lipoprotein cholesterol levels.

Inasmuch as the pharmaceutical compositions of the invention employ squalene synthetase inhibitors, they may also be useful in inhibiting formation of gallstones, treating tumors, lowering blood pressure, lowering blood sugar, treating diabetes mellitus, treating inflammation, for diuretic therapy, for inotropic therapy, for anti-arthritic (antirheumatic) therapy, in treating other diseases of calcium and phosphate metabolism including treatment of bone resorption, Paget's disease, osteoporosis, calcification of joints, implants and metastasis, as antitartar and anti-calculus therapy by means of toothpastes and mouthwashes, treating various stones and calculi, treating sickle cell anemia, treating hypoxia and ischemic tissue, and as anti-ameobal therapy, as well as in carrying out diagnostic techniques wherein the squalene synthetase inhibitors are employed in complexes with technetium-99m and radioiodinated derivatives.

The pharmaceutical compositions of the invention may also be employing the bisphosphonate squalene synthetase inhibitor in combination with an anti-hyperlipoproteinemic agent such as probucol and/or with one or more serum cholesterol lowering agents such as Lopid (gemfibrozil), bile acid sequestrants such as cholestyramine, colestipol, polidexide (DEAE-Sephadex) as well as clofibrate, nicotinic acid and its derivatives, neomycin, p-aminosalicyclic acid, bezafibrate and the like and/or one or more HMG CoA reductase inhibitors such as lovastatin, pravastatin, velostatin or simvastatin.

The above compounds to be employed in combination with the squalene synthetase inhibitor of the invention may also be used in amounts as indicated in the Physicians' Desk Reference (PDR).

The compounds employed in the pharmaceutical compositions of the invention may also be employed with sodium lauryl sulfate or other pharmaceutically acceptable detergents to enhance oral bioavailability of such compounds.

Inhibition of squalene synthetase may be measured by the following procedure.

Rat liver microsomal squalene synthetase activity is measured using farnesyl diphosphate as substrate and quantitating squalene synthesis using gas chromatographic analysis. The assay was developed by modifying conditions originally described by Agnew (Methods in Enzymology 110:357, 1985).

Preparation of Rat Liver Microsomes:

Livers are dissected from 2 or 3 decapitated Sprague Dawley rats and are quickly transferred to ice cold buffer (potassium phosphate, 0.05 M, (pH 7.4); $MgCl_2$, 0.004 M; EDTA, 0.001 M; and 2-mercaptoethanol 0.01 M) and rinsed thoroughly. The livers are minced in cold buffer (2.0 ml/g) and homogenized using a Potter-Elvejhem homogenizer. The homogenate is centrifuged at 5,000 x g, 10 minutes (4°C), and the supernatant poured through 2 layers of cheese cloth. The supernatant is then centrifuged at 15,000 x g for 15 minutes (4°). Again the supernatant is filtered through 2 layers of cheese cloth, and centrifuged a third time at 100,000 x g for 1.0 hour at 4°C. Following centrifugation the microsomal pellet is resuspended in a volume of buffer equivalent to 1/5 the volume of the original homogenate, and homogenized in a ground

glass homogenizer. Aliquotted microsomes are frozen at -80°C, and retain activity for at least two months.

Enzyme Assay:

Reaction Mixtures are prepared in 50 ml round bottom pyrex glass tubes with tight-fitting, teflon-lined, screw caps. Tubes are cooled to 4°C, and the following components are added in sequence:

| | |
|---|---|
| 1. Potassium phosphate buffer (0.275 M, pH 7.4) | 0.36 ml |
| 2. KF (55 mM) | 0.36 ml |
| 3. NADPH (5.0 mM, freshly prepared) | 0.36 ml |
| 4. $H_2O$ (or $H_2O$ + test compound) | 0.16 ml |
| 5. $MgCl_2$ (27.5 mM) | 0.36 ml |
| 6. Microsomal Enzyme (0.48 mg microsomal protein in homogenization buffer) (15 µl prep.) 4/23/86 | 0.20 ml |
| | 1.8 ml |

This mixture is equilibrated under $N_2$ at 4°C for 5-15 minutes. Reaction mixtures are then warmed to 30°C, and the enzyme reaction initiated by adding 0.2 ml of farnesyl pyrophosphate (219 µM) prepared in $H_2O$. Each tube is again overlayered with $N_2$, and incubated at 30°C for 60 minutes. The reaction is stopped by the addition of 1.0 ml KOH (40%). Ethanol (95%) (spectral grade) (1.0 ml) is added to each tube. Docosane (5 nmoles in hexane) is added to each tube as an internal standard. The mixture is saponified at 65°C for 30 minutes. The tubes are cooled to room temperature and extracted twice with 10.0 ml spectral grade hexane.

The upper organic phase fractions are pooled in glass 20.0 ml scintillation vials and reduced in volume to ≃ 1.0 ml under a stream of $N_2$. The sample is then transferred to acid-washed, conical bottom, glass (1.0 ml) microvials, and brought to dryness under $N_2$. The residue is resuspended in 50 µl hexane (spectral grade), and these samples are spun at 1000 rpm at room temperature for 10 minutes. Following centrifugation approximately 40 µl of supernatant is transferred to 100 µl acid-washed microvials with septa/crimp-top caps (compatible with the Hewlett-Packard GC auto injector).

Gas Chromatography:

Two µL of each sample is injected onto a fused silica megabore DB-17 column (15 M x 0.525 mm) (J&W Scientific) using a splitless mode of injection. Gas flow rates are listed below:

| | |
|---|---|
| Make up gas (helium) | 20 ml/min. |
| Air | 400 ml/min. |
| Hydrogen | 30 ml/min. |
| Carrier (helium) | 15 ml/min. |
| Septum purge vent | 5 ml/min. (Septum purge off 0.00 min., on at 0.5 min.) |

The injector temperature is 200°C, and the FID detector temperature is set at 270°C. Oven temperature is programmed through a two ramp sequence as follows:

Oven:

Initial temperature 180°C, initial time 10 minutes Ramp one: 20°C/minute

Second temperature 250°C, second time 10 minutes Ramp two: 20°C/minute

Third temperature 260°C, third time 10 minutes (Equilibration time 1.0 minute)

Using this gas chromatographic system, docasane (internal standard) has a retention time of 3.6-3.7 minutes, and squalene has a retention time of 14.7-14.9 minutes. The amount of squalene in each reaction mixture is determined by obtaining the areas under the squalene and docasane peaks and using the following formula to calculate the amount of squalene (nmoles) in the total reaction mixture.

$$\text{Squalene (nmoles/reaction mixture)} = 5.0 \text{ (nmoles docasane internal standard)} \times \frac{\text{Squalene Peak Area}}{\text{Docasane Peak Area}} \times RR^*$$

*RR = Response Ratio [Docasane/Squalene]

*RR = 0.56

Compounds Testing:

Compounds are dissolved in $H_2O$ and added to reaction mixtures prior to addition of farnesyl pyrophosphate substrate. All reaction mixtures are run in duplicate, at several concentrations. Additionally, all compound $I_{50}$ values are derived from composite dose response data.

In carrying out the invention, a pharmaceutical composition will be employed containing at least one bisphosphonate squalene synthetase inhibitor in association with a pharmaceutical vehicle or diluent. The pharmaceutical compostion can be formulated employing conventional solid or liquid vehicles or diluents and pharmaceutical additives of a type appropriate to the mode of desired administration. The compounds can be administered to mammalian species including humans, monkeys, dogs, etc. by an oral route, for example, in the form of tablets, capsules, granules or powders, or they can be administered by a parenteral route in the form of injectable preparations. The dose for adults is preferably between 200 and 2,000 mg per day, which can be administered in a single dose or in the form of individual doses from 1-4 times per day.

A typical capsule for oral administration contains bisphosphonate squalene synthetase inhibitor (250 mg), lactose (75 mg) and magnesium stearate (15 mg). The mixture is passed through a 60 mesh sieve and packed into a No. 1 gelatin capsule.

A typical injectible preparation is produced BY asceptically placing 250 mg of sterile bisphosphonate squalene synthetase inhibitor into a vial, asceptically freeze-drying and sealing. For use, the contents of the vial are mixed with 2 ml of physiological saline, to produce an injectible preparation.

The following Examples illustrate the preparation of preferred bisphosphonate compounds which may be employed in the pharmaceutical compositions of the invention for inhibiting cholesterol biosynthesis by inhibiting de novo squalene production.

All temperatures are reported in degrees Centigrade.

$^1H$ and $^{13}C$ chemical shifts are reported as $\delta$-values with respect to $Me_4Si$ ($\delta = 0$). $^{31}P$ spectra were measured on a JEOL FX90Q FT-NMR spectrometer, at 36.2 MHz, utilizing the $^1H$ decoupled mode. The $^{31}P$ data were obtained using 85% $H_3PO_4$ as an external reference ($\delta = 0$). Coupling constants J are reported in Hz. Chemical ionization mass spectra (CI-MS) were determined with a Finnigan TSQ-4600 instrument equipped with a direct exposure probe using the indicated reagent gases. Fast atom bombardment mass spectra (FAB-MS) were recorded on a VG Analytical ZAB-2F spectrometer. Ions were sputtered (8keV Xe) from a matrix containing dithiothreitol, dithioerythritol, DMSO, glycerol and water.

All reactions were carried out under an atmosphere of dry argon or nitrogen. The following reagents and solvents were distilled prior to use from the indicated drying agents, where applicable: $CH_2Cl_2$, 2,4,6-collidine, and diisopropylamine ($CaH_2$); THF and diethyl ether (K, benzophenone); N,N-diethyltrimethylsilylamine and oxalyl chloride. Benzene was passed through neutral alumina (activity I) and stored over 4A-molecular sieves. Lithium bromide was dried at 100°C over $P_2O_5$.(E,E)-Farnesol was purchased from Aldrich Chemical Company.

TLC was performed on E. Merck Silica Gel 60 F-254 plates (0.25 mm) or E. Merck Cellulose F plates (0.1 mm). Flash chromatography was carried out using E. Merck Kieselgel 60 (230-400 mesh).

Reverse-phase chromatographic purification of salts or mixed ester-salts was carried on CHP20P gel or SP207SS gel, a highly porous, polystyrene-divinyl benzene copolymers available from Mitsubishi Chemical Industries. The indicated general procedure was followed: An FMI Model RP-SY pump was utilized for

solvent delivery. A column of CHP20P (2.5 cm diameter, 12-22 cm height) was slurry packed and washed with water (500-1000 mL), and a basic, aqueous solution of the crude salt was applied to the top of the column. Typically, the column was eluted with water, followed by a gradient composed of increasing concentrations of acetonitrile or methanol in water. The gradient was created by placing the tip of a tightly stoppered separatory funnel containing 300-500 mL of the organic solvent, or an aqueous-organic mixture, just beneath the surface of a reservoir containing 300-500 mL of pure water. To start the gradient, the stopcock of the separatory funnel was opened, so that as the solvent was withdrawn by the pump from the reservoir, it was replaced with the solvent from the separatory funnel. HPLC-grade solvents and Lectrostill steam distilled water were employed. Fractions were collected (10-15 mL each) at a flow rate of 5-10 mL per minute. Those fractions that contained pure product as judged by TLC were pooled, the organic solvents were evaporated and the aqueous residue was lyophilized to dryness.

Example 1

(Undecylidene)bisphosphonic acid, trisodium salt

A. (Undecylidene)bisphosphonic acid, tetraethyl ester

A suspension of 0.29 g (12.07 mmol) of NaH in 13 mL of dry DMF at 0 °C under argon was treated with 3.45 g (11.98 mmol) of tetraethyl methylenediphosphonate over 0.3 hours to give a yellow solution. The reaction was allowed to warm to room temperature and stir for 0.5 hours when 2.70 g (10.06 mmol) of 1-iododecane (Aldrich Chemical) was added in one portion. The reaction mixture was stirred for 12 hours at room temperature when the reaction was quenched with 7.0 mL of water. The resulting emulsion was diluted with water (100 mL) and extracted with ethyl acetate (200 mL). The organic layer was washed with brine, dried over $Na_2SO_4$ and concentrated to provide a thick oil. The oil was purified by flash chromatography on 250 g of silica gel packed, loaded, and eluted with ethyl acetate (500 mL), followed by 1.5 L of 1:9 ethanol/ethyl acetate to provide 2.40 g (56%) of title ester as a pale yellow oil.
TLC Silica gel (1:9 ethanol:ethyl acetate) $R_f$ = 0.50.
IR ($CCl_4$ film) 2980, 2926, 2854, 1445, 1251, 1028, 968 $cm^{-1}$.
$^1H$ NMR ($CDCl_3$, 270 MHz): $\delta$ 4.12 (m, 8H), 2.30 (tt, 1H, J = 24.2, 6.0 Hz), 1.90 (m, 2H), 1.57 (m, 2H), 1.34 (t, 12H, J = 7.0 Hz), 1.26 (m, 14H), 0.88 (t, 3H, J = 6.5 Hz) ppm.
Mass Spec. (CI, $NH_3$, + ions) m/e 429 (M + H).

B. (Undecylidene)bisphosphonic acid, trisodium salt

To a stirred solution of 1.00 g (2.33 mmol) of Part A ester in 10 mL of dichloromethane at 0 °C was added 1.70 mL (11.68 mmol) of iodotrimethylsilane. The reaction was allowed to stir at room temperature for 12 hours when the solvent was evaporated and the residue pumped (~ 1 mm pressure) for 0.5 hour. The remainder was dissolved by adding 21 mL (10.5 mmol) of 0.5 N NaOH solution and freeze dried. The crude white solids were purified by MPLC on a column of CHP20P gel (700 mL of gel) eluting with water (1.5 L). The soapy fractions were combined, reduced to a 125 mL volume and filtered through a nylon membrane (pore size 0.2 µm). The filtrate was lyophilized to provide 0.56 g (63%) of title compound as a white lyophilate.
IR (KBr) 3668, 3375, 2957, 2924, 2872, 1641, 1468, 1155, 1107, 886 $cm^{-1}$.
$^1H$ NMR ($D_2O$, 400 MHz): $\delta$ 1.70 (m, 3H), 1.45 (m, 2H), 1.25 (m, 14H), 0.79 (t, 3H, J = 6.9 Hz) ppm.
Mass Spec. (FAB) m/e 405 (M + Nz), 383 (M + H), 365 (M-$H_2O$ + H), 361 (M-Na + 2H).

| Anal. Calc'd for $C_{11}H_{23}O_6Na_3P_2$ + 1.00 $H_2O$ | | | |
|---|---|---|---|
| | C, 33.01; | H, 6.30; | P, 15.48 |
| Found: | C, 32.83; | H, 6.26; | P, 15.20. |

Example 2

(Pentadecylidene)bisphosphonic acid, tripotassium salt

A. (Pentadecylidene)bisphosphonic acid, tetraethyl ester

A suspension of 0.26 g (11.0 mmol) of NaH in 12 mL of dry DMF at 0°C under argon was treated with 3.00 g (11.00 mmol) of tetraethyl methylenediphosphonate over 0.3 hours to give a yellow solution. The reaction was allowed to warm to room temperature and stir for 0.5 hours when 2.77 g (10.00 mmol) of 1-bromotetradecane (Aldrich Chemical) was added in one portion. The reaction mixture was stirred for 12 hours at room temperature when the reaction was quenched with 7.0 mL of water. The resulting emulsion was diluted with water (100 mL) and extracted with ethyl acetate (200 mL). The organic layer was washed with brine, dried over $Na_2SO_4$ and concentrated to provide a thick oil. The oil was purified by flash chromatography on 250 g of silica gel packed, loaded and eluted with ethyl acetate (500 mL) followed by 1.5 L of 1:9 ethanol/ethyl acetate to provide 2.70 g (56%) of title ester as a pale yellow oil.
TLC Silica gel (1:9 ethanol:ethyl acetate) $R_f = 0.50$.
IR ($CCl_4$ film) 2978, 2924, 2854, 1467, 1445, 1251, 1165, 1098, 1028, 968 $cm^{-1}$.
$^1H$ NMR ($CDCl_3$, 270 MHz): $\delta$ 4.18 (m, 8H), 2.26 (tt, 1H, J = 24.2, 5.9 Hz), 1.90 (m, 2H), 1.57 (m, 2H), 1.34 (t, 12H, J = 7.0 Hz), 1.26 (m, 22H), 0.88 (t, 3H, J = 6.2 Hz) ppm.
Mass Spec. (CI-$NH_3$, + ions) m/e 502 (M + $NH_4$), 485 (M + H).

B. (Pentadecylidene)bisphosphonic acid, tripotassium salt

To a stirred solution of 2.40 g (4.95 mmol) of Part A ester in 10 mL of dichloromethane at 0°C was added 3.30 mL (25.0 mmol) of bromotrimethylsilane. The reaction was allowed to stir at room temperature for 12 hours when the solvent was evaporated and the residue pumped (~ 1 mm pressure) for 0.5 hours. The remainder was dissolved by adding 15 mL (15.0 mmol) of 1 N KOH solution. The crude solution was purified by MPLC on a column of CHP20P gel (700 mL of gel) eluting with water (1.0 L) followed by 20% acetonitrile in water (2.0 L). The pure fractions were combined, reduced to a 225 mL volume and filtered through a nylon membrane (0.2 $\mu$m pore size). The filtrate was lyophilized to provide 1.71 g (71%) of title salt as a white lyophilate.
IR (KBr) 3668, 3136, 2920, 2852, 1641, 1467, 1114, 868 $cm^{-1}$.
$^1H$ NMR ($D_2O$, 400 MHz): $\delta$ 1.70 (m, 3H), 1.45 (m, 2H), 1.25 (m, 22H), 0.77 (t, 3H, J = 6.7 Hz) ppm.
Mass Spec. (FAB, + ions) m/e 525 (M + K), 487 (M + H), 469 (M-$H_2O$ + H), 449 (M-K + 2H).

| Anal. Calc'd for $C_{15}H_{31}O_6K_3P_2$ + 0.90 $H_2O$ | | | |
|---|---|---|---|
| | C, 35.82; | H, 6.58; | P, 12.32 |
| Found: | C, 35.58; | H, 6.95; | P, 12.69. |

The following compounds suitable for use in the method of the invention for inhibiting cholesterol biosynthesis by inhibiting de novo squalene production were prepared employing procedures similar to that described in Example 1;

heptylidenebisphosphonic acid, esters thereof, salts thereof, or mixed ester-salts thereof, or trisodium or tripotassium salt;

nonylidenebisphosphonic acid, esters thereof, salts thereof, or mixed ester-salts thereof, or tetrasodium or tetrapotassium salt;

decylidenebisphosphonic acid, esters thereof, salts thereof, or mixed ester-salts thereof, or tetrasodium or tetrapotassium salt;

tridecylidenebisphosphonic acid, esters thereof, salts thereof, or mixed ester-salts thereof;

octadecylidenebisphosphonic acid, esters thereof, salts thereof, or mixed ester-salts thereof, or tetrasodium or tetrapotassium salt;

heneicosylidenebisphosphonic acid, esters thereof, salts thereof, or mixed ester-salts thereof, or tetrasodium or tetrapotassium salt;

(6-methyl-5-heptenylidene)bisphosphonic acid, esters thereof, salts thereof, or mixed ester-salts thereof, or tetrapotassium or tetrasodium salt;

[2-(4-butylphenyl)ethylidene]bisphosphonic acid, esters thereof, salts thereof, or mixed ester-salts thereof.

**Claims**

**1.** Use of a therapeutically effective amount of a bisphosphonate squalene synthetase inhibitor, wherein

the phosphonates are bridged by a methylene group, and which includes at least one lipophilic group attached to the methylene group, wherein the term "lipophilic group" refers to a group which contains at least six carbons and is required for strong enzyme inhibitor binding and inhibition of the enzyme squalene synthetase or other enzymes in the cholesterol biosynthetic pathway,

for the preparation of a pharmaceutical composition useful in inhibiting cholesterol biosynthesis or inhibiting or treating hypercholesterolemia and thereby inhibiting or treating atherosclerosis.

**2.** Use of a bisphosphonate compound having the structure

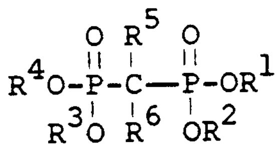

(I)

wherein $R^1$, $R^2$, $R^3$ and $R^4$ are the same or different and are H, alkyl, aryl, alkylaryl, arylalkyl, ammonium, alkali metal or a prodrug ester, wherein at least one of $R^5$ and $R^6$ is a hydrocarbyl group having at least 6 carbons (which is alkyl, alkenyl, alkynyl, cycloalkyl, aryl, alkylaryl, arylalkyl, or arylalkenyl); heterocyclic (which is succinimdyl, pyridyl, quinalyl, morpholino, furanyl, indolyl, picolinyl, thiophene, imidazole, oxazole, isoxazole, thiazole, pyridine, 1,2,3-triazole, 1,2,4-triazole, benzimidazole, tetrahydrofuranyl, pyrrolidino, piperidino, 5-membered heteroarylmethyl containing 2 to 4 N atoms or 1-2 N atoms plus an O or S atom); heterocyclicalkyl (wherein heterocyclic is as defined above such as 1-(decahydroquinolin-3-yl)methane); amino; alkylamino; dialkylamino; arylalkylaminoalkyl; ethylcarbonyloxymethylamino; cycloalkyl(alkyl)amino; alkenylamino, cycloalkylamino, aminocycloalkyl; aminocycloalkylalkyl; N-hydroxy-N-ethylamino; acetylamino; aminoalkyloxyalkyl; (benzo- or cyclohexeno-fused) 5 membered heteroaryl containing 2-4 N atoms or 1-2 Natoms plus an O or S atom; $R^8$-X-$(CH_2)_a$- (wherein $R^8$ is H, alkyl, or a nitrogen containing 6-membered aromatic ring which is pyridyl, indanyl, hexahydroindanyl or picolyl; X is O, NH or a single bond and a is 0 to 7);

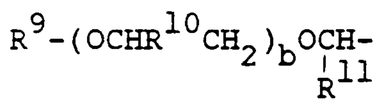

(wherein $R^9$ is $C_1$-$C_{10}$ alkyl, optionally substituted aryl, phenylalkyl or naphthylalkyl),

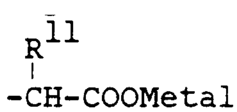

or

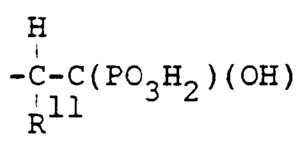

(wherein $R^{10}$ and and $R^{11}$ are the same or different and are H or methyl, b is 1 to 20));

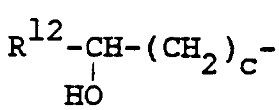

(wherein $R^{12}$ is H, phenyl or phenyl substituted with halogen, alkyl or hydroxy and c is 0 to 9);

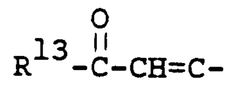

(wherein $R^{13}$ is tert-alkyl ($CR^{14}R^{15}R^{16}$ wherein $R^{14}$ and $R^{15}$ are independently $C_1$-$C_3$ alkyl and $R^{16}$ is $C_1$-$C_{10}$ alkyl), cycloalkyl, aryl or heteroaryl, or substituted cycloalkyl, substituted aryl or substituted heteroaryl wherein the substituent is halogen, $C_1$-$C_4$ alkyl, alkoxy or dialkylamino);

4-Cl-$C_6H_5$-S-$CH_2$; aryloxy;

$R^{17}$-$(QCH_2CH_2)_dO$- (wherein $R^{17}$ is $C_1$-$C_{10}$ alkyl, $C_2$-$C_{10}$ alkenyl, aryl or arylalkyl, or each of the above $R^{17}$ groups optionally substituted with $C_1$-$C_4$ alkyl, amino, alkylamino, carboxyl, alkoxycarbonyl, hydroxy, alkoxy, phenoxy, mercapto, alkylthio, phenylthio, halogen or trifluoromethyl, Q is O or S and d is 0, 1 or 2);

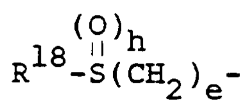

(wherein e is 0 to 10, h is 0, 1 or 2, $R^{18}$ is H, cycloalkyl, aryl, alkyl, each optionally substituted with OH, SH, halogen, alkoxycarbonyl or $NZ_1Z_2$, phenyl optionally substituted with halogen, nitro, lower alkyl, alkoxy, trifluoromethyl, amino, carboxyl, $CO_2$alkyl, -$CONZ_1Z_2$, -$CSNZ_1Z_2$, a 5- or 6-membered heterocyclic radical containing 1 or 2 heteroatoms, which are N or S, which may or may not be fused to a benzene ring, $Z_1$ and $Z_2$ are independently H or lower alkyl);

thiol; phenylthio; chlorophenylthio; 4-thiomorpholinyl;

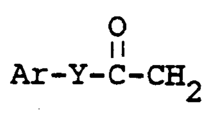

(wherein Ar is aryl, pyrrolyl or aryl optionally substituted with $C_1$-$C_4$alkyl, alkoxy, halo (F, Cl), naphthyl, biphenyl or thienyl and Y is NH or a single bond);

$R^{19}SCH_2$- (wherein $R^{19}$ is alkyl, aryl or arylalkyl);

A-$(CH_2)_f$-NH- (wherein A is $C_5$-$C_8$ cycloalkenyl, bicycloheptyl, bicycloheptenyl, saturated $C_4$-$C_7$ heterocycle containing O,S,SO or $SO_2$);

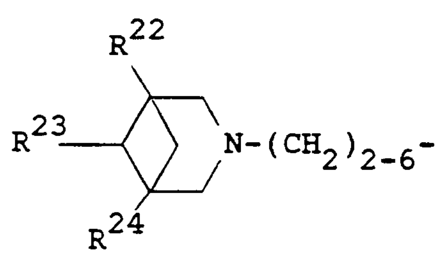

(wherein $R^{22}$ is H, $C_1$-$C_{20}$alkyl, alkoxy, aryl, $R^{23}$ is H, $C_1$-$C_{20}$alkyl, alkoxy, aryl, halo, carboxyl, $R^{24}$ is H, $C_1$-$C_{20}$ alkyl, alkoxy);

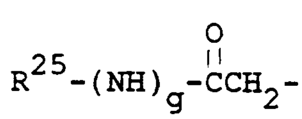

(wherein $R^{25}$ is (alkyl-substituted)pyrrolyl or phenyl and g is 0 or 1);

aromatic-substituted mono- or biazacyclylalkyl (alkyl group bonds with the N in the heterocycle) (such as 3-(4-phenylpiperidino)propyl);

$R^{31}$-Ax-CO-$CH_2$-

(wherein Ax is phenyl, naphthyl, mono- or bicyclic-N-containing heterocycle and $R^{31}$ is H, halo, lower alkyl or lower alkoxy);

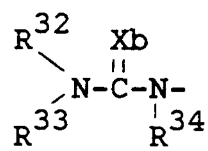

(wherein $R^{32}$ is aryl, aralkyl, alkyl, $R^{33}$ is H or aryl, Xb is O or S, and $R^{34}$ is H or alkyl);

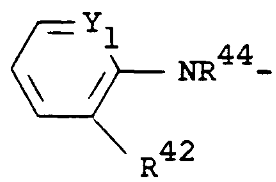

(wherein $R^{42}$ is H, alkyl or halo, $Y_1$ is N, NO, or $NR^{43}Y_2$ wherein $R^{43}$ is alkyl and $Y_2$ is halo; and $R^{44}$ is H or aliphatic acyl);

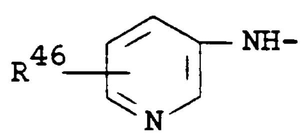

(wherein $R^{46}$ is H, halo or alkyl);

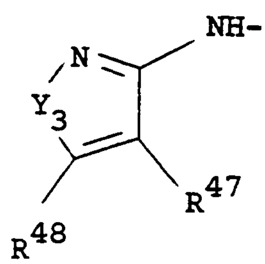

(wherein $Y_3$ is O or NH, $R^{47}$ is H, alkyl or halo, and $R^{48}$ is H or alkyl);

$R^{50}$ -NH-

(wherein $R^{50}$ is

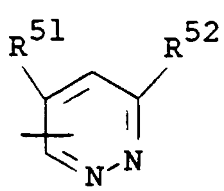

,

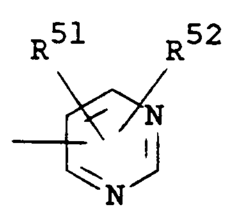

or

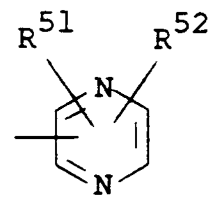

wherein $R^{51}$ and $R^{52}$ are H, halo, alkyl or hydroxy);

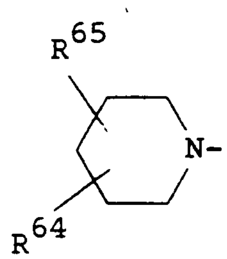

(wherein $R^{64}$ is alkyl and $R^{65}$ is H or alkyl;

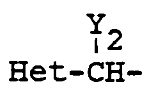

wherein Het is a heteroaromatic 5-membered ring with 2 or 3 heteroatoms, optionally partially hydrogenated and optionally substituted by one or more alkyl, alkoxy, phenyl, cyclohexyl, cyclohexylmethyl, halo or amino, with 2 adjacent alkyl optionally together forming a ring (Het cannot be pyrazole), and $Y_2$ is H or lower alkyl);

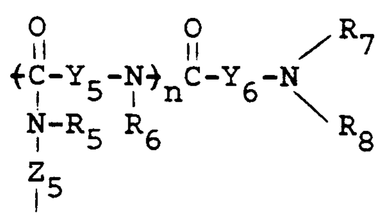

(wherein $Y_4$ is H or OH, $R_5$-$R_8$ are independently H or lower alkyl, whereby $R_7$ and $Y_6$ or $R_6$ and $Y_5$ or $R_5$ and $Z_5$, together with the nitrogen atom to which they are attached can form a 5- or 6-membered ring, $Y_6$ and $Y_5$ which can be the same or different are $C_1$-$C_6$ alkylene chains optionally substituted by aromatic or heteroaromatic radicals, $Z_5$ is $C_1$ to $C_6$ alkylene which can include heteroatoms and optionally substituted by aromatic or heteroaromatic, n is 0, 1 or 2;

$R_{27}$-$Z_9$-

(wherein $R_{27}$ is aryl or heterocyclyl both optionally substituted by one or more of lower alkyl, lower alkoxy, lower alkylthio, halo(lower)alkyl, acyl, acylamino or halo, or $R_{27}$ is lower alkyl substituted by heterocyclyl which is optionally substituted by acyl); $R_{27}$-$Z_9$ is $R_{27}$-NHC(=$X_9$), $R_{27}$-C(=O)NH-, $R_{27}$-$SO_2$-NH- (wherein $X_9$ is O or S);

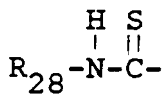

(wherein $R_{28}$ is phenyl, pyridyl or quinolyl substituted by lower alkylsulphonylamino, halo-lower alkylsulphonylamino, arylsulphonylamino and mono- or di-lower alkylamino);

$R_{29}$-CO-[-$R_{30}$($CH_2$)$_o$CO-]$_p$-NH-

(wherein $R_{29}$-CO- is a residue of a pharmaceutically active compound $R_{29}$-COOH, wherein $R_{29}$ is an anti-inflamatory agent, or antioncotic agent or hormone ,

$R_{30}$ is -NH- or -O-

p is 0 or 1;

o is 1-10);

$R_{33}$-($CH_2$)$_q$-

(wherein $R_{33}$ is an N-bonded azabicycloalkyl group with 3 to 8-membered rings and q is 2 to 4);

$R_{34}$-($CH_2$)$_r$-

(wherein $R_{34}$ is an N-bonded, aryl-substituted mono- or diazacycloaliphatic group);

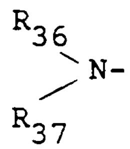

(wherein $R_{36}$ is 5 membered heteroaryl with 2-4 N or with 1-2 N plus an O or S atom, optionally fused to a benzo or cyclohexeno ring;

$R_{36}$ can be C substituted by lower alkyl, phenyl (optionally substituted by lower alkyl, alkoxy and/or halo), lower alkoxy, OH, di(lower alkyl)amino, lower alkylthio and/or halo, and/or N substituted by lower alkyl or phenyl (lower) alkyl (optionally substituted by lower alkyl, lower alkoxy and/or halo);

$R_{37}$ is H or lower alkyl; provided $R_{37}$ is not H if $R_{36}$ is optionally substituted alkyl and/or halo substituted 3-pyrazolyl or 3-isoxazolyl);

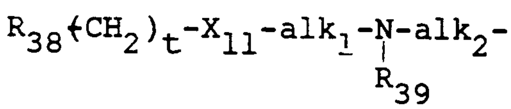

(wherein $R_{38}$ is aromatic residue;

t is 0-3;

$X_{11}$ is 0 S (optionally oxidized) or imino (optionally substituted by aliphatic group);

$alk_1$ and $alk_2$ are divalent aliphatic groups; $R_{39}$ is H or monovalent aliphatic group);

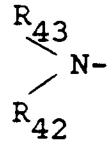

(wherein $R_{42}$ and $R_{43}$ are hydrogen, alkyl having one to 22 carbon atoms, cycloalkyl having five to six carbon atoms, phenyl alkylphenyl having seven to 18 carbon atoms, phenylalkyl having seven to 18 carbon atoms and together with the nitrogen atom, piperidino, pyrrolidino and morpholino);

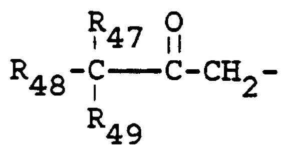

(wherein $R_{47}$ is optionally branched $C_1$-$C_8$alkyl,
$R_{48}$ and $R_{49}$ are each methyl or ethyl, and
M is H or a cation of a water-soluble base);

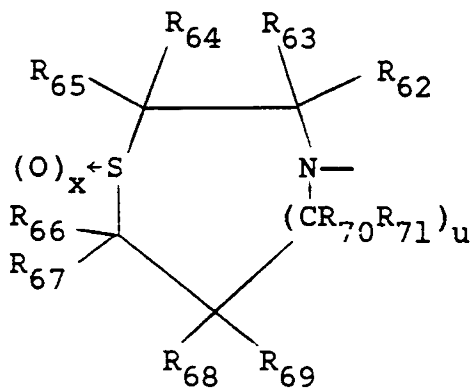

(wherein $R_{62}$-$R_{71}$ is H, straight, branched or alicyclic 1-10C hydrocarbyl, aryl or aryl-(1-4C)-alkyl;
x is 0 or 1;
u is 0, 1 or 2;
or $R_{62}$ and $R_{64}$ may complete a 5- to 7-membered saturated aliphatic ring optionally substituted by 1 or more alkyl groups);

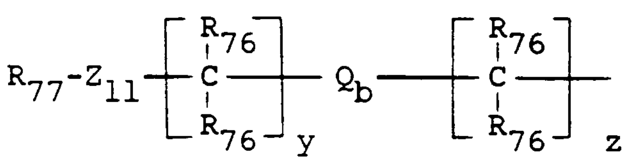

(wherein $Z_{11}$ is an N-containing 6-membered ring heterocycle moiety selected from piperidinyl, diazinyl or triazinyl;
$Q_b$ is a covalent bond, O, S or $NR_{76}$;
y, z, and y + z are integers of 0-10;
$R_{76}$ is H, or $C_1$-$C_3$alkyl;
$R_{77}$ is one or more substituted selected from H, halogen, 1-3C alkyl, unsubstituted amino and its amide derived from a 1-3C carboxylic acid, mono(1-3C alkyl) amino and its amide derived from a 1-3C carboxylic acid, di(1-3C alkyl)amino, tri(1-3C alkyl) ammonium, hydroxy or its ester derived from a 1-3C carboxylic acid, ether having 1-3C, $CO_2H$ and its salts and esters derived from 1-3C alcohols, its amide optionally substituted with one or two 1-3C alkyl groups, and $NO_2$);

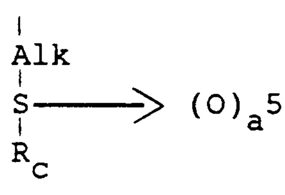

(wherein $R_c$ represents:

$C_1$-$C_6$ alkyl group,

$C_5$-$C_7$ cycloalkyl group,

phenyl group optionally monosubstituted or polysubstituted by a halogen, a $C_1$-$C_6$ alkyl group or a trifluoromethyl group, or

5-membered or 6-membered heterocycle containing 1 or 2 heteroatoms chosen from nitrogen and sulfur,

Alk denoted a linear or branched $C_1$-$C_6$ alkylene group,

$a^5$ represents 0 or the integer 1 or 2);

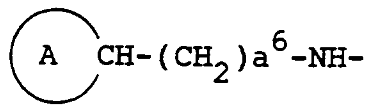

(wherein $a^6$ is 0 to 4 and

Ring A is 5-8C cycloalkenyl, bicycloheptyl, bicycloheptenyl or 4-7C saturated heterocyclyl containing 0, S, SO or $SO_2$);

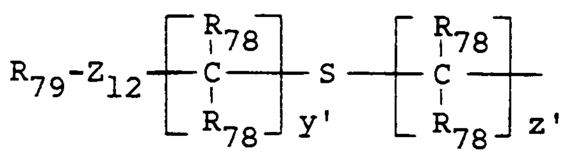

(wherein $Z_{12}$ is a 6-membered aromatic ring containing ≧ 1 N atom(s); where:

the ring is optionally substituted by (optionally substituted, optionally unsaturated) 1-6C alkyl, (optionally substituted) aryl, (optionally substituted) benzyl, OH, halogen, carbonyl, alkoxy, $NO_2$, $CONH_2$, (optionally substituted) $NH_2$ and/or carboxylate, such as pyridine, pyridazine, pyrimidine or pyrazine ring;

$R_{78}$ is H or (optionally substituted, optionally unsubstituted) 1-4C alkyl;

$R_{79}$ is H, (optionally substituted, optionally unsubstituted) 1-6C alkyl, (optionally substituted) aryl, (optionally substituted) benzyl, OH, halogen, carbonyl, alkoxy, $NO_2$, $CONH_2$, (optionally substituted) amino or carboxylate,

y' + z' is 0 to 5);

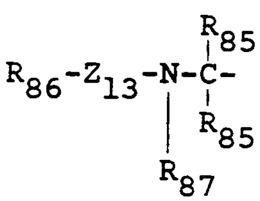

or

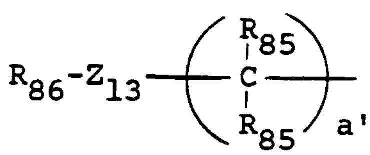

(wherein $Z_{13}$ is a pyridine, pyridazine, pyrimidine or pyrazine ring, optionally substituted by optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{86}$ is H or optionally substituted, optionally unsaturated 1-4C alkyl;

$R_{86}$ is one or more of H, optionally substituted, optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{87}$ is H, optionally substituted, optionally unsaturated 1-4C alkyl or acyl;

a' is 1-5);

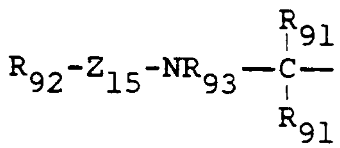

or

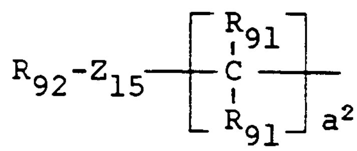

(wherein $Z_{15}$ is a 6 membered aromatic ring containing one or more N atoms such as pyridine, pyridazine, pyrimidine or pyrazine, which ring may be substituted with one or more optionally substituted, optionally unsaturated 1-6C alkyl, optionally substituted aryl, optionally substituted benzyl, OH, halogen, carbonyl, alkoxy, $NO_2$, amido, optionally substituted amino or carboxylate);

$R_{91}$ is H or optionally substituted, optionally unsaturated 1-4C alkyl;

$R_{92}$ is H or one or more substituents selected from optionally substituted, optionally unsaturated 1-6C alkyl, optionally substituted aryl, optionally substituted benzyl, OH, halogen, carbonyl, alkoxy, $NO_2$, amido, optionally substituted amino or carboxylate;

$R_{93}$ is H, optionally substituted, optionally unsaturated 1-4C alkyl or acyl;

$a^2$ is 1 to 5);

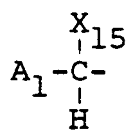

(wherein $X_{15}$ is hydrogen, methyl, or ethyl, and $A_1$ is phenyl substituted in the para-position by isobutyl, cyclohexyl, alkoxy, or 1-pyrrolinyl and, optionally substituted additionally in the meta-position by fluorine or chlorine, or phenyl substituted in the meta-position by benzoyl or phenoxy, or phenyl substituted in the ortho-position by 2,4-dichlorophenoxy or 2,6-dichlorophenylamino);

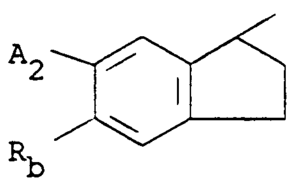

(wherein

$R_b$ is cyclohexyl or cyclophenylmethyl; and

$A_2$ is hydrogen or chlorine);

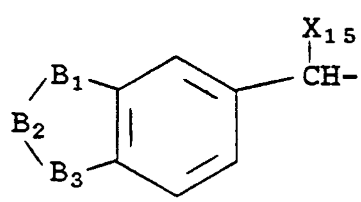

(wherein $X_{15}$ is as defined above, and

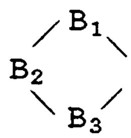 is

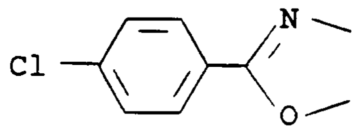

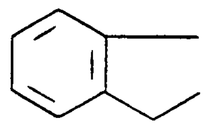

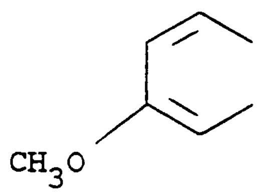 or 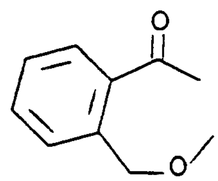 ;

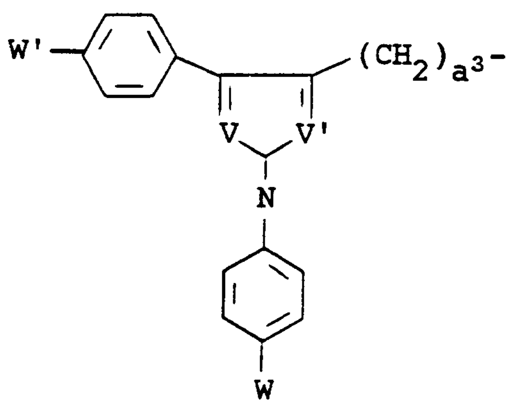

wherein $a^3$ is 1, 2 or 3;

W and W', are identical or different, and each is hydrogen, fluorine or chlorine, and

one of V and V' is nitrogen and the other is a methyne residue optionally substituted by a phenyl group, and

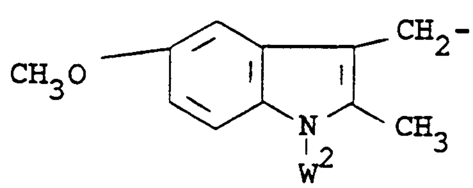

wherein $W^2$ is p-chlorobenzoyl or cinnamoyl);

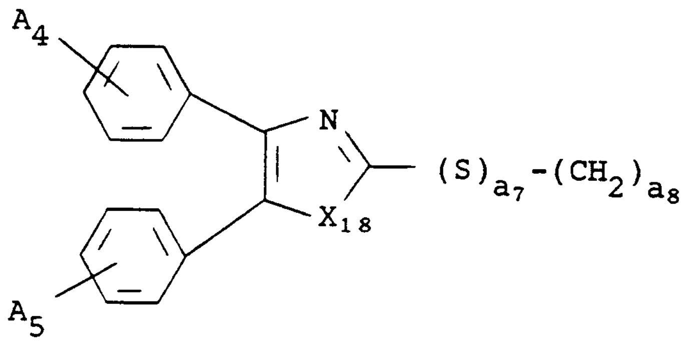

(wherein $A_4$ and $A_5$ are the same or different and are H, OH, lower alkoxy or halogen;
$X_{15}$ is O, S or NH;
$a_7$ is 0 or 1;
$a_8$ is 0 or an integer of 1-6);

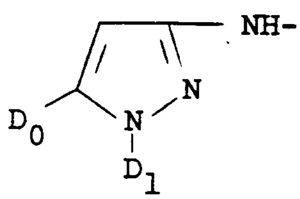

(wherein $D_0$ is H or alkyl;
$D_1$ is H or lower alkyl);

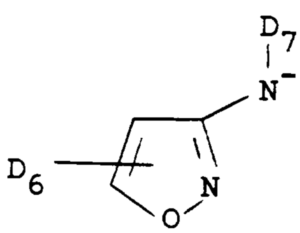

(wherein $D_6$ is H, 1-10C alkyl, 3-10C cycloalkyl, phenyl, 2-10C alkenyl (optionally substituted by phenyl) or phenyl(1-5C)alkyl (optionally ring-substituted by a 1-5C alkoxy);
$D_7$ is H or 2-6C alkanoyl);

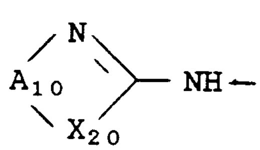

(wherein $A_{10}$ is a group of formula (a)-(c):

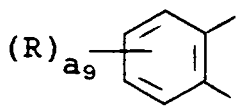 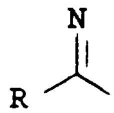 or 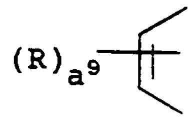

(a) (b) (c)

and $X_{20}$ is O, S or NH);

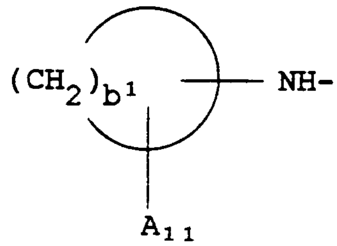

(wherein $A_{11}$ is H or 1-5C alkyl; $b_1$ is 3-10);

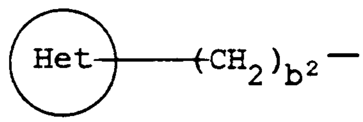

(wherein ring Het is a group of formula (A) or (B):

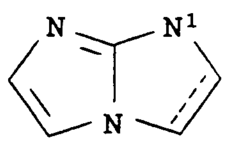 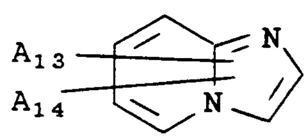

(A) (B)

the dotted line represents an optional double bond; $A_{13}$, $A_{14}$ are H, 1-5C alkyl, halogen or OH);

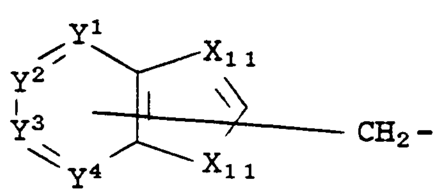

(wherein $X_{11}$ are both N or one is N and the other is CH; one of $Y^1$-$Y^4$ is N and the rest is CH);

and the other of $R^5$ and $R^6$ is H, halogen, $C_1$-$C_{30}$ alkyl, amino, alkylamino, dialkylamino, uriedo ($NK_2CO$-$N(R^{38})$- where $R^{38}$ is H, alkyl, benzyl, phenyl optionally substituted with Cl or $CH_3$); alkenylamino, cycloalkylamino, aryloxy, pyridinium, guanidinium, ammonium, di-and tri-lower alkanolammonium, hydroxy, arylalkyl, alkoxy, alkylaryloxy, $-CH_2CO_2H$, $-CH_2PO_3H_2$, $-CH(PO_3H_2)(OH)$, $-CH_2CO_2C_2H_5$, $-CH_2CH(PO_3H_2)_2$, a hydrocarbyl radical as defined herein, a heterocyclic radical as defined herein, alkanoyl, an $R^6$ or $R^5$ radical as defined herein, a prodrug ester (such as (1-alkanoyloxy)alkyl, for example $t$-$C_4H_9CO_2CH_2$-, $CH_3CO_2CH_2$-);

at least one of $R^5$ and $R^6$ being a lipophilic group, or $R^5$ and $R^6$ can be joined to form a carbocyclic ring containing 3 to 12 carbons or a heterocyclic ring containing N, O and/or S atoms, such as of the formula

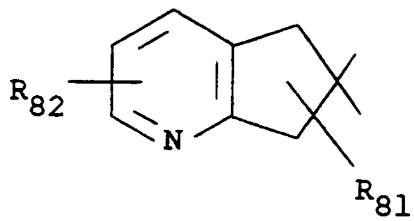

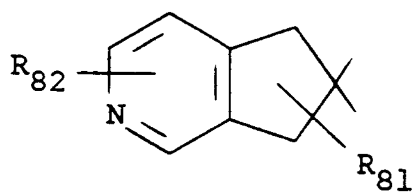

or

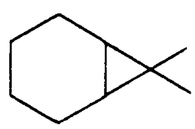

wherein $R_{81}$ and $R_{82}$ are each one or more substituents selected from H, optionally substituted saturated or unsaturated 1-6C alkyl, optionally substituted aryl, optionally substituted benzyl, amido, OH, halogen, optionally substituted amino, amido, COOH, carbonyl, carboxylate, alkoxy and $NO_2$,

for the preparation of a pharmaceutical composition useful in inhibiting cholesterol biosynthesis or inhibiting or treating hypercholesterolemia and thereby inhibiting atherosclerosis.

**3.** Use according to Claim 1 or 2 wherein the squalene synthetase inhibitor employed is
tetramethyl 1(p-chlorophenyl)methane-1-hydroxy 1,1-diphosphonate,
tetramethyl 2,2-dimethyl 2-(p-chlorophenoxy)ethane 1-hydroxy-1,1-diphosphonate,
tetramethyl 1-[4(4'-chlorobenzoyl)phenyl]methane 1-hydroxy 1,1-diphosphonate,
dimethyl 1[(dimethoxyphosphinyl)p-chlorobenzyl]phosphate,
dimethyl [1(dimethoxyphosphinyl) 2,2-dimethyl 2-phenyl]-ethyl phosphate,
dimethyl [1(dimethoxyphosphinyl)2,2-dimethyl 2(p-chlorophenyl] ethyl phosphate,
tetramethyl 4-phenylbutylidene, 1,1-diphosphonate.

**4.** Use according to Claim 1 wherein the lipophilic group in the bisphosphonate compound is optionally substituted alkyl, optionally substituted alkenyl or optionally substituted aryl.

**5.** Use according to Claim 2 wherein the bisphosphonate is heptylidenebisphosphonic acid, esters thereof, salts thereof, or mixed ester-salts thereof, or trisodium or tripotassium salt;

nonylidenebisphosphonic acid, esters thereof, salts thereof, or mixed ester-salts thereof, or tetrasodium or tetrapotassium salt;

decylidenebisphosphonic acid, esters thereof, salts thereof, or mixed ester-salts thereof, or tetrasodium or tetrapotassium salt;

tridecylidenebisphosphonic acid, esters thereof, salts thereof, or mixed ester-salts thereof;

octadecylidenebisphosphonic acid, esters thereof, salts thereof, or mixed ester-salts thereof, or tetrasodium or tetrapotassium salt;

heneicosylidenebisphosphonic acid, esters thereof, salts thereof, or mixed ester-salts thereof, or tetrasodium or tetrapotassium salt;

(6-methyl-5-heptenylidene)bisphosphonic acid, esters thereof, salts thereof, or mixed ester-salts thereof, or tetrapotassium or tetrasodium salt;

[2-(4-butylphenyl)ethylidene]bisphosphonic acid, esters thereof, salts thereof, or mixed ester-salts thereof.

**Claims for the following Contracting States : GR, ES**

1. A method for the preparation of a pharmaceutical composition useful in inhibiting cholesterol biosynthesis or inhibiting or treating hypercholesterolemia and thereby inhibiting or treating atherosclerosis, which comprises combining a therapeutically effective amount of a bisphosphonate squalene synthetase inhibitor, wherein the phosphonates are bridged by a methylene group, and which includes at least one lipophilic group attached to the methylene group, wherein the term "lipophilic group" refers to a group which contains at least six carbons and is required for strong enzyme inhibitor binding and inhibition of the enzyme squalene synthetase or other enzymes in the cholesterol biosynthetic pathway, with a pharmaceutically acceptable carrier.

2. A method for the preparation of a pharmaceutical composition useful in inhibiting cholesterol biosynthesis or inhibiting or treating hypercholesterolemia and thereby inhibiting or treating atherosclerosis, which comprises combining a bisphosphonate compound having the structure

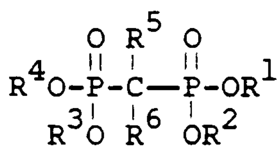

(I)

wherein $R^1$, $R^2$, $R^3$ and $R^4$ are the same or different and are H, alkyl, aryl, alkylaryl, arylalkyl, ammonium, alkali metal or a prodrug ester, wherein at least one of $R^5$ and $R^6$ is a hydrocarbyl group having at least 6 carbons (which is alkyl, alkenyl, alkynyl, cycloalkyl, aryl, alkylaryl, arylalkyl, or arylalkenyl); heterocyclic (which is succinimdyl, pyridyl, quinalyl, morpholino, furanyl, indolyl, picolinyl, thiophene, imidazole, oxazole, isoxazole, thiazole, pyridine, 1,2,3-triazole, 1,2,4-triazole, benzimidazole, tetrahydrofuranyl, pyrrolidino, piperidino, 5-membered heteroarylmethyl containing 2 to 4 N atoms or 1-2 N atoms plus an O or S atom); heterocyclicalkyl (wherein heterocyclic is as defined above such as 1-(decahydroquinolin-3-yl)methane); amino; alkylamino; dialkylamino; arylalkylaminoalkyl; ethylcarbonyloxymethylamino; cycloalkyl(alkyl)amino; alkenylamino, cycloalkylamino, aminocycloalkyl; aminocycloalkylalkyl; N-hydroxy-N-ethylamino; acetylamino; aminoalkyloxyalkyl; (benzo- or cyclohexeno-fused) 5 membered heteroaryl containing 2-4 N atoms or 1-2 Natoms plus an O or S atom; $R^8$-X-$(CH_2)_a$-(wherein $R^8$ is H, alkyl, or a nitrogen containing 6-membered aromatic ring which is pyridyl, indanyl, hexahydroindanyl or picolyl; X is O, NH or a single bond and a is 0 to 7);

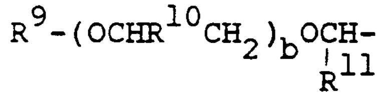

(wherein $R^9$ is $C_1$-$C_{10}$ alkyl, optionally substituted aryl, phenylalkyl or naphthylalkyl),

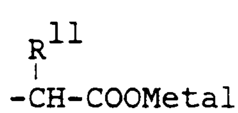

or

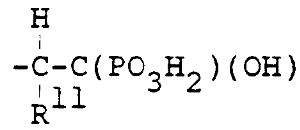

(wherein $R^{10}$ and and $R^{11}$ are the same or different and are H or methyl, b is 1 to 20));

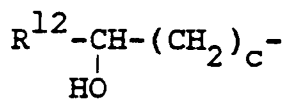

(wherein $R^{12}$ is H, phenyl or phenyl substituted with halogen, alkyl or hydroxy and c is 0 to 9);

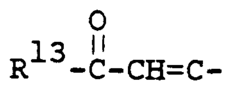

(wherein $R^{13}$ is tert-alkyl ($CR^{14}R^{15}R^{16}$ wherein $R^{14}$ and $R^{15}$ are independently $C_1$-$C_3$ alkyl and $R^{16}$ is $C_1$-$C_{10}$ alkyl), cycloalkyl, aryl or heteroaryl, or substituted cycloalkyl, substituted aryl or substituted heteroaryl wherein the substituent is halogen, $C_1$-$C_4$ alkyl, alkoxy or dialkylamino);

4-Cl-$C_6H_5$-S-$CH_2$; aryloxy;

$R^{17}$-$(QCH_2CH_2)_d$O- (wherein $R^{17}$ is $C_1$-$C_{10}$ alkyl, $C_2$-$C_{10}$ alkenyl, aryl or arylalkyl, or each of the above $R^{17}$ groups optionally substituted with $C_1$-$C_4$ alkyl, amino, alkylamino, carboxyl, alkoxycarbonyl, hydroxy, alkoxy, phenoxy, mercapto, alkylthio, phenylthio, halogen or trifluoromethyl, Q is O or S and d is 0, 1 or 2);

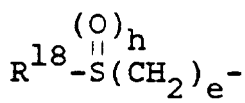

(wherein e is 0 to 10, h is 0, 1 or 2, $R^{18}$ is H, cycloalkyl, aryl, alkyl, each optionally substituted with OH, SH, halogen, alkoxycarbonyl or $NZ_1Z_2$, phenyl optionally substituted with halogen, nitro, lower alkyl, alkoxy, trifluoromethyl, amino, carboxyl, $CO_2$alkyl, -$CONZ_1Z_2$, -$CSNZ_1Z_2$, a 5- or 6-membered heterocyclic radical containing 1 or 2 heteroatoms, which are N or S, which may or may not be fused to a benzene ring, $Z_1$ and $Z_2$ are independently H or lower alkyl);

thiol; phenylthio; chlorophenylthio; 4-thiomorpholinyl;

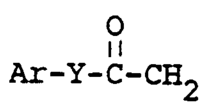

(wherein Ar is aryl, pyrrolyl or aryl optionally substituted with $C_1$-$C_4$alkyl, alkoxy, halo (F, Cl), naphthyl, biphenyl or thienyl and Y is NH or a single bond);

$R^{19}SCH_2$- (wherein $R^{19}$ is alkyl, aryl or arylalkyl);

A-$(CH_2)_f$-NH- (wherein A is $C_5$-$C_8$ cycloalkenyl, bicycloheptyl, bicycloheptenyl, saturated $C_4$-$C_7$ heterocycle containing O,S,SO or $SO_2$);

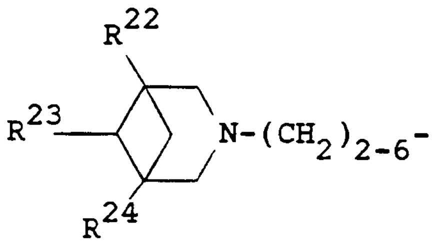

(wherein $R^{22}$ is H, $C_1$-$C_{20}$alkyl, alkoxy, aryl, $R^{23}$ is H, $C_1$-$C_{20}$alkyl, alkoxy, aryl, halo, carboxyl, $R^{24}$ is H, $C_1$-$C_{20}$ alkyl, alkoxy);

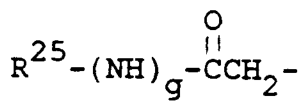

(wherein $R^{25}$ is (alkyl-substituted)pyrrolyl or phenyl and g is 0 or 1);

aromatic-substituted mono- or biazacyclylalkyl (alkyl group bonds with the N in the heterocycle) (such as 3-(4-phenylpiperidino)propyl);

$R^{31}$-Ax-CO-$CH_2$-

(wherein Ax is phenyl, naphthyl, mono- or bicyclic-N-containing heterocycle and $R^{31}$ is H, halo, lower alkyl or lower alkoxy);

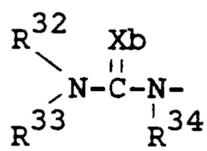

(wherein $R^{32}$ is aryl, aralkyl, alkyl, $R^{33}$ is H or aryl, Xb is O or S, and $R^{34}$ is H or alkyl);

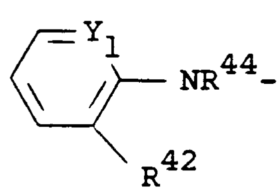

(wherein $R^{42}$ is H, alkyl or halo, $Y_1$ is N, NO, or $NR^{43}Y_2$ wherein $R^{43}$ is alkyl and $Y_2$ is halo; and $R^{44}$ is H or aliphatic acyl);

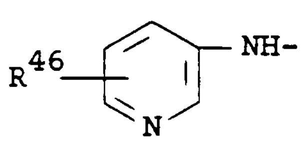

(wherein $R^{46}$ is H, halo or alkyl);

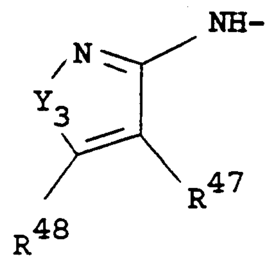

(wherein $Y_3$ is O or NH, $R^{47}$ is H, alkyl or halo, and $R^{48}$ is H or alkyl);

$R^{50}$ -NH-

(wherein $R^{50}$ is

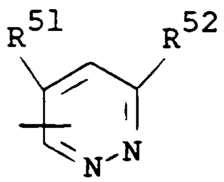

,

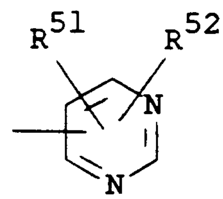

or

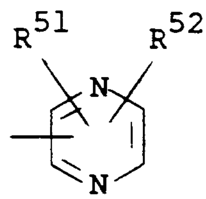

wherein $R^{51}$ and $R^{52}$ are H, halo, alkyl or hydroxy);

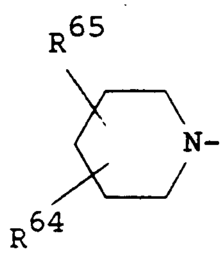

(wherein $R^{64}$ is alkyl and $R^{65}$ is H or alkyl;

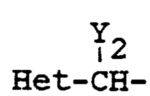

wherein Het is a heteroaromatic 5-membered ring with 2 or 3 heteroatoms, optionally partially hydrogenated and optionally substituted by one or more alkyl, alkoxy, phenyl, cyclohexyl, cyclohexylmethyl, halo or amino, with 2 adjacent alkyl optionally together forming a ring (Het cannot be pyrazole), and $Y_2$ is H or lower alkyl);

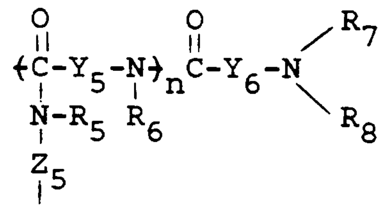

(wherein $Y_4$ is H or OH, $R_5$-$R_8$ are independently H or lower alkyl, whereby $R_7$ and $Y_6$ or $R_6$ and $Y_5$ or $R_5$ and $Z_5$, together with the nitrogen atom to which they are attached can form a 5- or 6-membered ring, $Y_6$ and $Y_5$ which can be the same or different are $C_1$-$C_6$ alkylene chains optionally substituted by aromatic or heteroaromatic radicals, $Z_5$ is $C_1$ to $C_6$ alkylene which can include heteroatoms and optionally substituted by aromatic or heteroaromatic, n is 0, 1 or 2;

$R_{27}$-$Z_9$-

(wherein $R_{27}$ is aryl or heterocyclyl both optionally substituted by one or more of lower alkyl, lower alkoxy, lower alkylthio, halo(lower)alkyl, acyl, acylamino or halo, or $R_{27}$ is lower alkyl substituted by heterocyclyl which is optionally substituted by acyl); $R_{27}$-$Z_9$ is $R_{27}$-NHC(=$X_9$), $R_{27}$-C(=O)NH-, $R_{27}$-$SO_2$-NH- (wherein $X_9$ is O or S);

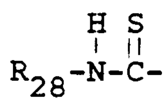

(wherein $R_{28}$ is phenyl, pyridyl or quinolyl substituted by lower alkylsulphonylamino, halo-lower alkylsulphonylamino, arylsulphonylamino and mono- or di-lower alkylamino);

$R_{29}$-CO-[-$R_{30}$($CH_2$)$_o$CO-]$_p$-NH-

(wherein $R_{29}$-CO- is a residue of a pharmaceutically active compound $R_{29}$-COOH, wherein $R_{29}$ is an anti-inflamatory agent, or antioncotic agent or hormone ,

$R_{30}$ is -NH- or -O-

p is 0 or 1;

o is 1-10);

$R_{33}$-($CH_2$)$_q$-

(wherein $R_{33}$ is an N-bonded azabicycloalkyl group with 3 to 8-membered rings and q is 2 to 4);

$R_{34}$-($CH_2$)$_r$-

(wherein $R_{34}$ is an N-bonded, aryl-substituted mono- or diazacycloaliphatic group);

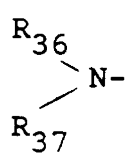

(wherein $R_{36}$ is 5 membered heteroaryl with 2-4 N or with 1-2 N plus an O or S atom, optionally fused to a benzo or cyclohexeno ring;

$R_{36}$ can be C substituted by lower alkyl, phenyl (optionally substituted by lower alkyl, alkoxy and/or halo), lower alkoxy, OH, di(lower alkyl)amino, lower alkylthio and/or halo, and/or N substituted by lower alkyl or phenyl (lower) alkyl (optionally substituted by lower alkyl, lower alkoxy and/or halo);

$R_{37}$ is H or lower alkyl; provided $R_{37}$ is not H if $R_{36}$ is optionally substituted alkyl and/or halo substituted 3-pyrazolyl or 3-isoxazolyl);

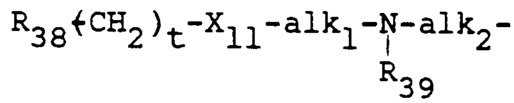

(wherein $R_{38}$ is aromatic residue;

t is 0-3;

$X_{11}$ is 0 S (optionally oxidized) or imino (optionally substituted by aliphatic group);

$alk_1$ and $alk_2$ are divalent aliphatic groups; $R_{39}$ is H or monovalent aliphatic group);

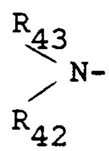

(wherein $R_{42}$ and $R_{43}$ are hydrogen, alkyl having one to 22 carbon atoms, cycloalkyl having five to six carbon atoms, phenyl alkylphenyl having seven to 18 carbon atoms, phenylalkyl having seven to 18 carbon atoms and together with the nitrogen atom, piperidino, pyrrolidino and morpholino);

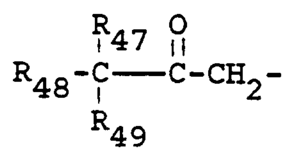

(wherein $R_{47}$ is optionally branched $C_1$-$C_8$alkyl,

$R_{48}$ and $R_{49}$ are each methyl or ethyl, and

M is H or a cation of a water-soluble base);

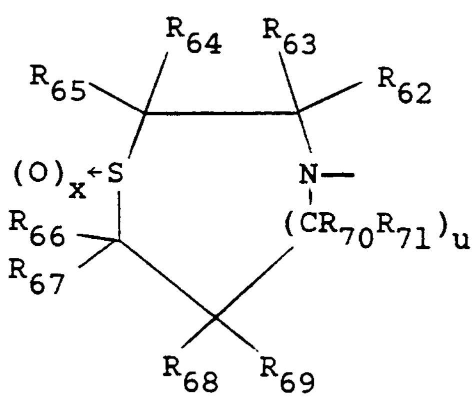

(wherein $R_{62}$-$R_{71}$ is H, straight, branched or alicyclic 1-10C hydrocarbyl, aryl or aryl-(1-4C)-alkyl;

x is 0 or 1;

u is 0, 1 or 2;

or $R_{62}$ and $R_{64}$ may complete a 5- to 7-membered saturated aliphatic ring optionally substituted by 1 or more alkyl groups);

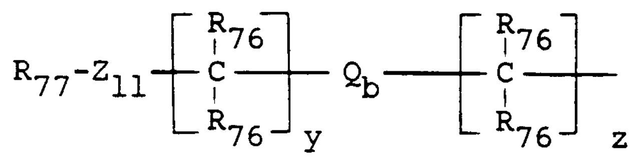

(wherein $Z_{11}$ is an N-containing 6-membered ring heterocycle moiety selected from piperidinyl, diazinyl or triazinyl;

$Q_b$ is a covalent bond, O, S or $NR_{76}$;

y, z, and y + z are integers of 0-10;

$R_{76}$ is H, or $C_1$-$C_3$alkyl;

$R_{77}$ is one or more substituted selected from H, halogen, 1-3C alkyl, unsubstituted amino and its amide derived from a 1-3C carboxylic acid, mono(1-3C alkyl) amino and its amide derived from a 1-3C carboxylic acid, di(1-3C alkyl)amino, tri(1-3C alkyl) ammonium, hydroxy or its ester derived from a 1-3C carboxylic acid, ether having 1-3C, $CO_2H$ and its salts and esters derived from 1-3C alcohols, its amide optionally substituted with one or two 1-3C alkyl groups, and $NO_2$);

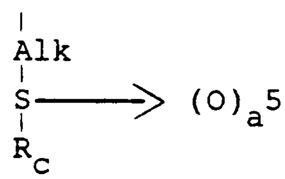

(wherein $R_c$ represents:

$C_1$-$C_6$ alkyl group,

$C_5$-$C_7$ cycloalkyl group,

phenyl group optionally monosubstituted or polysubstituted by a halogen, a $C_1$-$C_6$ alkyl group or a trifluoromethyl group, or

5-membered or 6-membered heterocycle containing 1 or 2 heteroatoms chosen from nitrogen and sulfur,

Alk denoted a linear or branched $C_1$-$C_6$ alkylene group,

$a^5$ represents 0 or the integer 1 or 2);

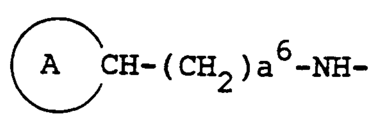

(wherein $a^6$ is 0 to 4 and

Ring A is 5-8C cycloalkenyl, bicycloheptyl, bicycloheptenyl or 4-7C saturated heterocyclyl containing 0, S, SO or $SO_2$);

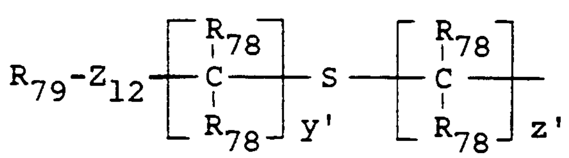

(wherein $Z_{12}$ is a 6-membered aromatic ring containing ≧ 1 N atom(s); where:

the ring is optionally substituted by (optionally substituted, optionally unsaturated) 1-6C alkyl, (optionally substituted) aryl, (optionally substituted) benzyl, OH, halogen, carbonyl, alkoxy, $NO_2$, $CONH_2$, (optionally substituted) $NH_2$ and/or carboxylate, such as pyridine, pyridazine, pyrimidine or pyrazine ring;

$R_{78}$ is H or (optionally substituted, optionally unsubstituted) 1-4C alkyl;

$R_{79}$ is H, (optionally substituted, optionally unsubstituted) 1-6C alkyl, (optionally substituted) aryl, (optionally substituted) benzyl, OH, halogen, carbonyl, alkoxy, $NO_2$, $CONH_2$, (optionally substituted) amino or carboxylate,

y' + z' is 0 to 5);

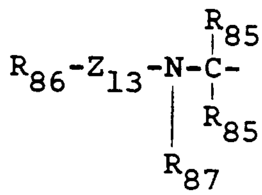

or

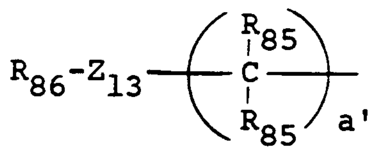

(wherein $Z_{13}$ is a pyridine, pyridazine, pyrimidine or pyrazine ring, optionally substituted by optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{86}$ is H or optionally substituted, optionally unsaturated 1-4C alkyl;

$R_{86}$ is one or more of H, optionally substituted, optionally unsaturated 1-6C alkyl, optionally substituted aryl, optionally substituted benzyl, OH, halogen, oxo, alkoxy, $NO_2$, amido, optionally substituted $NH_2$ or carboxylate;

$R_{87}$ is H, optionally substituted, optionally unsaturated 1-4C alkyl or acyl;

a' is 1-5);

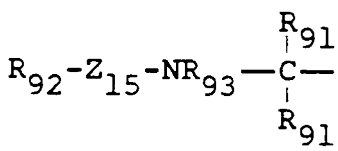

or

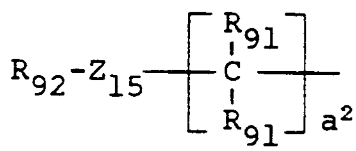

(wherein $Z_{15}$ is a 6 membered aromatic ring containing one or more N atoms such as pyridine, pyridazine, pyrimidine or pyrazine, which ring may be substituted with one or more optionally substituted, optionally unsaturated 1-6C alkyl, optionally substituted aryl, optionally substituted benzyl, OH, halogen, carbonyl, alkoxy, $NO_2$, amido, optionally substituted amino or carboxylate);

$R_{91}$ is H or optionally substituted, optionally unsaturated 1-4C alkyl;

$R_{92}$ is H or one or more substituents selected from optionally substituted, optionally unsaturated 1-6C alkyl, optionally substituted aryl, optionally substituted benzyl, OH, halogen, carbonyl, alkoxy, $NO_2$, amido, optionally substituted amino or carboxylate;

$R_{93}$ is H, optionally substituted, optionally unsaturated 1-4C alkyl or acyl;

$a^2$ is 1 to 5);

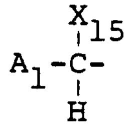

(wherein $X_{15}$ is hydrogen, methyl, or ethyl, and $A_1$ is phenyl substituted in the para-position by isobutyl, cyclohexyl, alkoxy, or 1-pyrrolinyl and, optionally substituted additionally in the meta-position by fluorine or chlorine, or phenyl substituted in the meta-position by benzoyl or phenoxy, or phenyl substituted in the ortho-position by 2,4-dichlorophenoxy or 2,6-dichlorophenylamino);

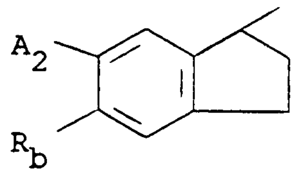

(wherein
$R_b$ is cyclohexyl or cyclophenylmethyl; and
$A_2$ is hydrogen or chlorine);

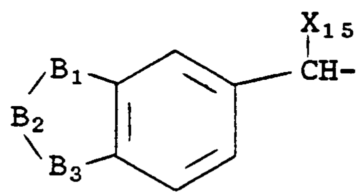

(wherein $X_{15}$ is as defined above, and

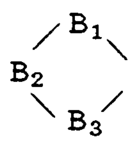

is

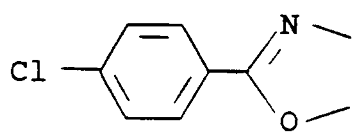

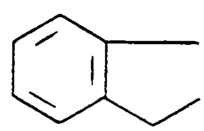

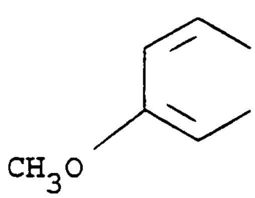

or

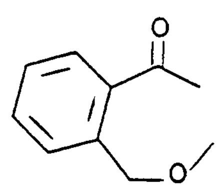

;

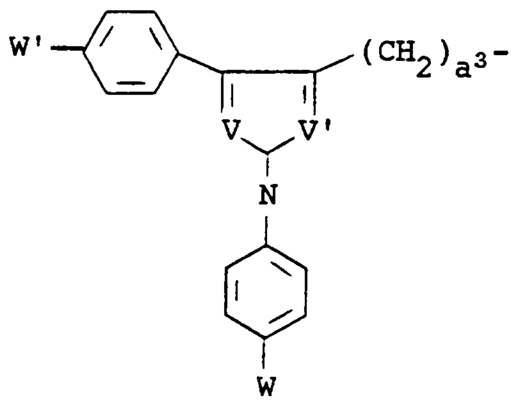

wherein $a^3$ is 1, 2 or 3;

W and W', are identical or different, and each is hydrogen, fluorine or chlorine, and

one of V and V' is nitrogen and the other is a methyne residue optionally substituted by a phenyl group, and

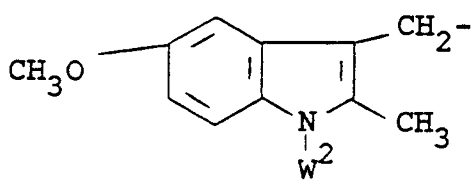

wherein $W^2$ is p-chlorobenzoyl or cinnamoyl);

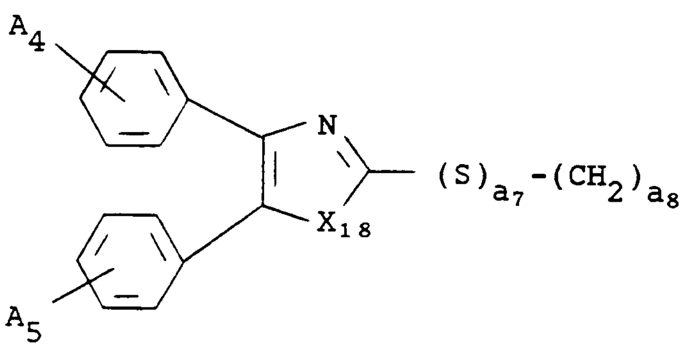

(wherein $A_4$ and $A_5$ are the same or different and are H, OH, lower alkoxy or halogen;

$X_{15}$ is O, S or NH;

$a_7$ is 0 or 1;

$a_8$ is 0 or an integer of 1-6);

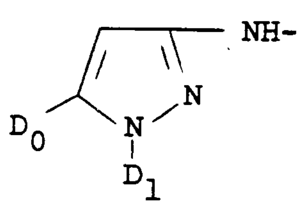

(wherein $D_0$ is H or alkyl;
$D_1$ is H or lower alkyl);

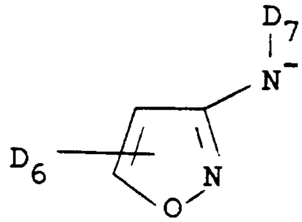

(wherein $D_6$ is H, 1-10C alkyl, 3-10C cycloalkyl, phenyl, 2-10C alkenyl (optionally substituted by phenyl) or phenyl(1-5C)alkyl (optionally ring-substituted by a 1-5C alkoxy);
$D_7$ is H or 2-6C alkanoyl);

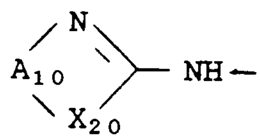

(wherein $A_{10}$ is a group of formula (a)-(c):

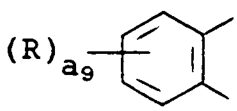

(a)

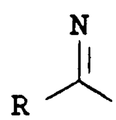

(b)

or

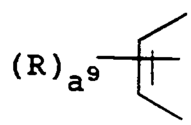

(c)

and $X_{20}$ is O, S or NH);

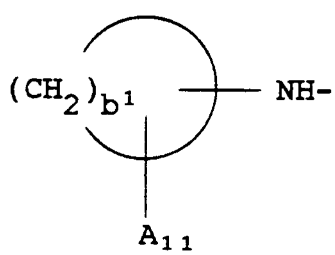

(wherein $A_{11}$ is H or 1-5C alkyl; $b_1$ is 3-10);

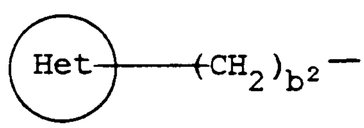

(wherein ring Het is a group of formula (A) or (B):

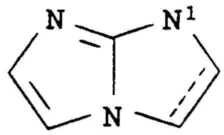

(A)

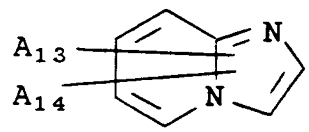

(B)

the dotted line represents an optional double bond; $A_{13}$, $A_{14}$ are H, 1-5C alkyl, halogen or OH);

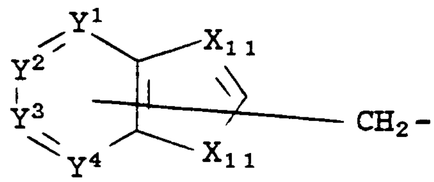

(wherein $X_{11}$ are both N or one is N and the other is CH; one of $Y^1$-$Y^4$ is N and the rest is CH);

and the other of $R^5$ and $R^6$ is H, halogen, $C_1$-$C_{30}$ alkyl, amino, alkylamino, dialkylamino, uriedo ($NH_2CO$-$N(R^{38})$-where $R^{38}$ is H, alkyl, benzyl, phenyl optionally substituted with Cl or $CH_3$); alkenylamino, cycloalkylamino, aryloxy, pyridinium, guanidinium, ammonium, di-and tri-lower alkanolammonium, hydroxy, arylalkyl, alkoxy, alkylaryloxy, $-CH_2CO_2H$, $-CH_2PO_3H_2$, $-CH(PO_3H_2)(OH)$, $-CH_2CO_2C_2H_5$, $-CH_2CH(PO_3H_2)_2$, a hydrocarbyl radical as defined herein, a heterocyclic radical as defined herein, alkanoyl, an $R^6$ or $R^5$ radical as defined herein, a prodrug ester (such as (1-alkanoyloxy)alkyl, for example $t$-$C_4H_9CO_2CH_2$-, $CH_3CO_2CH_2$-);

at least one of $R^5$ and $R^6$ being a lipophilic group, or $R^5$ and $R^6$ can be joined to form a carbocyclic ring containing 3 to 12 carbons or a heterocyclic ring containing N, O and/or S atoms, such as of the formula

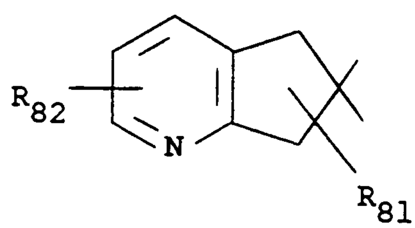

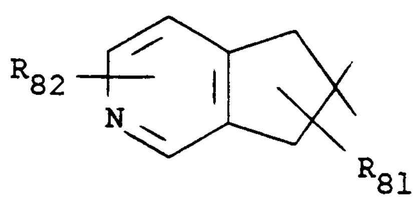

or

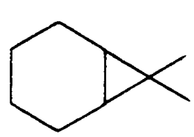

wherein $R_{81}$ and $R_{82}$ are each one or more substituents selected from H, optionally substituted saturated or unsaturated 1-6C alkyl, optionally substituted aryl, optionally substituted benzyl, amido, OH, halogen, optionally substituted amino, amido, COOH, carbonyl, carboxylate, alkoxy and $NO_2$,

with a pharmaceutically acceptable carrier.

**3.** The method as defined in Claim 1 or 2 wherein the squalene synthetase inhibitor employed is
tetramethyl 1(p-chlorophenyl)methane-1-hydroxy 1,1-diphosphonate,
tetramethyl 2,2-dimethyl 2-(p-chlorophenoxy)ethane 1-hydroxy-1,1-diphosphonate,
tetramethyl 1-[4(4'-chlorobenzoyl)phenyl]methane 1-hydroxy 1,1-diphosphonate,
dimethyl 1[(dimethoxyphosphinyl)p-chlorobenzyl]phosphate,
dimethyl [1(dimethoxyphosphinyl) 2,2-dimethyl 2-phenyl]-ethyl phosphate,
dimethyl [1(dimethoxyphosphinyl)2,2-dimethyl 2(p-chlorophenyl] ethyl phosphate,
tetramethyl 4-phenylbutylidene, 1,1-diphosphonate.

**4.** The method as defined in Claim 1 wherein the lipophilic group is optionally substituted alkyl, optionally substituted alkenyl or optionally substituted aryl.

**5.** The method as defined in Claim 2 wherein the bisphosphonate is heptylidenebisphosphonic acid, esters thereof, salts thereof, or mixed ester-salts thereof, or trisodium or tripotassium salt;
nonylidenebisphosphonic acid, esters thereof, salts thereof, or mixed ester-salts thereof, or tetrasodium or tetrapotassium salt;
decylidenebisphosphonic acid, esters thereof, salts thereof, or mixed ester-salts thereof, or tetrasodium or tetrapotassium salt;
tridecylidenebisphosphonic acid, esters thereof, salts thereof, or mixed ester-salts thereof;
octadecylidenebisphosphonic acid, esters thereof, salts thereof, or mixed ester-salts thereof, or tetrasodium or tetrapotassium salt;
heneicosylidenebisphosphonic acid, esters thereof, salts thereof, or mixed ester-salts thereof, or tetrasodium or tetrapotassium salt;
(6-methyl-5-heptenylidene)bisphosphonic acid, esters thereof, salts thereof, or mixed ester-salts thereof, or tetrapotassium or tetrasodium salt;
[2-(4-butylphenyl)ethylidene]bisphosphonic acid, esters thereof, salts thereof, or mixed ester-salts thereof.